# EUROPEAN PATENT SPECIFICATION

(11) **EP 4 352 252 B1**
(45) Date of publication and mention of the grant of the patent: **28.01.2026**
(21) Application number: 22751905.5
(22) Date of filing: 12.07.2022
(51) Int. Cl.: C12Q 1/6816, C12Q 1/6837

(54) **METHODS FOR SPATIAL ANALYSIS USING TARGETED PROBE SILENCING**
VERFAHREN ZUR RÄUMLICHEN ANALYSE UNTER VERWENDUNG VON GEZIELTER SONDENABSCHALTUNG
PROCÉDÉS D'ANALYSE SPATIALE UTILISANT UN SILENÇAGE DE SONDE CIBLÉE

(30) Priority: 13.07.2021 US 202163221297 P
(43) Date of publication of application: 17.04.2024
(73) Proprietor: 10X Genomics, Inc., Pleasanton, CA 94588-3260 (US)
(72) Inventor: KATIRAEE, Layla, Pleasanton, California 94588-3260 (US); BORGSTROM, Erik Leonard Henrik, 113 63 Stockholm (SE); ROELLI, Patrick, 113 63 Stockholm (SE); DRENNON, Tingsheng Yu, Pleasanton, California 94588-3260 (US)
(74) Representative: Vossius & Partner Patentanwälte Rechtsanwälte mbB
(86) International application number: PCT/US2022/036787
(87) International publication number: WO 2023/287765

(56) References cited:
- WO-A1-2020/176788
- GB-A- 2 520 765
- YAN FU ET AL: "Repeat subtraction-mediated seqeunce capture from a complex genome", PLANT JOURNAL, BLACKWELL SCIENCE, OXFORD, GB, vol. 62, no. 5, 1 May 2010 (2010-05-01), pages 1 - 12, XP002580274, ISSN: 1365-313X, [retrieved on 20100304], DOI: 10.1111/J.1365-313X.2010.04196.X

## Description

### TECHNICAL FIELD OF THE INVENTION

Provided herein are methods for selective silencing of one or more probes used in templated ligation, or RNA-templated ligation. In some embodiments, the selective silencing is achieved using one or more blocker oligonucleotides.

### BACKGROUND

Cells within a tissue of a subject have differences in cell morphology and/or function due to varied analyte levels (e.g., gene and/or protein expression) within the different cells. The specific position of a cell within a tissue (e.g., the cell's position relative to neighboring cells or the cell's position relative to the tissue microenvironment) can affect, e.g., the cell's morphology, differentiation, fate, viability, proliferation, behavior, and signaling and crosstalk with other cells in the tissue.

Spatial heterogeneity has been previously studied using techniques that only provide data for a small handful of analytes in the context of an intact tissue or a portion of a tissue, or provide a lot of analyte data for single cells, but fail to provide information regarding the position of the single cell in a parent biological sample (e.g., tissue sample).

Generally, targeting a particular analyte in a biological sample utilizes a capture probe that targets a common transcript sequence such as a poly(A) mRNA-like tail. However, this approach is capable of detecting a high number of off-target analytes. Methods such as templated ligation, or RNA-templated ligation (RTL), offer an alternative to indiscriminant capture of a common transcript sequence. *See, e.g.,* Yeakley, PLoS One, 25;12(5):e0178302 (2017), which is incorporated by reference in its entirety. Probe design in templated ligation results in the ability to achieve coverage of an entire transcriptome. However, some analytes are highly expressed at a particular spatial location but are not of interest in a particular experimental setting. Because capture probes indiscriminately detect analytes (e.g., by hybridizing to the poly(A) tail on an mRNA analyte), an experiment can detect analytes that are not of interest because probes specific to the analyte of interest are used to achieve coverage of an entire transcriptome. Thus, there is a need to develop a method to selectively silence some probes used for templated ligation.

### SUMMARY

The present invention is set out in the appended set of claims.

Targeted probe silencing provides a method to selectively silence one or more probes used in templated ligation processes. By designing synthetic molecules that are antisense to probes to be silenced, one can selectively silence such probes and block hybridization with their target analyte(s) (e.g., a gene transcript) in a biological sample. In some cases, after a panel (or a pool) of templated ligation probes is generated, one can selectively silence a subset of the probes (e.g., probes targeting a highly expressed gene transcript in a tissue sample). Further, a subset of probes can be generated by mistake, or their existence can cause poor performance during templated ligation processes. For example, such probes may have non-specific binding and cannot be easily "removed" from the panel of templated ligation probes. By designing synthetic molecules that are antisense to undesired probes, one can selectively "silence" these probes. In general, methods described herein can improve the overall efficiency for determining location and/or abundance of an analyte using templated ligation. For example, number of genes detected, sensitivity, sequencing saturation level, and/or spatiality (or diversity) of a templated-ligation-based spatial analysis can be significantly improved.

The disclosure also provides an example of silencing template ligation probes against *SERF1A* gene transcripts, using human brain tissue samples. Spatial analysis parameters using different antisense oligonucleotides (e.g., unmodified or modified by LNA) and hybridization conditions for target temaplated ligation probe binding were compared and discussed. In some embodiments, the synthetic probes are not phosphorylated or ligated. In some embodiments, the blocker oligonucleotides described herein are modified (e.g., biotinylated, LNA modifications, inverted thymines).

In some embodiments, methods described herein increases number of genes detected, sensitivity, and/or spatiality (or diversity) of a templated-ligation-based spatial analysis by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a reference biological sample where the probes targeted for reduction or exclusion are not silenced. In some embodiments, methods described herein decreases sequencing saturation level to less than 95%, less than 90%, less than 85%, less than 80%, less than 75%, less than 70%, less than 65%, less than 60%, less than 55%, or less than 50% compared to a reference biological sample where the probes targeted for reduction or exclusion are not silenced.

In one aspect, provided herein is a method for determining a location of an analyte in a biological sample, the method comprising: (a) providing the biological sample on an array comprising a plurality of capture probes, in some embodiments, a capture probe of the plurality of capture probes comprises: (i) a spatial barcode and (ii) a capture domain, in some embodiments, the biological sample comprises a plurality of analytes comprising the analyte and one or more overexpressed analytes; (b) contacting the biological sample with a plurality of probes, in some embodiments, the plurality of probes comprise: (i) a first probe and a second probe, in some embodiments, each probe comprises sequences that are substantially complementary to sequences of the analyte, and in some embodiments, the second probe comprises a capture probe capture domain; (ii) a first and a second probe targeted for reduction or exclusion, in some embodiments, each probe targeted for reduction or exclusion comprises sequences that are substantially complementary to sequences of the one or more overexpressed analytes; and (iii) a first blocker oligonucleotide and a second blocker oligonucleotide, in some embodiments, each blocker oligonucleotide is substantially complementary to all or a portion of either the first or the second probe targeted for reduction or exclusion; (c) hybridizing: (i) the first probe and the second probe to the analyte; (ii) the first probe targeted for reduction or exclusion to the first blocker oligonucleotide and the second probe targeted for reduction or exclusion to the second blocker oligonucleotide; (d) ligating the first probe and the second probe, thereby producing a ligation product; (e) capturing the ligation product by the capture domain on the array, and (f) determining (i) all or part of the sequence of the ligation product bound to the capture domain, or a complement thereof, and (ii) all or part of the sequence of the spatial barcode, or a complement thereof, and using the determined sequences of (i) and (ii) to determine the location of the analyte in the biological sample.

In one aspect, provided herein is a method for decreasing the capture of one or more overexpressed analytes in a biological sample comprising: (a) providing the biological sample on an array comprising a plurality of capture probes, in some embodiments, a capture probe of the plurality of capture probes comprises: (i) a spatial barcode and (ii) a capture domain, in some embodiments, the biological sample comprises a plurality of analytes comprising the analyte and one or more overexpressed analytes; (b) contacting the biological sample with a plurality of probes, in some embodiments, the plurality of probes comprise: (i) a first probe and a second probe, in some embodiments, the first probe and the second probe each comprises sequences that are substantially complementary to sequences of the analyte; (ii) a first and a second probe targeted for reduction or exclusion, in some embodiments, each probe targeted for reduction or exclusion comprises sequences that are substantially complementary to sequences of the one or more overexpressed analytes; and (iii) a first blocker oligonucleotide and a second blocker oligonucleotide, in some embodiments, each blocker oligonucleotide is substantially complementary to all or a portion of the first or the second probe targeted for reduction or exclusion; (c) hybridizing: (i) the first probe and the second probe to the analyte; (ii) the first probe targeted for reduction or exclusion to the first blocker oligonucleotide, and the second probe targeted for reduction or exclusion to the second blocker oligonucleotide; (d) ligating the first probe and the second probe, thereby generating a ligation product; (e) capturing the ligation product by the capture domain on the array, and (f) determining (i) all or part of the sequence of the ligation product bound to the capture domain, or a complement thereof, and (ii) all or part of the sequence of the spatial barcode, or a complement thereof, in some embodiments, the decreasing of the capture of the one or more overexpressed analytes is compared relative to a reference biological sample to which the capture of the one or more overexpressed analytes has not been blocked using the blocker oligonucleotides.

In one aspect, provided herein is a method for increasing diversity of spatial analysis results in a biological sample comprising: (a) providing the biological sample on an array comprising a plurality of capture probes, in some embodiments, a capture probe of the plurality of capture probes comprises: (i) a spatial barcode and (ii) a capture domain, in some embodiments, the biological sample comprises a plurality of analytes comprising an analyte and one or more overexpressed analytes; (b) contacting the biological sample with a plurality of probes, in some embodiments, the plurality of probes comprise: (i) a first probe and a second probe, in some embodiments, the first probe and the second probe each comprises sequences that are substantially complementary to sequences of the analyte; (ii) a first and a second probe targeted for reduction or exclusion, in some embodiments, each probe targeted for reduction or exclusion comprises sequences that are substantially complementary to sequences of the one or more overexpressed analytes; and (iii) a first blocker oligonucleotide and a second blocker oligonucleotide, in some embodiments, each blocker oligonucleotide is substantially complementary to all or a portion of either the first or the second probe targeted for reduction or exclusion; (c) hybridizing: (i) the first probe and the second probe to the analyte; (ii) the first probe targeted for reduction or exclusion to the first blocker oligonucleotide; and (iii) the second probe targeted for reduction or exclusion to the second blocker oligonucleotide; (d) ligating the first probe and the second probe, thereby generating a ligation product; (e) capturing the ligation product by the capture domain on the array; and (f) determining (i) all or part of the sequence of the ligation product bound to the capture domain, or a complement thereof, and (ii) all or part of the sequence of the spatial barcode, or a complement thereof, in some embodiments, the diversity of spatial analysis results is increased relative to a reference biological sample to which the capture of the one or more overexpressed analytes has not been blocked using the blocker oligonucleotides.

As used herein, the term "increasing diversity" refers to increasing the number of different analytes that are detected by the assay. For example, without using the blocker oligonucleotides described herein, the spatial array can capture the one or more overexpressed analytes (e.g., rRNA), thereby reducing the detection limit for other analytes of interest. In some embodiments, the capture probe capture domain comprises a sequence that is complementary to all or a portion of the capture domain.

In one aspect, provided herein is a method for decreasing the capture of one or more analytes in a biological sample for spatial analysis comprising: (a) providing an array comprising a plurality of capture probes, in some embodiments, a capture probe of the plurality of capture probes comprises: (i) a spatial barcode and (ii) a capture domain; (b) contacting the biological sample comprising a plurality of analytes, in some embodiments, the plurality of analytes comprise the one or more analytes for decreased capture and an analyte, with: (i) a plurality of probe pairs comprising a first probe and a second probe, in some embodiments, the first probe and the second probe each comprises sequences that are substantially complementary to sequences of either the analyte or the one or more analytes for decreased capture; and (ii)a first blocker oligonucleotide, in some embodiments, the first blocker oligonucleotide is substantially complementary to the sequence of the first or second probe in a probe pair targeting the one or more analytes for decreased capture; (c) hybridizing: (i) the plurality of probe pairs to the plurality of analytes, in some embodiments, the first probe and the second probe are hybridized to the ananlyte; and (ii) the first blocker oligonucleotide to the first or second probe in the probe pair targeting the one or more analytes for decreased capture; (d) ligating the hybridized first probe and the second probe, thereby generating a ligation product; and (e) capturing the ligation product (e.g., by the capture domain) on the array, in some embodiments, the capture of the ligation product for spatial analysis of one or more analytes for decreased capture is decreased compared to the capture of a ligation product where no blocker oligonucleotide is present.

In one aspect, provided herein is a method for increasing nucleic acid diversity in spatial transcriptomic analysis in a biological sample comprising: (a) providing an array comprising a plurality of capture probes, in some embodiments, a capture probe of the plurality of capture probes comprises: (i) a spatial barcode and (ii) a capture domain; (b) contacting the biological sample comprising a plurality of nucleic acids, in some embodiments, the plurality of nucleic acids comprise one or more nucleic acids for decreased capture and a nucleic acid, with: (i) a plurality of probe pairs comprising a first probe and a second probe, in some embodiments, the first probe and the second probe each comprises sequences that are substantially complementary to sequences of either the nucleic acid or one or more nucleic acids for decreased capture; and (ii) a first blocker oligonucleotide, in some embodiments, the first blocker oligonucleotide is substantially complementary to the sequence of the first or second probe in a probe pair targeting the one or more nucleic acids for decreased capture; (c) hybridizing: (i) the plurality of probe pairs to the plurality of nucleic acids, in some embodiments, the first probe and the second probe are hybridized to the nucleic acid; and (ii) the first blocker oligonucleotide to the first or second probe in the probe pair targeting the one or more nucleic acids for decreased capture; (d) ligating the hybridized first probe and the second probe, thereby generating a ligation product; and (e) capturing the ligation product by the capture domain on the array, in some embodiments, the nucleic acid diversity in spatial transcriptomic analysis is increased relative to the nucleic acid diversity in a spatial transcriptomic assay where no blocker oligonucleotide is present.

In one aspect, provided herein is a method for increasing the detection of one or more rare or low-expressing nucleic acids in a biological sample for spatial analysis comprising: (a) providing an array comprising a plurality of capture probes, in some embodiments, a capture probe of the plurality of capture probes comprises: (i) a spatial barcode and (ii) a capture domain; (b) contacting the biological sample comprising a plurality of nucleic acids, in some embodiments, the plurality of nucleic acids comprise one or more nucleic acids for decreased capture and a rare or low-expressing nucleic acid, with: (i) a plurality of probe pairs comprising a first probe and a second probe, in some embodiments, the first probe and the second probe each comprises sequences that are substantially complementary to sequences of either the rare or low-expressing nucleic acid or the one or more nucleic acids for decreased capture; and (ii) a first blocker oligonucleotide, in some embodiments, the first blocker oligonucleotide is substantially complementary to the sequence of the first or second probe in a probe pair targeting the one or more nucleic acids for decreased capture; (c) hybridizing: (i) the plurality of probe pairs to the plurality of nucleic acids, in some embodiments, the first probe and the second probe are hybridized to the rare or low-expressing nucleic acid; and (ii) the first blocker oligonucleotide to the first or second probe in the probe pair targeting the one or more nucleic acids for decreased capture; (d) ligating the hybridized first probe and the second probe, thereby generating a ligation product; and (e) capturing the ligation product by the capture domain on the array, in some embodiments, the detection of one or more rare or low-expressing nucleic acids is increased compared to a reference biological sample where no blocker oligonucleotide is present.

In some embodiments, the method described herein further comprises: contacting the biological sample with a second blocker oligonucleotide, in some embodiments, the second blocker oligonucleotide is substantially complementary to the sequence of the first or second probe in the probe pair targeting the one or more analytes or nucleic acids for decreased capture; and hybridizing the second blocker oligonucleotide to the first or second probe in the probe pair targeting the one or more analytes or nucleic acids for decreased capture. In some embodiments, the first blocker oligonucleotide is substantially complementary to the sequence of the first probe in the probe pair targeting the one or more analytes or nucleic acids for decreased capture, and the second blocker oligonucleotide is substantially complementary to the sequence of the second probe in the probe pair targeting the one or more analytes or nucleic acids for decreased capture. In some embodiments, the first blocker oligonucleitde and/or the second blocker oligonucleotide block the first and/or second probes in the probe pair targeting the one or more analytes or nucleic acids for decreased capture from hybridizing to the one or more analytes or nucleic acids for decreased capture.

In some embodiments, the the one or more analytes or nucleic acids for decreased capture comprises a highly expressed gene transcript. In some embodiments, the highly expressed gene transcript accounts for at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, or at least 50% of all analytes detected in the biological sample where no blocker oligonucleotide is present. In some embodiments, the one or more analytes or nucleic acids for decreased capture comprises an RNA, e.g., a messenger RNA (mRNA), ribosomal RNA (rRNA), mitocontdrial RNA (mtRNA), and/or transfer RNA (tRNA), or fragment thereof. In some embodiments, the one or more analytes or nucleic acids for decreased capture comprises a housekeeping analyte, e.g., glyceraldehyde-3-phosphate dehydrogenase (GAPDH), TATA-binding protein (TBP), and/or ribosomal proteins (RP). In some embodiments, the probe pair targeting the one or more analytes for decreased capture can hybridize to off-target sequences.

In some embodiments, the first and second probes in the probe pair targeting the one or more analytes or nucleic acids for decreased capture are not hybridized to the one or more analytes or nucleic acids for decrased capture. In some embodiments, the first and second probes in the probe pair targeting the one or more analytes or nucleic acids for decreased capture are not ligated together. Isome embodiments, the first and second probes in the probe pair targeting the one or more analytes or nucleic acids for decreased capture are not captured by the capture domain on the array.

In some embodiments, the biological sample is mounted on a first substrate, and the array is mounted on a second substrate. In some embodiments, the first substrate is aligned with the second substrate prior to step (e), such that at least a portion of the biological sample is aligned with at least a portion of the array.

In some embodiments, the first blocker oligonucleotide and/or the second blocker oligonucleotide each does not comprise a phosphorylated nucleotide at its 5' end and/or does not comprise one or more ribonucleic acid bases at its 3' end.

In some embodiments, the second probe in the probe pair targeting the one or more analytes or nucleic acids for decreased capture comprises a capture probe capture domain, and first blocker oligonucleotide and/or the second blocker oligonucleotide each does not comprise a sequence that is substantially complementary to the capture probe capture domain.

In some embodiments, the first blocker oligonucleotide and/or the second blocker oligonucleotide decrease the concentration of a ligation product generated from the first and second probes in the probe pair targeting the one or more analytes or nucleic acids for decreased capture that hybridize to the plurality of capture probes by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a reference biological sample where no blocker oligonucleotide is present.

In some embodiments, the first blocker oligonucleotide and/or the second blocker oligonucleotide increase the concentration of one or more ligation products that hybridize to the plurality of capture probes by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, or more compared to a reference biological sample where no blocker oligonucleotide is present.

In some embodiments, the first blocker oligonucleotide is substantially complementary to the sequence of the first probe in the probe pair targeting the one or more analytes or nucleic acids for decreased capture that is complementary to the one or more analytes or nucleic acids for decreased capture, and/or the second blocker oligonucleotide is substantially complementary to the sequence of the second probe in the probe pair targeting the one or more analytes or nucleic acids for decreased capture that is complementary to the one or more analytes or nucleic acids for decrased capture.

In some embodiments, the first blocker oligonucleotide hybridizes to one or more portions of the sequence of the first probe in the probe pair targeting the one or more analytes or nucleic acids for decreased capture, and/or the second blocker oligonucleotide hybridizes to one or more portions of the sequence of the second probe in the probe pair targeting the one or more analytes or nucleic acids for decreased capture.

In some embodiments, the first blocker oligonucleotide hybridizes to the sequence of the first probe in the probe pair targeting the one or more analytes or nucleic acids for decreased capture that is complementary to the one or more analytes or nucleic acids for decreased capture, and/or the second blocker oligonucleotide hybridizes to the sequence of the second probe in the probe pair targeting the one or more analytes or nucleic acids for decreased capture that is complementary to the one or more analytes or nucleic acids for decreased capture.

In some embodiments, the contacting the biological sample with a plurality of probe pairs comprises contacting the biological sample with 5000 or more probe pairs, in some embodiments, a first probe pair of the 5000 or more probe pairs comprises the first probe and the second probe, in some embodiments, a second probe pair of the 5000 or more probe pairs comprises the first and second probes targeting the one or more analytes or nucleic acids for decreased capture.

In some embodiments, the biological sample comprises at least 1, at least 2, at least 3, at least 4, at least 5, at least 6, at least 7, at least 8, at least 9, at least 10, at least 20, at least 30, at least 40, at least 50, at least 60, at least 70, at least 80, at least 90, at least 100 or more analytes or nucleic acids for decreased capture.

In some embodiments, the biological sample is contacted with at least 1, at least 2, at least 3, at least 4, at least 5, at least 6, at least 7, at least 8, at least 9, at least 10, at least 20, at least 30, at least 40, at least 50, at least 60, at least 70, at least 80, at least 90, or at least 100 blocker oligonucleotide pairs, in some embodiments, a blocker oligonucleotide pair of the blocker oligonucleotide pairs comprises the first blocker oligonucleotide and the second blocker oligonucleotide.

In some embodiments, the concentration of the first blocker oligonucleotide and/or the second blocker oligonucleotide is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 2-fold, at least 3-fold, at least 4-fold, at least 5-fold, at least 6-fold, at least 7-fold, at least 8-fold, at least 9-folds, at least 10-fold compared to the concentration of the first and/or second probes in the probe pair targeting the one or more analytes or nucleic acids for decreased capture.

In some embodiments, the first blocker oligonucleotide and/or the second blocker oligonucleotide are about 10, about 15, about 20, about 25, about 30, about 35, about 40, about 45, about 50, about 55, about 60, about 65, about 70, about 75, about 80, about 85, about 90, about 95, or about 100 nucleotides in length.

In some embodiments, the first blocker oligonucleotide and/or the second blocker oligonucleotide comprise one or more modifications.

In some embodiments, the one or more modifications comprise linking the first blocker oligonucleotide and/or the second blocker oligonucleotide with biotins, amino-modifiers, azide, cholesteryl, alkynes, and/or thiol modifiers.

In some embodiments, the one or more modifications comprise a fluorophore.

In some embodiments, the one or more modifications comprise a 3'-3'-inverted thymine.

In some embodiments, the first blocker oligonucleotide and/or the second blocker oligonucleotide comprise a non-natural nucleic acid (e.g., at least 1, 2, 3, 4, 5, 6, 7, 8, 9, or 10 non-natural nucleic acids). In some embodiments, the non-natural nucleic acid is a locked nucleic acid (LNA).

In some embodiments, the first blocker oligonucleotide and/or the second blocker oligonucleotide have a primer melting temperature (Tm) of about 55°C, about 60°C, about 65°C, or about 70°C.

In some embodiments, the first blocker oligonucleotide and/or the second blocker oligonucleotide comprise at least one LNA, in some embodiments, the Tm of the first blocker oligonucleotide and/or the second blocker oligonucleotide is about 110%, about 120%, about 130%, about 140%, about 150%, about 160%, about 170%, about 180%, about 190%, about 200%, or higher as compared to the Tm of a reference oligonucleotide without the LNA.

In some embodiments, the one or more analytes for decreased capture are expressed in a cell from an organ selected from adrenal gland, bladder, bone, bone marrow, brain, bronchi of the lung, diaphragm, esophagus, eyes, fallopian tube, gallbladder, heart, hypothalamus, kidney, large intestine, liver, lungs, lymph node, mammary gland, nasal cavity, ovary, pancreas, penis, placenta, pituitary gland, prostate, skeletal muscle, skin, small intestine, spinal cord, spleen, stomach, testes, thymus gland, thyroid, trachea, tongue, urethra, uterus, and vagina, or any combination thereof.

In some embodiments, the one or more analytes for decreased capture are derived (e.g., transcribed) from a housekeeping gene. In some embodiments, the housekeeping gene encodes 18S ribosomal RNA, beta actin (actB), glyceraldehyde-3-phosphate dehydrogenase (GAPDH), phosphoglycerate kinase 1 (PGK1), peptidylprolyl isomerase A (PPIA), ribosomal protein L13a (RPL13A), ribosomal protein, large, P0 (RPLP0), acidic ribosomal phosphoprotein PO (ARBP), beta-2-microglobulin (B2M), tyrosine 3-monooxygenase/tryptophan5-monooxygenase activation protein, zeta polypeptide (YWHAZ), succinate dehydrogenase complex, subunit A, flavoprotein (Fp) (SDHA), transferrin receptor (TFRC), glucuronidase, beta (GUSB), hydroxymethylbilane synthase (HMBS), hypoxanthine phosphoribosyltransferase 1 (HPRT1), TATA-binding protein (TBP), or any combination thereof.

In some embodiments, the first probe and the second probe are substantially complementary to adjacent sequences of the analyte, the nucleic acid, or the rare or low-expressing nucleic acid.

In some embodiments, the first probe and the second probe hybridize to sequences that are not adjacent to each other on the analyte, the nucleic acid, or the rare or low-expressing nucleic acid.

In some embodiments, the first probe is extended with a DNA polymerase, thereby filling in a gap between the first probe and the second probe and generating an extended first probe.

In some embodiments, the first probe comprises at least two ribonucleic acid bases at the 3' end, and in some embodiments, the second probe comprises a phosphorylated nucleotide at the 5' end.

In some embodiments, generating a ligation product comprises ligating (i) the first probe to the second probe or (ii) the extended first probe to the second probe using enzymatic ligation or chemical ligation, in some embodiments, the enzymatic ligation utilizes a ligase. In some embodiments, the ligase is a T4 RNA ligase (Rnl2), PBCV-1 ligase, a Chlorella virus DNA ligase, a single stranded DNA ligase, or a T4 DNA ligase.

In some embodiments, the second probe comprises a pre-adenylated phosphate group at its 5' end, and in some embodiments, the first probe comprises at least two ribonucleic acid bases at the 3' end.

In some embodiments, the step of ligating the hybridized first probe and the second probe comprises ligating a 3' end of the first probe to the 5' end of the second probe using a ligase that does not require adenosine triphosphate for ligase activity. In some embodiments, the ligase is selected from the group consisting of: thermostable 5' AppDNA/RNA Ligase, truncated T4 RNA Ligase 2, truncated T4 RNA Ligase 2 K227Q, truncated T4 RNA Ligase 2 KQ, PBCV-1 DNA ligase, and a Chlorella Virus DNA Ligase, or any combination thereof.

In some embodiments, the first probe further comprises a functional sequence, in some embodiments, the functional sequence is a primer sequence.

In some embodiments, the method described herein further comprises providing a capture probe capture domain blocking moiety that interacts with the capture probe capture domain. In some embodiments, the method described herein further comprises releasing the capture probe capture domain blocking moiety from the capture probe capture domain prior to step (e).

In some embodiments, the capture probe capture domain comprises a poly-adenylated (poly(A)) sequence or a complement thereof. In some embodiments, the capture probe capture domain blocking moiety comprises a poly-uridine sequence, a poly-thymidine sequence, or both. In some embodiments, releasing the poly-uridine sequence from the poly(A) sequence comprises denaturing the ligation product or contacting the ligation product with an endonuclease, exonuclease and/or ribonuclease.

In some embodiments, the capture probe capture domain comprises a sequence that is complementary to all or a portion of the capture domain of the capture probe.

In some embodiments, the capture probe capture domain comprises a degenerate sequence.

In some embodiments, the first probe and/or the second probe are DNA probes.

In some embodiments, the capture probe capture domain blocking moiety is a DNA probe.

In some embodiments, the method described herein further comprises, prior to step (e), releasing the ligation product from the analyte, the nucleic acid, the rare or low-expressing nucleic acid. In some embodiments, the releasing of (i) the ligation product from the analyte, the nucleic acid, or the rare or low-expressing nucleic acid, or (ii) the capture probe capture domain blocking moiety from the capture domain binding domain, comprises contacting the ligated probe with an endoribonuclease. In some embodiments, the endoribonuclease is RNase H, RNase A, RNase C, or RNase I. In some embodiments, the RNase H comprises RNase H1, RNase H2, or RNase H1 and RNase H2.

In some embodiments, the biological sample is a tissue sample. In some embodiments, the tissue sample is a formalin-fixed, paraffin-embedded (FFPE) tissue sample, a fresh or a frozen tissue sample. In some embodiments, the tissue sample is the FFPE tissue sample, and the tissue sample is decrosslinked.

In some embodiments, the biological sample was previously stained. In some embodiments, the biological sample was previously stained using immunofluorescence or immunohistochemistry. In some embodiments, the biological sample was previously stained using hematoxylin and eosin (H&E).

In some embodiments, the method further comprises contacting the biological sample with a permeabilization agent, in some embodiments, the permeabilization agent is selected from an organic solvent, a detergent, and an enzyme, or a combination thereof. In some embodiments, the permeabilization agent is selected from the group consisting of: an endopeptidase, a protease sodium dodecyl sulfate (SDS), polyethylene glycol tert-octylphenyl ether, polysorbate 80, and polysorbate 20, N-lauroylsarcosine sodium salt solution, saponin, a nonionic detergent (e.g., Triton X-100^{™}), and a nonionic surfactant (e.g., Tween-20^{™}). In some embodiments, the endopeptidase is pepsin or proteinase K.

In some embodiments, the method described herein further comprises, prior to step (a), fixing the biological sample. In some embodiments, the fixing the biological sample is performed using one or both of methanol and acetone.

In some embodiments, the analyte and/or the one or more analytes for decreased capture comprise RNA. In some embodiments, the RNA is an mRNA.

In some embodiments, the method described herein further comprises: determining (i) all or part of the sequence of the ligation product bound to the capture domain, or a complement thereof, and (ii) all or part of the sequence of the spatial barcode, or a complement thereof. In some embodiments, the determining step comprises amplifying all or part of the ligation product specifically bound to the capture domain. In some embodiments, an amplifying product comprises (i) all or part of the sequence of the ligation product specifically bound to the capture domain, or a complement thereof, and (ii) all or part of the sequence of the spatial barcode, or a complement thereof. In some embodiments, the determining step comprises sequencing (i) all or part of the sequence of the ligation product, or a complement thereof, and (ii) all or a part of the sequence of the spatial barcode, or a complement thereof. In some embodiments, the sequencing comprises *in situ* sequencing, Sanger sequencing methods, next-generation sequencing methods, and nanopore sequencing.

In one aspect, provided herein is a kit comprising: (a) a substrate comprising a plurality of capture probes, wherein a capture probe of the plurality of the capture probes comprises a spatial barcode and a capture domain; (b) a plurality of probe pairs comprising a first probe and a second probe, each comprising sequences that are substantially complementary to an analyte or one or more analytes for decreased capture; (c) a first blocker oligonucleotide, wherein the first blocker oligonucleotide is substantially complementary to the sequence of the first or second probe in a probe pair targeting the one or more analytes for decreased capture; and (d) instructions for performing the method described herein. **In** some embodiments, the kit further comprises a second blocker oligonucleotide, wherein the second blocker oligonucleotide is substantially complementary to the sequence of the the first or second probe in a probe pair targeting the one or more analytes for decreased capture. In some embodiments, the second probe comprises a capture probe capture domain, and the second blocker oligonucleotide is substantially complementary to the sequence of the second probe excluding the capture probe capture domain.

In one aspect, provided herein is a kit comprising: (a) an array comprising a plurality of capture probes; (b) a plurality of probe pairs comprising a first probe and a second probe, wherein the first probe and the second probe are substantially complementary to adjacent sequences of an analyte, wherein the second probe comprises (i) a capture probe capture domain that is capable of binding to a capture domain of the capture probe and (ii) a linker sequence; (c) a plurality of enzymes comprising a ribonuclease and a ligase; (d) a first blocker oligonucleotide and a second blocker oligonucleotide, wherein the first blocker oligonucleotide is substantially complementary to the sequence of the first probe in a probe pair targeting one or more analytes for decreased capture, and the second blocker oligonucleotide is substantially complementary to the sequence of the second probe in the probe pair targeting the one or more analytes for decreased capture, wherein the first and second probes in the probe pair targeting the one or more analytes each comprises sequences that are substantially complementary to sequences of one or more analytes for decreased capture; and (e) instructions for performing the method described herein.

In one aspect, provided herein is a kit comprising: (a) an array comprising a plurality of capture probes; (b) a plurality of probes comprising a first probe and a second probe, wherein the first probe and the second probe are substantially complementary to adjacent sequences of an analyte, wherein the first probe includes a linker sequence, wherein the second probe comprises a capture probe capture domain that is capable of binding to a capture domain of the capture probe; (c) a plurality of enzymes comprising a ribonuclease and a ligase; (d) a first blocker oligonucleotide and a second blocker oligonucleotide, wherein the first blocker oligonucleotide is substantially complementary to the sequence of the first probe in a probe pair targeting one or more analytes for decreased capture, and the second blocker oligonucleotide is substantially complementary to the sequence of the second probe in the probe pair targeting the one or more analytes for decreased capture, wherein the first and second probes in the probe pair targeting the one or more analytes each comprises sequences that are substantially complementary to sequences of one or more analytes for decreased capture; and (e) instructions for performing the method described herein. In some embodiments, the second probe comprises a capture probe capture domain, and the second blocker oligonucleotide is substantially complementary to the sequence of the second probe excluding the capture probe capture domain. In some embodiments, the kit comprises a second probe comprising a preadenylated phosphate group at its 5' end, and a ligase that does not require adenosine triphosphate for ligase activity.

In one aspect, provided herein is a composition comprising (a) a biological sample on the spatial array, wherein the biological sample comprises a plurality of analytes comprising an analyte and one or more analytes for decreased capture, (b) a first probe and a second probe that are ligated together, wherein the first probe and the second probe each comprises one or more sequences that are substantially complementary to sequences of the analyte, and wherein either the first probe or the second probe comprises a capture probe capture domain that is hybridized to the capture domain of the capture probe of the spatial array; and (c) a third probe targeting the one or more analytes for decreased capture, wherein the third probe comprises one or more sequences that are substantially complementary to sequences of the one or more analytes for decreased capture, wherein a first blocker oligonucleotide with sequence complementary to the third probe is hybridized to the third probe. In some embodiments, the composition further comprises a fourth probe targeting the one or more analytes for decreased capture, wherein the fourth probe comprises one or more sequence that are substantially complementary to sequences of the one or more analytes for decreased capture, wherein a second bloker oligonucleotide with sequence complementary to the fourth probe is hybridized to the fourth probe. In some embodiments, the composition further comprises a spatial array comprising a plurality of capture probes, wherein a capture probe of the plurality of capture probes comprises a spatial barcode and a capture domain. In some embodiments, the biological sample is on the array. In some embodiments, the biological sample is mounted on a first substrate, and the array is mounted on a second substrate . In some embodiments, the third probe comprises a capture probe capture domain, and the sequence of the first blocker oligonucleotide is substantially complementary to the sequence of the third probe excluding the capture probe capture domain; or the fourth probe comprises a capture probe capture domain, and the sequence of the second blocker oligonucleotide is substantially complementary to the sequence of the fourth probe excluding the capture probe capture domain. In some embodiments, the composition further comprises an RNase H enzyme. In some embodiments, the composition further comprises a ligase. In some embodiments, the first probe or the second probe that does not comprise the capture probe capture domain comprises a functional domain.

In some embodiments, the analyte described herein is a nucleic acid. In some embodiments, the one or more analytes for decrased capture are one or more nucleic acids for decreased capture.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated.

The term "each," when used in reference to a collection of items, is intended to identify an individual item in the collection but does not necessarily refer to every item in the collection, unless expressly stated otherwise, or unless the context of the usage clearly indicates otherwise.

The singular form "a", "an", and "the" include plural references unless the context clearly dictates otherwise. For example, the term "a cell" includes one or more cells, including mixtures thereof. "A and/or B" is used herein to include all of the following alternatives: "A", "B", "A or B", and "A and B".

### DESCRIPTION OF DRAWINGS

The following drawings illustrate certain embodiments of the features and advantages of this disclosure. Like reference symbols in the drawings indicate like elements.
**FIG. 1** is a schematic diagram showing an example of a barcoded capture probe, as described herein.
**FIG. 2** is a schematic illustrating a cleavable capture probe, wherein the cleaved capture probe can enter into a cell and bind to target analytes within the sample.
**FIG. 3** is a schematic diagram of an exemplary multiplexed spatially-barcoded feature.
**FIG. 4** is a schematic diagram of an exemplary analyte capture agent.
**FIG. 5** is a schematic diagram depicting an exemplary interaction between a feature-immobilized capture probe **524** and an analyte capture agent **526.**
**FIGs. 6A****,** **6B****,** and **6C** are schematics illustrating how streptavidin cell tags can be utilized in an array-based system to produce spatially-barcoded cells or cellular contents.
**FIG. 7** shows an exemplary spatial analysis workflow.
**FIG. 8** is a schematic diagram showing an exemplary workflow for templated ligation.
**FIG. 9** is a schematic diagram showing an exemplary workflow for capturing a ligation product on a substrate that includes capture probes.
**FIG. 10** is a schematic diagram showing an example of a first probe that includes a linker sequence and a second probe.
**FIG. 11** is a schematic diagram showing an example of a second probe that includes a linker sequence and a first probe.
**FIG. 12** is a schematic diagram showing an example of a first probe, a second probe, and a spanning probe.
**FIG. 13** is a schematic diagram showing an example of templated ligation using a first probe oligonucleotide, a second probe oligonucleotide, and a third probe oligonucleotide.
**FIGs. 14A-14E** show various approaches for chemically-mediated nucleic acid ligation. **FIG. 14A** illustrates formation of a triazole bond. **FIG. 14B** illustrates formation of a phosphorothioate bond. **FIG. 14C** illustrates formation of an amide bond. **FIG. 14D** illustrates a formation of phosphoramidate bond. **FIG. 14E** illustrates a conjugation reaction.
**FIG. 15** shows an exemplary templated ligation workflow.
**FIG. 16** is a schematic diagram showing an example of using a first blocker oligonucleotide and a second blocker oligonucleotide to block hybridization between probes and an analyte.
**FIG. 17** shows an exemplary spatial analysis workflow using first and second blocker oligonucleotides.

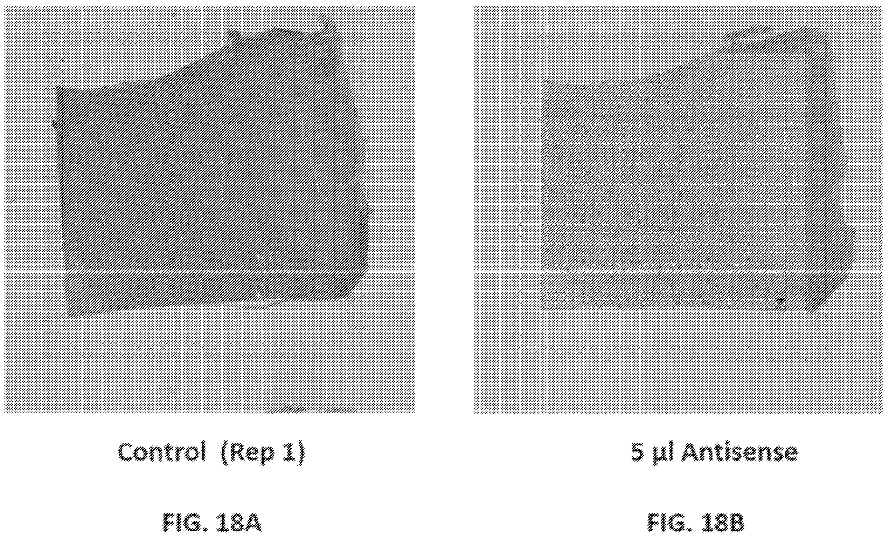

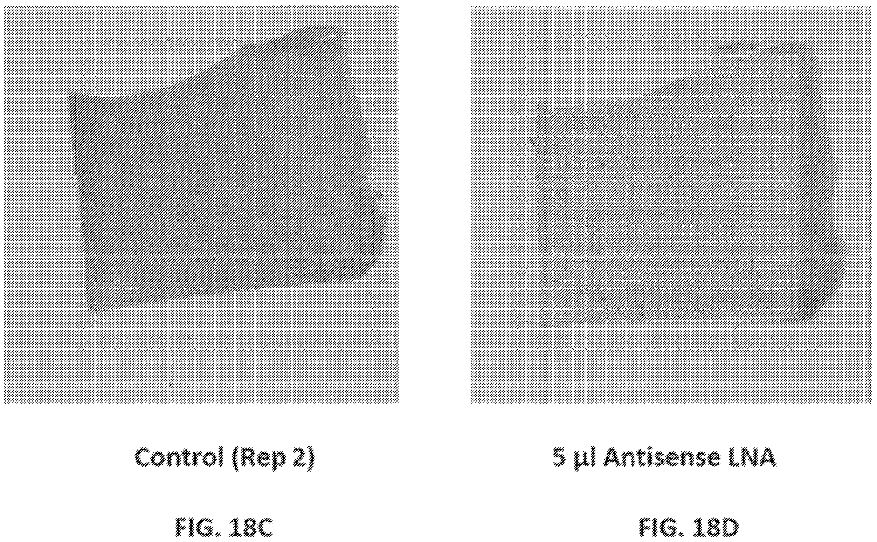

**FIGs. 18A-18D** show spatial expression patterns of *SERF1A* gene in human brain tissue samples after H&E stain. **FIG. 18A** shows the spatial expression pattern of *SERF1A* gene in an untreated control sample (replicate 1, or "Rep 1"). **FIG. 18B** shows the spatial expression pattern of *SERF1A* gene in a sample with added unmodified antisense oligonucleotides ("5 µl Antisense"). **FIG. 18C** shows the spatial expression pattern of *SERF1A* gene in an untreated control sample (replicate 2, or "Rep 2"). **FIG. 18D** shows the spatial expression pattern of *SERF1A* gene in a sample with added LNA-modified antisense oligonucleotide ("5 µl Antisense LNA").
**FIG. 19** shows the number of detected UMI reads of *SERF1A* and *SERF1B* genes in human brain tissue samples. Two samples (Sample 1 and Sample 2) were used for templated ligation detection of *SERF1A* gene expression and one sample was used for templated ligation detection of *SERF1B* gene expression.

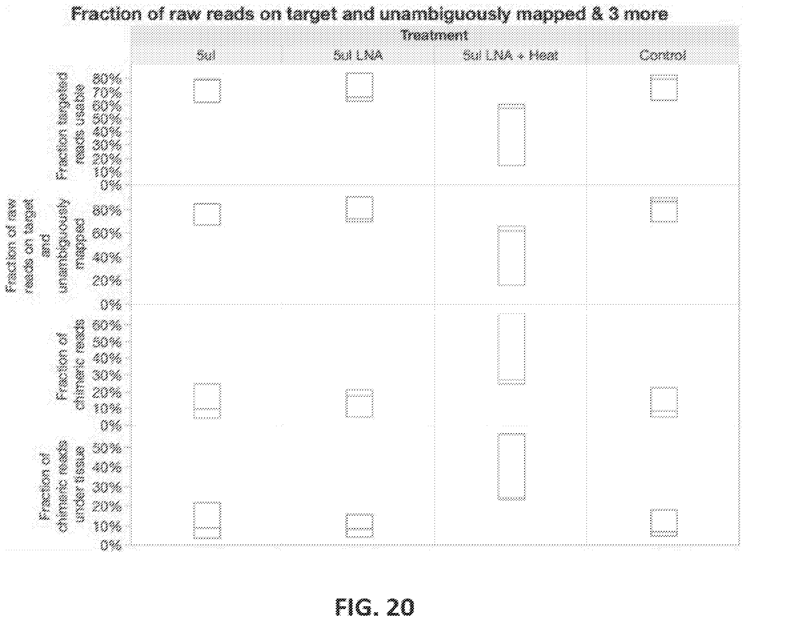

**FIG. 20** shows the fraction of targeted reads usable (first row), fraction of raw reads on target and unambiguously mapped (second row), fraction of chimeric reads (third row), and fraction of chimeric reads under tissue (fourth row) in human brain tissue samples that were treated with one of 1) antisense oligonucleotides directed to templated ligation probes targeting *SERF1A* gene, 2) LNA modified antisense oligonucleotides directed to templated ligation probes targeting *SERF1A* gene, or 3) LNA modified antisense oligonucleotides directed to templated ligation probes targeting *SERF1A* gene in combination with high heat. An untreated sample was used as a control.
**FIG. 21** shows median panel UMI counts at 1000 panel reads (second row) or 5000 panel reads (first row) per spot in human brain tissue samples that were treated with one of the three types of antisense oligonucleotides in **FIG. 20** targeting *SERF1A* gene. An untreated sample was used as a control.
**FIG. 22** shows median panel genes detected at 250 panel reads (second row) or 1000 panel reads (first row) per spot in mouse brain tissue samples that were treated one of the three types of antisense oligonucleotide conditions in **FIG. 20** targeting *SERF1A* gene. An untreated sample was used as a control.
**FIG. 23** shows the percentage of panel nucleic acid PCR duplication (1000 panel reads per spot) in mouse brain tissue samples that were treated with one of the three types of antisense oligonucleotide conditions in **FIG. 20** targeting *SERF1A* gene. An untreated sample was used as a control.
**FIG. 24** shows median Moran's I of top 50 genes (first row) and 10th percentage of Moran's I values (second row) in mouse brain tissue samples that were one of the three types of antisense oligonucleotide conditions in **FIG. 20** targeting *SERF1A* gene. An untreated sample was used as a control.

### DETAILED DESCRIPTION

### I. Introduction

Provided herein are methods of targeted nucleic acid capture. Targeted nucleic acid capture is an attractive alternative to, for example, poly(A) mRNA capture in order to interrogate spatial gene expression in FFPE tissue. Compared to poly(A) mRNA capture, targeted RNA capture is less affected by RNA degradation associated with FFPE fixation than methods dependent on oligo-dT capture and reverse transcription of mRNA; allowing for improved sensitive measurement of specific genes of interest that otherwise might be missed with a whole transcriptomic approach; and is scalable, with demonstrated probes targeting a large fraction of the transcriptome.

Spatial analysis methodologies and compositions described herein can provide a vast amount of analyte and/or expression data for a variety of analytes within a biological sample at high spatial resolution, while retaining native spatial context. Spatial analysis methods and compositions can include, e.g., the use of a capture probe including a spatial barcode (e.g., a nucleic acid sequence that provides information as to the location or position of an analyte within a cell or a tissue sample (e.g., mammalian cell or a mammalian tissue sample) and a capture domain that is capable of binding to an analyte (e.g., a protein and/or a nucleic acid) produced by and/or present in a cell. Spatial analysis methods and compositions can also include the use of a capture probe having a capture domain that captures an intermediate agent for indirect detection of an analyte. For example, the intermediate agent can include a nucleic acid sequence (e.g., a barcode) associated with the intermediate agent. Detection of the intermediate agent is therefore indicative of the analyte in the cell or tissue sample.

Non-limiting aspects of spatial analysis methodologies and compositions are described in U.S. Patent Nos. 10,774,374, 10,724,078, 10,480,022, 10,059,990, 10,041,949, 10,002,316, 9,879,313, 9,783,841, 9,727,810, 9,593,365, 8,951,726, 8,604,182, 7,709,198, U.S. Patent Application Publication Nos. 2020/239946, 2020/080136, 2020/0277663, 2020/024641, 2019/330617, 2019/264268, 2020/256867, 2020/224244, 2019/194709, 2019/161796, 2019/085383, 2019/055594, 2018/216161, 2018/051322, 2018/0245142, 2017/241911, 2017/089811, 2017/067096, 2017/029875, 2017/0016053, 2016/108458, 2015/000854, 2013/171621, WO 2018/091676, WO 2020/176788, Rodriques et al., Science 363(6434):1463-1467, 2019; Lee et al., Nat. Protoc. 10(3):442-458, 2015; Trejo et al., PLOS ONE 14(2):e0212031, 2019; Chen et al., Science 348(6233):aaa6090, 2015; Gao et al., BMC Biol. 15:50, 2017; and Gupta et al., Nature Biotechnol. 36:1197-1202, 2018; the Visium Spatial Gene Expression Reagent Kits User Guide (e.g., Rev D, dated October 2020), and/or the Visium Spatial Tissue Optimization Reagent Kits User Guide (e.g., Rev D, dated October 2020), both of which are available at the 10x Genomics Support Documentation website, and can be used herein in any combination.

WO 2020/176788 A1 discloses methods for spatial profiling of analytes present in a biological sample. Also provided are methods for using spatially barcoded substrates to detect a biological analyte in a cell culture, an organism, and organoid. Also provided are methods for using spatially barcoded substrates to detect the temporal profile of a biological analyte.

GB 2520765 A discloses a method of detecting a species of nucleic acid in a sample, comprising contacting the sample with a set of probes, wherein each probe specifically recognises a distinct target sequence, providing conditions under which the target sequences are at least partially single stranded, providing conditions for annealing and ligation, under which conditions the probes hybridise to their target sequences and generate ligation products, each ligation product comprising a ligation junction, and detecting a cumulative signal which is a combination of individual signals from all ligation products, wherein detection of the signal indicates the presence of the species of nucleic acid in the sample. Further non-limiting aspects of spatial analysis methodologies and compositions are described herein.

Some general terminologies that may be used in this disclosure can be found in Section (I)(b) of WO 2020/176788 and/or U.S. Patent Application Publication No. 2020/0277663. Typically, a "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes. For the purpose of this disclosure, an "analyte" can include any biological substance, structure, moiety, or component to be analyzed. The term "target" can similarly refer to an analyte of interest.

Analytes can be broadly classified into one of two groups: nucleic acid analytes, and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral proteins (e.g., viral capsid, viral envelope, viral coat, viral accessory, viral glycoproteins, viral spike, etc.), extracellular and intracellular proteins, antibodies, and antigen binding fragments. In some embodiments, the analyte(s) can be localized to subcellular location(s), including, for example, organelles, e.g., mitochondria, Golgi apparatus, endoplasmic reticulum, chloroplasts, endocytic vesicles, exocytic vesicles, vacuoles, lysosomes, etc. In some embodiments, analyte(s) can be peptides or proteins, including without limitation antibodies and enzymes. Additional examples of analytes can be found in Section (I)(c) of WO 2020/176788 and/or U.S. Patent Application Publication No. 2020/0277663. In some embodiments, an analyte can be detected indirectly, such as through detection of an intermediate agent, for example, a connected probe (e.g., a ligation product) or an analyte capture agent (e.g., an oligonucleotide-conjugated antibody), such as those described herein.

A "biological sample" is typically obtained from the subject for analysis using any of a variety of techniques including, but not limited to, biopsy, surgery, and laser capture microscopy (LCM), and generally includes cells and/or other biological material from the subject. In some embodiments, a biological sample can be a tissue section. In some embodiments, a biological sample can be a fixed and/or stained biological sample (e.g., a fixed and/or stained tissue section). Non-limiting examples of stains include histological stains (e.g., hematoxylin and/or eosin) and immunological stains (e.g., fluorescent stains). In some embodiments, a biological sample (e.g., a fixed and/or stained biological sample) can be imaged. Biological samples are also described in Section (I)(d) of WO 2020/176788 and/or U.S. Patent Application Publication No. 2020/0277663.

In some embodiments, a biological sample is permeabilized with one or more permeabilization reagents. For example, permeabilization of a biological sample can facilitate analyte capture. Exemplary permeabilization agents and conditions are described in Section (I)(d)(ii)(13) or the Exemplary Embodiments Section of WO 2020/176788 and/or U.S. Patent Application Publication No. 2020/0277663.

Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of the analytes within the biological sample. The spatial location of an analyte within the biological sample is determined based on the feature to which the analyte is bound (e.g., directly or indirectly) on the array, and the feature's relative spatial location within the array.

A "capture probe" refers to any molecule capable of capturing (directly or indirectly) and/or labelling an analyte (e.g., an analyte of interest) in a biological sample. In some embodiments, the capture probe is a nucleic acid or a polypeptide. In some embodiments, the capture probe includes a barcode (e.g., a spatial barcode and/or a unique molecular identifier (UMI)) and a capture domain). In some embodiments, a capture probe can include a cleavage domain and/or a functional domain (e.g., a primer-binding site, such as for next-generation sequencing (NGS)).

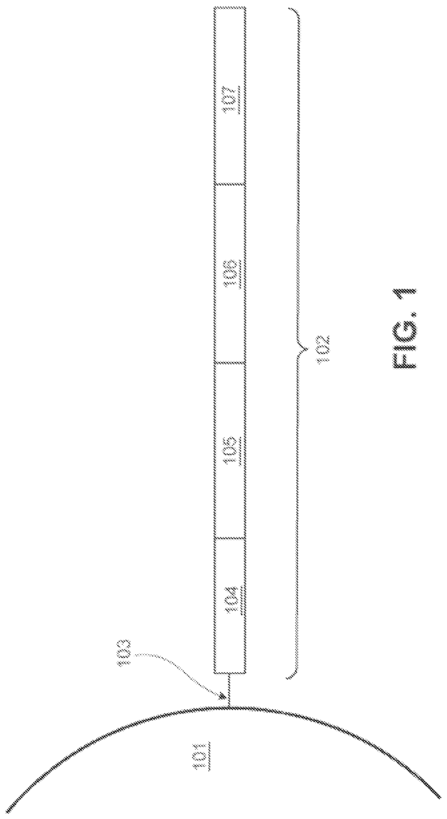

**FIG. 1** is a schematic diagram showing an exemplary capture probe, as described herein. As shown, the capture probe **102** is optionally coupled to a feature **101** by a cleavage domain **103,** such as a disulfide linker. The capture probe can include a functional sequence **104** that is useful for subsequent processing. The functional sequence **104** can include all or a part of sequencer specific flow cell attachment sequence (e.g., a P5 or P7 sequence), all or a part of a sequencing primer sequence, (e.g., a R1 primer binding site, a R2 primer binding site), or combinations thereof. The capture probe can also include a spatial barcode **105.** The capture probe can also include a unique molecular identifier (UMI) sequence **106.** While **FIG. 1** shows the spatial barcode **105** as being located upstream (5') of UMI sequence **106,** it is to be understood that capture probes wherein UMI sequence **106** is located upstream (5') of the spatial barcode **105** is also suitable for use in any of the methods described herein. The capture probe can also include a capture domain **107** to facilitate capture of a target analyte. The capture domain can have a sequence complementary to a sequence of a nucleic acid analyte. The capture domain can have a sequence complementary to a connected probe described herein. The capture domain can have a sequence complementary to a capture handle sequence present in an analyte capture agent. The capture domain can have a sequence complementary to a splint oligonucleotide. Such splint oligonucleotide, in addition to having a sequence complementary to a capture domain of a capture probe, can have a sequence of a nucleic acid analyte, a sequence complementary to a portion of a connected probe described herein, and/or a capture handle sequence described herein.

The functional sequences can generally be selected for compatibility with any of a variety of different sequencing systems, e.g., Ion Torrent Proton or PGM, Illumina sequencing instruments, PacBio, Oxford Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the spatial barcode **105** and functional sequences **104** are common to all of the probes attached to a given feature. In some embodiments, the UMI sequence **106** of a capture probe attached to a given feature is different from the UMI sequence of a different capture probe attached to the given feature.

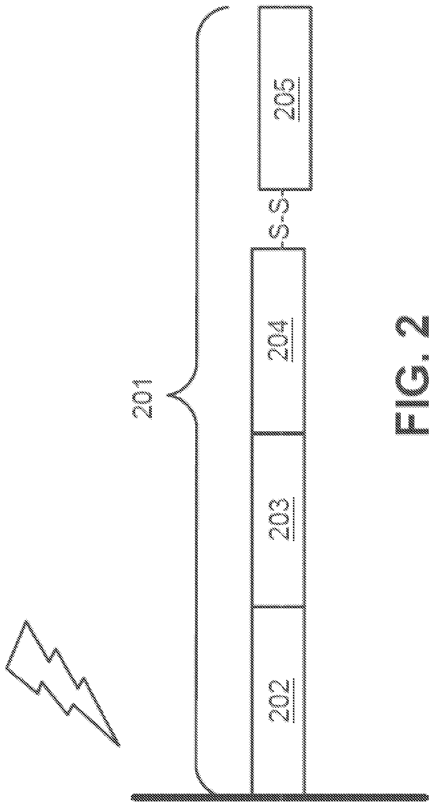

**FIG.** 2 is a schematic illustrating a cleavable capture probe, wherein the cleaved capture probe can enter into a non-permeabilized cell and bind to analytes within the sample. The capture probe **201** contains a cleavage domain **202,** a cell penetrating peptide **203,** a reporter molecule **204,** and a disulfide bond (-S-S-). **205** represents all other parts of a capture probe, for example a spatial barcode and a capture domain.

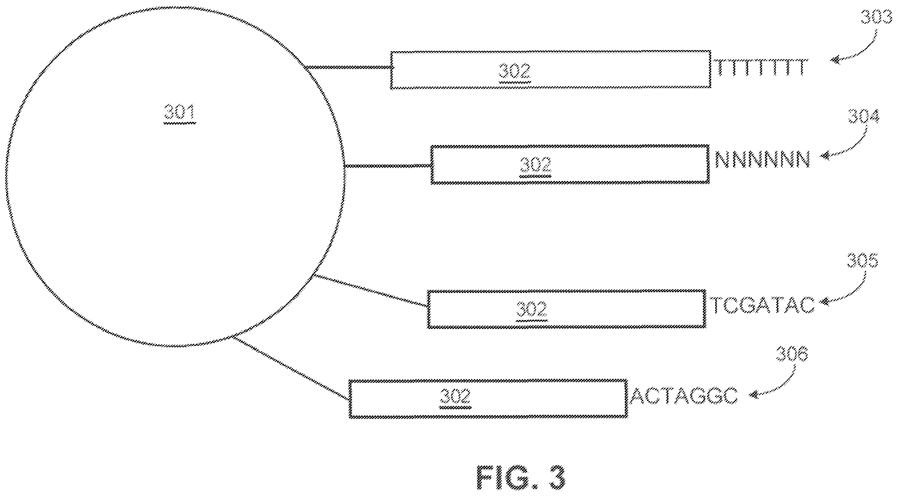

**FIG. 3** is a schematic diagram of an exemplary multiplexed spatially-barcoded feature. In **FIG. 3****,** the feature **301** can be coupled to spatially-barcoded capture probes, wherein the spatially-barcoded probes of a particular feature can possess the same spatial barcode, but have different capture domains designed to associate the spatial barcode of the feature with more than one target analyte. For example, a feature may be coupled to four different types of spatially-barcoded capture probes, each type of spatially-barcoded capture probe possessing the spatial barcode **302.** One type of capture probe associated with the feature includes the spatial barcode **302** in combination with a poly(T) capture domain **303,** designed to capture mRNA target analytes. A second type of capture probe associated with the feature includes the spatial barcode **302** in combination with a random N-mer capture domain **304** for gDNA analysis. A third type of capture probe associated with the feature includes the spatial barcode **302** in combination with a capture domain complementary to a capture handle sequence of an analyte capture agent of interest **305.** A fourth type of capture probe associated with the feature includes the spatial barcode **302** in combination with a capture domain that can specifically bind a nucleic acid molecule **306** that can function in a CRISPR assay (e.g., CRISPR/Cas9). While only four different capture probe-barcoded constructs are shown in **FIG. 3****,** capture-probe barcoded constructs can be tailored for analyses of any given analyte associated with a nucleic acid and capable of binding with such a construct. For example, the schemes shown in **FIG. 3** can also be used for concurrent analysis of other analytes disclosed herein, including, but not limited to: (a) mRNA, a lineage tracing construct, cell surface or intracellular proteins and metabolites, and gDNA; (b) mRNA, accessible chromatin (e.g., ATAC-seq, DNase-seq, and/or MNase-seq) cell surface or intracellular proteins and metabolites, and a perturbation agent (e.g., a CRISPR crRNA/sgRNA, TALEN, zinc finger nuclease, and/or antisense oligonucleotide as described herein); (c) mRNA, cell surface or intracellular proteins and/or metabolites, a barcoded labelling agent (e.g., the MHC multimers described herein), and a V(D)J sequence of an immune cell receptor (e.g., T-cell receptor). In some embodiments, a perturbation agent can be a small molecule, an antibody, a drug, an aptamer, a miRNA, a physical environmental (e.g., temperature change), or any other known perturbation agents. See, e.g., Section (II)(b) (e.g., subsections (i)-(vi)) of WO 2020/176788 and/or U.S. Patent Application Publication No. 2020/0277663. Generation of capture probes can be achieved by any appropriate method, including those described in Section (II)(d)(ii) of WO 2020/176788 and/or U.S. Patent Application Publication No. 2020/0277663.

In some embodiments, more than one analyte type (e.g., nucleic acids and proteins) from a biological sample can be detected (e.g., simultaneously or sequentially) using any appropriate multiplexing technique, such as those described in Section (IV) of WO 2020/176788 and/or U.S. Patent Application Publication No. 2020/0277663.

In some embodiments, detection of one or more analytes (e.g., protein analytes) can be performed using one or more analyte capture agents. As used herein, an "analyte capture agent" refers to an agent that interacts with an analyte (e.g., an analyte in a biological sample) and with a capture probe (e.g., a capture probe attached to a substrate or a feature) to identify the analyte. In some embodiments, the analyte capture agent includes: (i) an analyte binding moiety (e.g., that binds to an analyte), for example, an antibody or antigen-binding fragment thereof; (ii) analyte binding moiety barcode; and (iii) a capture handle sequence. As used herein, the term "analyte binding moiety barcode" refers to a barcode that is associated with or otherwise identifies the analyte binding moiety. As used herein, the term "analyte capture sequence" or "capture handle sequence" refers to a region or moiety configured to hybridize to, bind to, couple to, or otherwise interact with a capture domain of a capture probe. In some embodiments, a capture handle sequence is complementary to a capture domain of a capture probe. In some cases, an analyte binding moiety barcode (or portion thereof) may be able to be removed (e.g., cleaved) from the analyte capture agent.

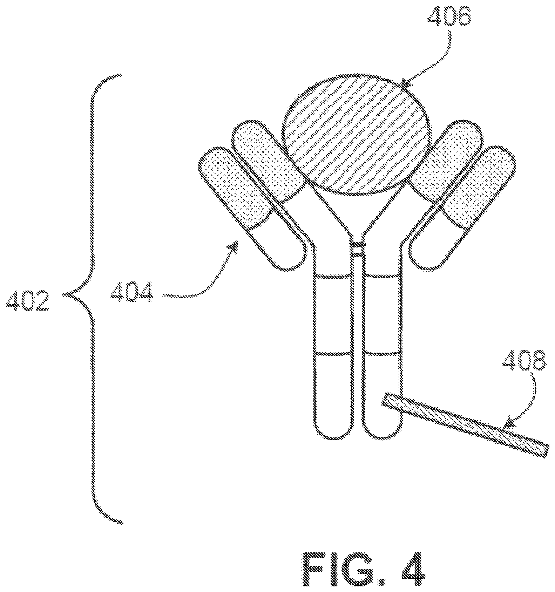

**FIG. 4** is a schematic diagram of an exemplary analyte capture agent **402** comprised of an analyte-binding moiety **404** and an analyte-binding moiety barcode domain **408.** The exemplary analyte -binding moiety **404** is a molecule capable of binding to an analyte **406** and the analyte capture agent is capable of interacting with a spatially-barcoded capture probe. The analyte -binding moiety can bind to the analyte **406** with high affinity and/or with high specificity. The analyte capture agent can include an analyte -binding moiety barcode domain **408,** a nucleotide sequence (e.g., an oligonucleotide), which can hybridize to at least a portion or an entirety of a capture domain of a capture probe. The analyte-binding moiety barcode domain 408 can comprise an analyte binding moiety barcode and a capture handle sequence described herein. The analyte -binding moiety **404** can include a polypeptide and/or an aptamer. The analyte -binding moiety **404** can include an antibody or antibody fragment (e.g., an antigen-binding fragment).

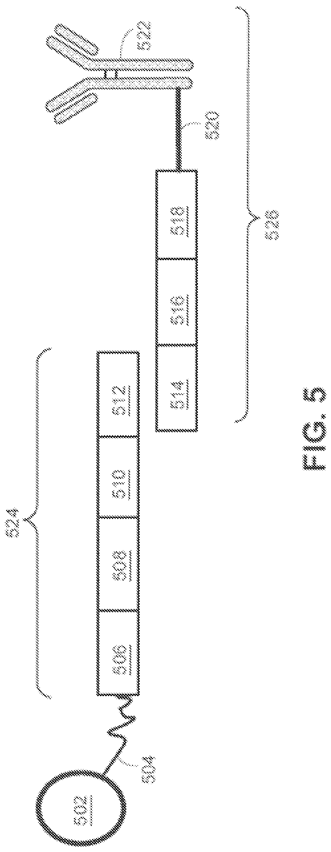

**FIG. 5** is a schematic diagram depicting an exemplary interaction between a feature-immobilized capture probe **524** and an analyte capture agent **526.** The feature-immobilized capture probe **524** can include a spatial barcode **508** as well as functional sequences **506** and UMI **510,** as described elsewhere herein. The capture probe can also include a capture domain **512** that is capable of binding to an analyte capture agent **526.** The analyte capture agent **526** can include a functional sequence **518,** analyte binding moiety barcode **516,** and a capture handle sequence **514** that is capable of binding to the capture domain **512** of the capture probe **524.** The analyte capture agent can also include a linker **520** that allows the capture agent barcode domain **516** to couple to the analyte binding moiety **522.**

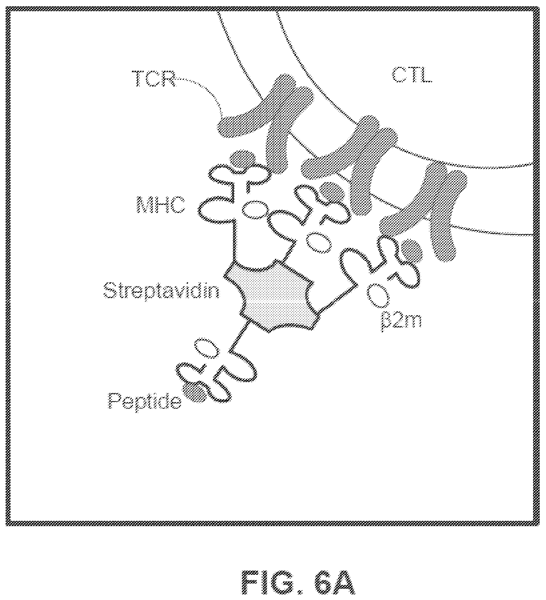

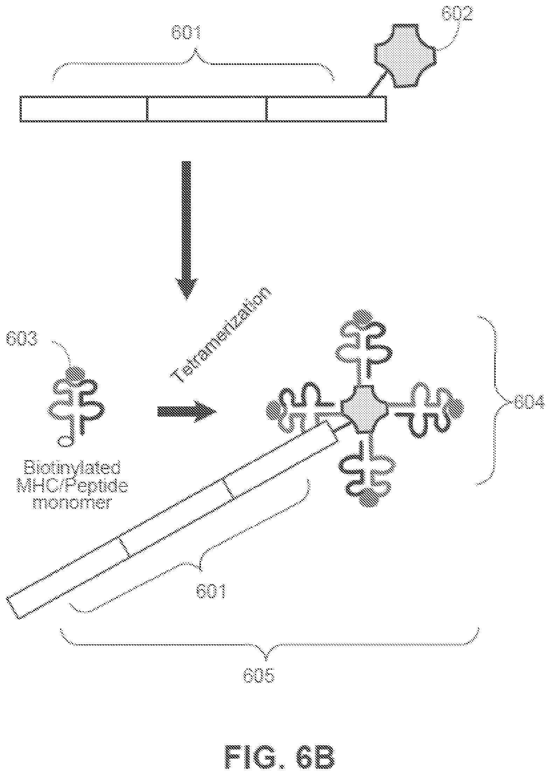

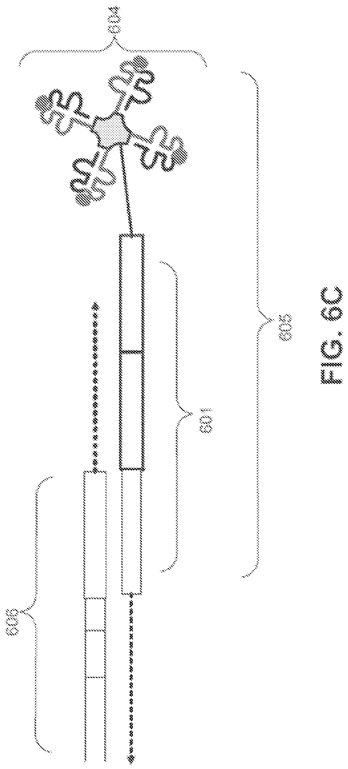

**FIGs. 6A****,** **6B****,** and **6C** are schematics illustrating how streptavidin cell tags can be utilized in an array-based system to produce a spatially-barcoded cell or cellular contents. For example, as shown in **FIG. 6A****,** peptide-bound major histocompatibility complex (MHC) can be individually associated with biotin (β2m) and bound to a streptavidin moiety such that the streptavidin moiety comprises multiple pMHC moieties. Each of these moieties can bind to a TCR such that the streptavidin binds to a target T-cell via multiple MHC/TCR binding interactions. Multiple interactions synergize and can substantially improve binding affinity. Such improved affinity can improve labelling of T-cells and also reduce the likelihood that labels will dissociate from T-cell surfaces. As shown in **FIG. 6B****,** a capture agent barcode domain **601** can be modified with streptavidin **602** and contacted with multiple molecules of biotinylated MHC **603** such that the biotinylated MHC **603** molecules are coupled with the streptavidin conjugated capture agent barcode domain **601,** thereby forming a tetramerized MHC complex **604.** The result is a barcoded MHC multimer complex **605.** As shown in **FIG. 6B****,** the capture agent barcode domain sequence **601** can identify the MHC as its associated label and also includes optional functional sequences such as sequences for hybridization with other oligonucleotides. As shown in **FIG. 6C****,** one example oligonucleotide is capture probe **606** that comprises a complementary sequence (e.g., rGrGrG corresponding to C C C), a barcode sequence and other functional sequences, such as, for example, a UMI, an adapter sequence (e.g., comprising a sequencing primer sequence (e.g., R1 or a partial R1 ("pR1"), R2), a flow cell attachment sequence (e.g., P5 or P7 or partial sequences thereof)), etc. In some cases, capture probe **606** may at first be associated with a feature (e.g., a gel bead) and released from the feature. In other embodiments, capture probe **606** can hybridize with a capture agent barcode domain **601** of the MHC-oligonucleotide complex **605.** The hybridized oligonucleotides (Spacer C C C and Spacer rGrGrG) can then be extended in primer extension reactions such that constructs comprising sequences that correspond to each of the two spatial barcode sequences (the spatial barcode associated with the capture probe, and the barcode associated with the MHC-oligonucleotide complex) are generated. In some cases, one or both of the corresponding sequences may be a complement of the original sequence in capture probe **606** or capture agent barcode domain **601.** In other embodiments, the capture probe and the capture agent barcode domain are ligated together. The resulting constructs can be optionally further processed (e.g., to add any additional sequences and/or for clean-up) and subjected to sequencing. As described elsewhere herein, a sequence derived from the capture probe **606** spatial barcode sequence may be used to identify a feature and the sequence derived from spatial barcode sequence on the capture agent barcode domain **601** may be used to identify the particular peptide MHC complex **604** bound on the surface of the cell (e.g., when using MHC-peptide libraries for screening immune cells or immune cell populations).

Additional description of analyte capture agents can be found in Section (II)(b)(ix) of WO 2020/176788 and/or Section (II)(b)(viii) U.S. Patent Application Publication No. 2020/0277663.

There are at least two methods to associate a spatial barcode with one or more neighboring cells, such that the spatial barcode identifies the one or more cells, and/or contents of the one or more cells, as associated with a particular spatial location. One method is to promote analytes or analyte proxies (e.g., intermediate agents) out of a cell and towards a spatially-barcoded array (e.g., including spatially-barcoded capture probes). Another method is to cleave spatially-barcoded capture probes from an array and promote the spatially-barcoded capture probes towards and/or into or onto the biological sample.

In some cases, capture probes may be configured to prime, replicate, and consequently yield optionally barcoded extension products from a template (e.g., a DNA or RNA template, such as an analyte or an intermediate agent (e.g., a connected probe (e.g., a ligation product) or an analyte capture agent), or a portion thereof), or derivatives thereof (see, e.g., Section (II)(b)(vii) of WO 2020/176788 and/or U.S. Patent Application Publication No. 2020/0277663 regarding extended capture probes). In some cases, capture probes may be configured to form a connected probe (e.g., a ligation product) with a template (e.g., a DNA or RNA template, such as an analyte or an intermediate agent, or portion thereof), thereby creating ligations products that serve as proxies for a template.

As used herein, an "extended capture probe" refers to a capture probe having additional nucleotides added to the terminus (e.g., 3' or 5' end) of the capture probe thereby extending the overall length of the capture probe. For example, an "extended 3' end" indicates additional nucleotides were added to the most 3' nucleotide of the capture probe to extend the length of the capture probe, for example, by polymerization reactions used to extend nucleic acid molecules including templated polymerization catalyzed by a polymerase (e.g., a DNA polymerase or a reverse transcriptase). In some embodiments, extending the capture probe includes adding to a 3' end of a capture probe a nucleic acid sequence that is complementary to a nucleic acid sequence of an analyte or intermediate agent specifically bound to the capture domain of the capture probe. In some embodiments, the capture probe is extended using reverse transcription. In some embodiments, the capture probe is extended using one or more DNA polymerases. The extended capture probes include the sequence of the capture probe and the sequence of the spatial barcode of the capture probe.

In some embodiments, extended capture probes are amplified (e.g., in bulk solution or on the array) to yield quantities that are sufficient for downstream analysis, e.g., via DNA sequencing. In some embodiments, extended capture probes (e.g., DNA molecules) act as templates for an amplification reaction (e.g., a polymerase chain reaction). Additional variants of spatial analysis methods, including in some embodiments, an imaging step, are described in Section (II)(a) of WO 2020/176788 and/or U.S. Patent Application Publication No. 2020/0277663. Analysis of captured analytes (and/or intermediate agents or portions thereof), for example, including sample removal, extension of capture probes, sequencing (e.g., of a cleaved extended capture probe and/or a nucleic acid molecule complementary to an extended capture probe), sequencing on the array (e.g., using, for example, in situ hybridization or in situ ligation approaches), temporal analysis, and/or proximity capture, is described in Section (II)(g) of WO 2020/176788 and/or U.S. Patent Application Publication No. 2020/0277663. Some quality control measures are described in Section (II)(h) of WO 2020/176788 and/or U.S. Patent Application Publication No. 2020/0277663.
Spatial information can provide information of biological and/or medical importance. For example, the methods and compositions described herein can allow for: identification of one or more biomarkers (e.g., diagnostic, prognostic, and/or for determination of efficacy of a treatment) of a disease or disorder; identification of a candidate drug target for treatment of a disease or disorder; identification (e.g., diagnosis) of a subject as having a disease or disorder; identification of stage and/or prognosis of a disease or disorder in a subject; identification of a subject as having an increased likelihood of developing a disease or disorder; monitoring of progression of a disease or disorder in a subject; determination of efficacy of a treatment of a disease or disorder in a subject; identification of a patient subpopulation for which a treatment is effective for a disease or disorder; modification of a treatment of a subject with a disease or disorder; selection of a subject for participation in a clinical trial; and/or selection of a treatment for a subject with a disease or disorder.

Spatial information can provide information of biological importance. For example, the methods and compositions described herein can allow for: identification of transcriptome and/or proteome expression profiles (e.g., in healthy and/or diseased tissue); identification of multiple analyte types in close proximity (e.g., nearest neighbor analysis); determination of up- and/or down-regulated genes and/or proteins in diseased tissue; characterization of tumor microenvironments; characterization of tumor immune responses; characterization of cells types and their co-localization in tissue; and identification of genetic variants within tissues (e.g., based on gene and/or protein expression profiles associated with specific disease or disorder biomarkers).

Typically, for spatial array-based methods, a substrate functions as a support for direct or indirect attachment of capture probes to features of the array. A "feature" is an entity that acts as a support or repository for various molecular entities used in spatial analysis. In some embodiments, some or all of the features in an array are functionalized for analyte capture. Exemplary substrates are described in Section (II)(c) of WO 2020/176788 and/or U.S. Patent Application Publication No. 2020/0277663. Exemplary features and geometric attributes of an array can be found in Sections (II)(d)(i), (II)(d)(iii), and (II)(d)(iv) of WO 2020/176788 and/or U.S. Patent Application Publication No. 2020/0277663.

Generally, analytes and/or intermediate agents (or portions thereof) can be captured when contacting a biological sample with a substrate including capture probes (e.g., a substrate with capture probes embedded, spotted, printed, fabricated on the substrate, or a substrate with features (e.g., beads, wells) comprising capture probes). As used herein, "contact," "contacted," and/or "contacting," a biological sample with a substrate refers to any contact (e.g., direct or indirect) such that capture probes can interact (e.g., bind covalently or non-covalently (e.g., hybridize)) with analytes from the biological sample. Capture can be achieved actively (e.g., using electrophoresis) or passively (e.g., using diffusion). Analyte capture is further described in Section (II)(e) of WO 2020/176788 and/or U.S. Patent Application Publication No. 2020/0277663.

In some cases, spatial analysis can be performed by attaching and/or introducing a molecule (e.g., a peptide, a lipid, or a nucleic acid molecule) having a barcode (e.g., a spatial barcode) to a biological sample (e.g., to a cell in a biological sample). In some embodiments, a plurality of molecules (e.g., a plurality of nucleic acid molecules) having a plurality of barcodes (e.g., a plurality of spatial barcodes) are introduced to a biological sample (e.g., to a plurality of cells in a biological sample) for use in spatial analysis. In some embodiments, after attaching and/or introducing a molecule having a barcode to a biological sample, the biological sample can be physically separated (e.g., dissociated) into single cells or cell groups for analysis. Some such methods of spatial analysis are described in Section (III) of WO 2020/176788 and/or U.S. Patent Application Publication No. 2020/0277663.

In some cases, spatial analysis can be performed by detecting multiple oligonucleotides that hybridize to an analyte. In some instances, for example, spatial analysis can be performed using templated ligation, or RNA-templated ligation (RTL). Methods of templated ligation, or RTL, have been described previously. See, e.g., Credle et al., Nucleic Acids Res. 2017 Aug 21;45(14):e128. Typically, templated ligation includes hybridization of two oligonucleotides to adjacent sequences on an analyte (e.g., an RNA molecule, such as an mRNA molecule). In some instances, the oligonucleotides are DNA molecules. In some instances, one of the oligonucleotides includes at least two ribonucleic acid bases at the 3' end and/or the other oligonucleotide includes a phosphorylated nucleotide at the 5' end. In some instances, one of the two oligonucleotides includes a capture domain (e.g., a poly(A) sequence, a non-homopolymeric sequence). After hybridization to the analyte, a ligase (e.g., SplintR ligase) ligates the two oligonucleotides together, creating a connected probe (e.g., a ligation product). In some instances, the two oligonucleotides hybridize to sequences that are not adjacent to one another. For example, hybridization of the two oligonucleotides creates a gap between the hybridized oligonucleotides. In some instances, a polymerase (e.g., a DNA polymerase) can extend one of the oligonucleotides prior to ligation. After ligation, the connected probe (e.g., a ligation product) is released from the analyte. In some instances, the connected probe (e.g., a ligation product) is released using an endonuclease (e.g., RNAse H). The released connected probe (e.g., a ligation product) can then be captured by capture probes (e.g., instead of direct capture of an analyte) on an array, optionally amplified, and sequenced, thus determining the location and optionally the abundance of the analyte in the biological sample.

During analysis of spatial information, sequence information for a spatial barcode associated with an analyte is obtained, and the sequence information can be used to provide information about the spatial distribution of the analyte in the biological sample. Various methods can be used to obtain the spatial information. In some embodiments, specific capture probes and the analytes they capture are associated with specific locations in an array of features on a substrate. For example, specific spatial barcodes can be associated with specific array locations prior to array fabrication, and the sequences of the spatial barcodes can be stored (e.g., in a database) along with specific array location information, so that each spatial barcode uniquely maps to a particular array location.

Alternatively, specific spatial barcodes can be deposited at predetermined locations in an array of features during fabrication such that at each location, only one type of spatial barcode is present so that spatial barcodes are uniquely associated with a single feature of the array. Where necessary, the arrays can be decoded using any of the methods described herein so that spatial barcodes are uniquely associated with array feature locations, and this mapping can be stored as described above.

When sequence information is obtained for capture probes and/or analytes during analysis of spatial information, the locations of the capture probes and/or analytes can be determined by referring to the stored information that uniquely associates each spatial barcode with an array feature location. In this manner, specific capture probes and captured analytes are associated with specific locations in the array of features. Each array feature location represents a position relative to a coordinate reference point (e.g., an array location, a fiducial marker) for the array. Accordingly, each feature location has an "address" or location in the coordinate space of the array.

Some exemplary spatial analysis workflows are described in the Exemplary Embodiments section of WO 2020/176788 and/or U.S. Patent Application Publication No. 2020/0277663. See, for example, the Exemplary embodiment starting with "In some non-limiting examples of the workflows described herein, the sample can be immersed..." of WO 2020/176788 and/or U.S. Patent Application Publication No. 2020/0277663. See also, e.g., the Visium Spatial Gene Expression Reagent Kits User Guide (e.g., Rev D, dated October 2020), and/or the Visium Spatial Tissue Optimization Reagent Kits User Guide (e.g., Rev D, dated October 2020). In some embodiments, spatial analysis can be performed using dedicated hardware and/or software, such as any of the systems described in Sections (II)(e)(ii) and/or (V) of WO 2020/176788 and/or U.S. Patent Application Publication No. 2020/0277663, or any of one or more of the devices or methods described in Sections *Control Slide for Imaging, Methods of Using Control Slides and Substrates for, Systems of Using Control Slides and Substrates for Imaging,* and/or *Sample and Array Alignment Devices and Methods, Informational labels* of WO 2020/123320.

Suitable systems for performing spatial analysis can include components such as a chamber (e.g., a flow cell or sealable, fluid-tight chamber) for containing a biological sample. The biological sample can be mounted for example, in a biological sample holder. One or more fluid chambers can be connected to the chamber and/or the sample holder via fluid conduits, and fluids can be delivered into the chamber and/or sample holder via fluidic pumps, vacuum sources, or other devices coupled to the fluid conduits that create a pressure gradient to drive fluid flow. One or more valves can also be connected to fluid conduits to regulate the flow of reagents from reservoirs to the chamber and/or sample holder.

The systems can optionally include a control unit that includes one or more electronic processors, an input interface, an output interface (such as a display), and a storage unit (e.g., a solid state storage medium such as, but not limited to, a magnetic, optical, or other solid state, persistent, writeable and/or re-writeable storage medium). The control unit can optionally be connected to one or more remote devices via a network. The control unit (and components thereof) can generally perform any of the steps and functions described herein. Where the system is connected to a remote device, the remote device (or devices) can perform any of the steps or features described herein. The systems can optionally include one or more detectors (e.g., CCD, CMOS) used to capture images. The systems can also optionally include one or more light sources (e.g., LED-based, diode-based, lasers) for illuminating a sample, a substrate with features, analytes from a biological sample captured on a substrate, and various control and calibration media.

The systems can optionally include software instructions encoded and/or implemented in one or more of tangible storage media and hardware components such as application specific integrated circuits. The software instructions, when executed by a control unit (and in particular, an electronic processor) or an integrated circuit, can cause the control unit, integrated circuit, or other component executing the software instructions to perform any of the method steps or functions described herein.

In some cases, the systems described herein can detect (e.g., register an image) the biological sample on the array. Exemplary methods to detect the biological sample on an array are described in WO2021102003A1 and/or US20210150707A1.

Prior to transferring analytes from the biological sample to the array of features on the substrate, the biological sample can be aligned with the array. Alignment of a biological sample and an array of features including capture probes can facilitate spatial analysis, which can be used to detect differences in analyte presence and/or level within different positions in the biological sample, for example, to generate a three-dimensional map of the analyte presence and/or level. Exemplary methods to generate a two- and/or three-dimensional map of the analyte presence and/or level are described in PCT Application Publication No. WO 2021/067514 and spatial analysis methods are generally described in WO 2020/061108 and/or US20210155982A1.

In some cases, a map of analyte presence and/or level can be aligned to an image of a biological sample using one or more fiducial markers, e.g., objects placed in the field of view of an imaging system which appear in the image produced, as described in the *Substrate Attributes* Section, *Control Slide for Imaging* Section of WO 2020/123320, WO2021102005A1, and/or US20210158522A1. Fiducial markers can be used as a point of reference or measurement scale for alignment (e.g., to align a sample and an array, to align two substrates, to determine a location of a sample or array on a substrate relative to a fiducial marker) and/or for quantitative measurements of sizes and/or distances.

### II. Targeted Probe Silencing in Templated Ligation Using Blocker Oligonucleotides

Targeted templated ligation probe silencing allows for reduced occurrence of hybridization between one or more templated ligation probes and corresponding analytes in a biological sample, thereby reducing the amount of ligated products generated from a defined templated ligation probe set. Subsequent or concurrent application of capture probes to the sample can result in targeted, improved, and/or increased capture of the ligation products released from other types of analytes (e.g., desired RNA molecules), ligation products from templated ligation probes that are not targeted with blocker oligonucleotides. To silence the one or more templated ligation probes, one or more blocker oligonucleotides are designed that hybridize to the one or more templated ligation probes. In some embodiments, a selected group of blocker oligonucleotides can be added to a pool of templated ligation probes (any combinations of the first probes, the second probes, the spanning probes, and/or the third oligonucleotides described herein) to hybridize with one or more templated ligation probes within the pool, thereby selectively decreasing the sequencing output of those analytes targeted by the one or more templated ligation probes, i.e., silencing the templated ligation probes.

The methods provided herein utilize one or more blocker oligonucleotides that target one or both of the templated ligation probes in a pair of ligation probes (or sets; the terms are interchangeable). In some instances, a blocker oligonucleotide is designed to target (e.g., hybridize to) one of the templated ligation probes in a pair of ligation probes. In some instances, two blocker oligonucleotides target each templated ligation probe in a pair of ligation probes (e.g., one blocker oligonucleotide targets a first probe and another blocker oligonucleotide targets a second probe). In some instances, the blocker oligonucleotides are designed so that each blocker oligonucleotide (e.g., antisense oligonucleotide) hybridizes to a sequence in a templated ligation probe that is substantially complementary to its target analyte. That is, in some instances, a single blocker oligonucleotide pair can be specific to a single templated ligation probe pair. For example, a first blocker oligonucleotide can hybridize to a first probe described herein, and a second blocker oligonucleotide can hybridize to a second probe described herein. In some embodiments, blocker oligonucleotides are designed to hybridize to any one or more of the first probes, the second probes, the spanning probes, and/or the third oligonucleotides described herein.

In some embodiments, methods described herein can decrease the concentration of a ligation product generated from one or more probes by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a reference biological sample to which blocker oligonucleotides have not been added. In some embodiments, methods described herein can increase the concentration of one or more ligation products that are captured by a spatial array by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, or more compared to a reference biological sample to which blocker oligonucleotides have not been added.

### (a) Templated Ligation Background

Although techniques such as whole genome sequencing and whole exome sequencing are available, these techniques can have drawbacks in that they might provide a lot of unnecessary information and increase costs for an experiment. In situations where one prefers to examine a more limited number of analytes, methods herein are provided for targeted RNA capture. Capturing a derivative of an analyte (e.g., a ligation product) provides enhanced specificity with respect to detection of an analyte. This is because at least two probes specific for a target are required to hybridize to the target and facilitate ligation and ultimate capture of a proxy of the target nucleic acid.

Targeted RNA capture is an attractive alternative to poly(A) mRNA capture to interrogate spatial gene expression in a sample (e.g., an FFPE tissue). Compared to poly(A) mRNA capture, targeted RNA capture as described herein is less affected by RNA degradation associated with FFPE fixation compared to methods dependent on oligo-dT capture and reverse transcription of mRNA. Further targeted RNA capture as described herein allows for sensitive measurement of specific genes of interest that otherwise might be missed with a whole transcriptomic approach. Targeted RNA capture can be used to capture a defined set of RNA molecules of interest, or it can be used at a whole transcriptome level, or anything in between. When combined with the spatial methods disclosed herein, the location and abundance of the RNA targets can be determined.

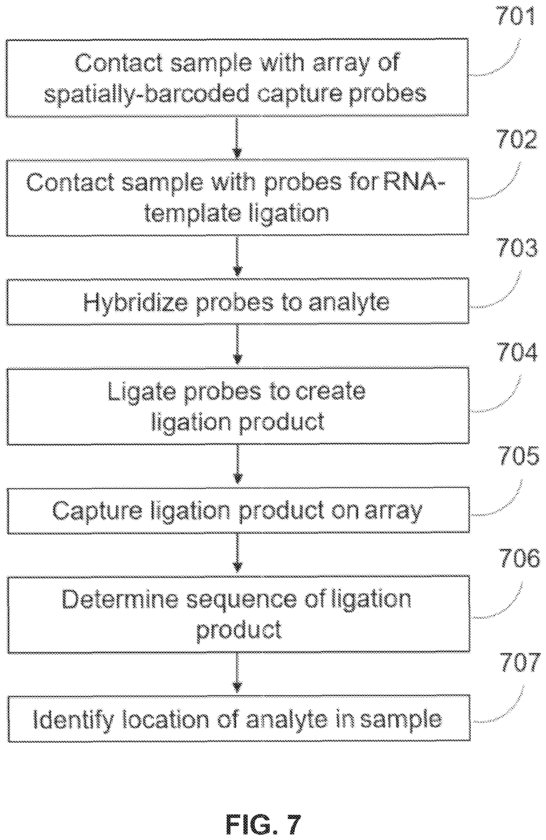

Referring to **FIG. 7****,** in an exemplary embodiment of the disclosure, provided are methods for identifying a location of an analyte in a biological sample. In some instances, the methods include **701** contacting a biological sample, or analytes or proxies of an analyte from a biological sample, with an array of spatially-barcoded capture probes. In some instances, the array is on a substrate and the array includes a plurality of capture probes, wherein a capture probe of the plurality includes: (i) a spatial barcode and (ii) a capture domain. After placing the biological sample on the array, the biological sample **702** is contacted with a first probe and a second probe, wherein the first probe and the second probe each include one or more sequences that are substantially complementary to sequences of the analyte, and wherein the second probe includes a capture probe capture domain; the first probe and the second probe **703** hybridize to complementary sequences in the analyte. After hybridization a ligation product comprising the first probe and the second probe **704** is generated, and the ligation product is released from the analyte. The liberated ligation product is freed **705** to hybridize to the capture domain of a probe on the array. After capture, (i) all or a part of the sequence of the ligation product specifically bound to the capture domain, or a complement thereof, and (ii) the sequence of the spatial barcode, or a complement thereof **706** can be determined, and then one can use the determined sequence of (i) and (ii) **707** to identify the location of the analyte in the biological sample (i.e., correlate the determined sequences of the spatial barcodes to their known locations on the array thereby identifying the locations of the spatially barcoded associated analyte in the biological sample). Blocker oligonucleotides can be added to the biological sample **702** concurrently with a plurality of templated ligation probes. During hybridization **703,** the blocker oligonucleotides can hybridize to templated ligation probes, thereby blocking their hybridization to their target analyte.

Referring again to **FIG. 7****,** the workflow can be modified such that steps **701, 702, 703** and **704** can be combined and practiced on a a slide containing a biological sample, the slide of which is not arrayed with capture probes. For example, the template ligation probes can be applied to a biological sample on a array-less slide, where the ligation probes hybridize to their target sequences or concurrently with blocker oligonucleotides. The hybridzed template ligation probes can be ligated together and transferred to an arrayed slide, the arrayed slide of which comprises capture probes as previously defined. Such methods are found in, for example, WO2020/123320.

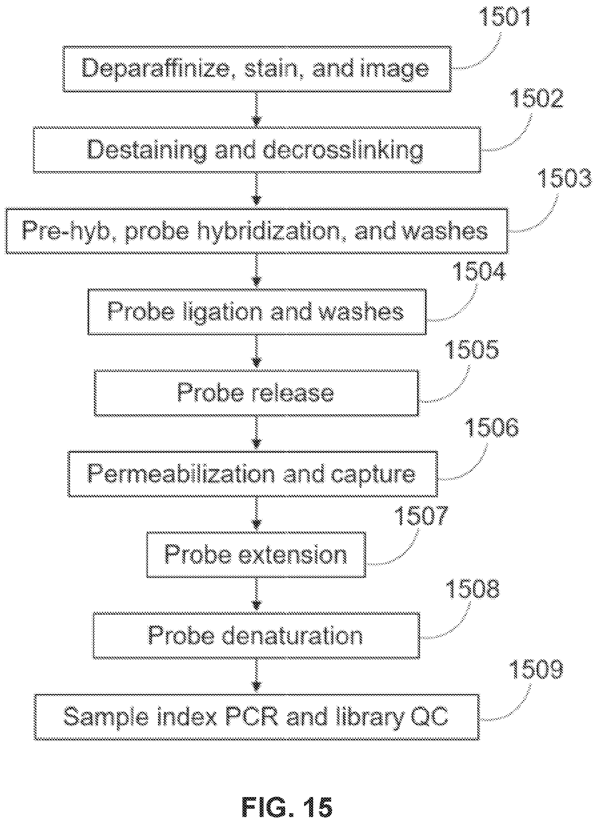

Referring to **FIG. 15****,** in another non-limiting example, a biological sample is deparaffinized, stained, and imaged **1501.** After destaining and optionally decrosslinking **1502,** probes are added to the sample and the probes are hybridized to an analyte **1503.** In some instances, the probes are DNA probes. In some instances, the probes are diribo-containing probes. Probes are ligated **1504** and released using an endonuclease such as RNAse H **1505.** Ligated probes are captured on an array by a capture probe **1506,** extended using a polymerase **1507** and released from the capture probe **1508.** After library preparation **1509,** the abundance and/or location of an analyte is determined. Blocker oligonucleotides can be added to the sample **1503** concurrently with the probes, such that the blocker oligonucleotides can hybridize to their complementary probes, thereby blocking their hybridization to the target analyte.

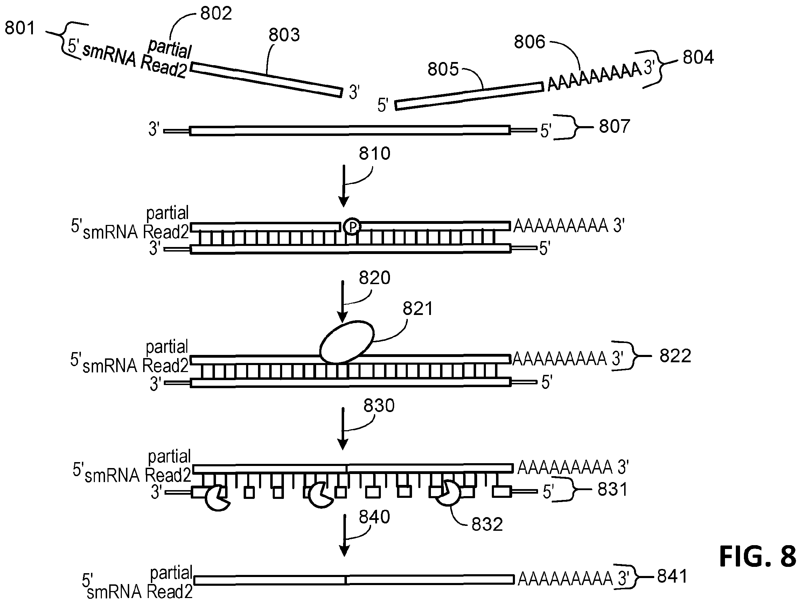

A non-limiting example of the templated ligation methods disclosed herein is depicted in **FIG. 8****.** A biological sample is contacted with a (a) a first probe **801** having a target-hybridization sequence **803** and a primer sequence **802** and (b) a second probe **804** having a target-hybridization sequence **805** and a capture domain (e.g., a poly-A sequence) **806,** the first probe **801** and a second probe **804** hybridize **810** to an analyte **807** in the biological sample. A ligase **821** ligates **820** the first probe to the second probe thereby generating a ligation product **822;** a proxy for the target analyte to which the probes had hybridized. The ligation product is released **830** from the analyte **831** by digesting the analyte using an endoribonuclease **832.** The sample is permeabilized **840** and the ligation product **841** is allowed to hybridize to a capture probe on an arrayed substrate comprising a plurality of capture probes. In situations when an analyte **807** is targeted for exclusion from capture, a first blocker oligonucleotide can be designed to hybridize with the target-hybridization sequence **803** of the first analyte specific probe **801,** and a second blocker oligonucleotide can be designed to hybridize with the target-hybridization sequence **805** of the second analyte specific probe **804.** The blocker oligonucleotides can block the first probe **801** and/or the second probe **804** from hybridizing **810** to the analyte **807** targeted for capture exclusion. Without proper hybridization **810,** none or a minimal amount of the ligation product **822** can be generated from the first probe and the second probe. As a result, the first probe **801** and the second probe **804** are silenced by the blocker oligonucleotides and the proxy ligation products of the analyte targeted for exclusion are greatly minimized or excluded from downstream transcriptomic analysis.

In some embodiments, a first blocker oligonucleotide can be designed to hybridize with the target-hybridization sequence **803** of the first probe **801.** In some embodiments, the first blocker oligonucleotide does not hybridize to the functional sequence **802** of the first probe 801. In some embodiments, the first blocker oligonucleotide only hybridizes to the target-hybridization sequence **803** of the first probe **801,** in order to avoid off-target binding.

In some embodiments, a second blocker oligonucleotide can be designed to hybridize with the target-hybridization sequence **805** of the second probe **804.** In some embodiments, the second blocker oligonucleotide does not hybridize to the capture probe capture domain **806** of the second probe **804.** It is contemplated that including a sequence that is substantially complementary to the capture probe capture domain **806** (e.g., a poly-(A) sequence) in the second blocker oligonucleotide may cause off-target binding. As such, in some embodiments, the second blocker oligonucleotide only hybridizes to the target-hybridization sequence **805** of the second probe **804.**

Also provided herein are methods for identifying a location of an analyte in a biological sample that includes a second probe including a pre-adenylated phosphate group at its 5' end, which enables the ligation step to use a ligase that does not require adenosine triphosphate for ligase activity. Such probes can also be silenced using the methods described herein.

Also provided herein are methods for identifying a location of an analyte in a biological sample that includes one or more spanning probes, in addition to the first and second probes. Using a spanning probe enables greater flexibility in designing templated ligation probes, primarily by increasing the sequences within the analyte that can be used as optional target sequences. In such cases, the one or more spanning probes, together with the first and second probes, can all be silenced using blocker oligonucleotides targeting each probe.

Also provided herein are methods for identifying a location of an analyte in a biological sample that includes optimized hybridizing, washing, and releasing steps.

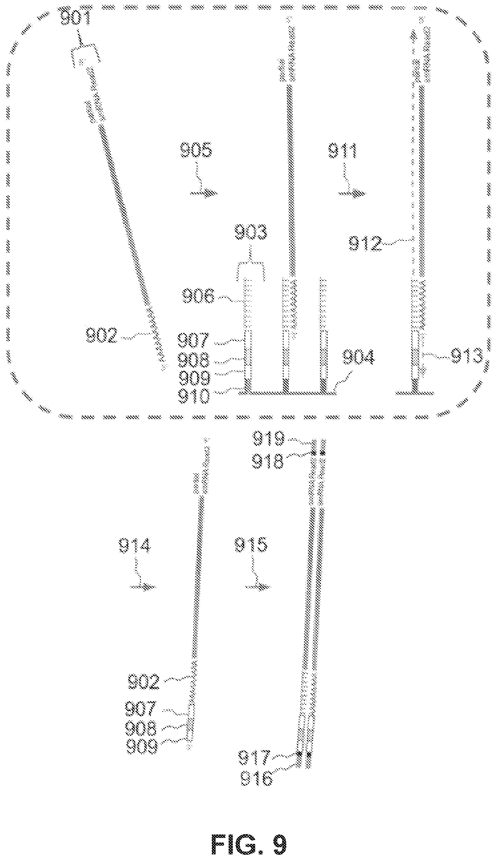

In some embodiments, as shown in **FIG. 9****,** the ligation product **901** includes a capture probe capture domain **902,** which can hybridize to a capture probe **903** (e.g., a capture probe immobilized, directly or indirectly, on a substrate **904).** In some embodiments, methods provided herein include contacting **905** a biological sample with a substrate **904,** wherein the capture probe **903** is affixed to the substrate (e.g., immobilized to the substrate, directly or indirectly). In some embodiments, a biological sample is on a substrate that does not comprise capture probes **903.** In some embodiments, the capture probe capture domain **902** of the ligated product specifically binds to the capture domain **906.** The capture probe can also include a unique molecular identifier (UMI) **907,** a spatial barcode **908,** a functional sequence **909,** and a cleavage domain **910.** In some embodiments, the ligation products generated in a biological sample that is on a slide that does not comprise capture probes are migrated to a substrate that does comprise capture probes as seen in **FIG. 9****.**

In some embodiments, methods provided herein include permeabilization of the biological sample such that the capture probe can more easily hybridize to the ligation product (i.e., compared to no permeabilization). In some embodiments, reverse transcription (RT) reagents can be added to permeabilized biological samples. Incubation with the RT reagents can extend **911the** capture probes **903** to produce spatially-barcoded full-length nucleic acids **912** and **913** from the captured ligation product.

In some embodiments, the extended ligation product/capture probe hybridization complex is denatured **914** and the released extended ligation product is transferred (e.g., to a clean tube) for amplification, and/or library construction. The spatially-barcoded-ligation product can be amplified **915** via PCR prior to library construction. P5 **916,** i5 **917,** i7 **918,** and P7 **919,** and can be used as sample indexes, and TruSeq Read 2 can be added via End Repair, A-tailing, Adaptor Ligation, and PCR. The resulting fragments can then be sequenced using paired-end sequencing using TruSeq Read 1 and TruSeq Read 2 as sequencing primer sites.

### (b) Minimized or Excluded Analyte(s) Capture

In some embodiments, the methods described herein can be used to silence (e.g., via hybridization of antisense oligonucleotides) or minimize the ligation event between one or more templated ligation probes, thereby reducing the sequencing reads of an analyte (e.g., a highly expressed gene transcript) in a templated-ligation-based spatial analysis. Blocking the one or both of the ligation probes targeting an analyte (e.g. via hybridization of antisense oligonucleotides) can reduce or eliminate the amount of ligation products generated from such templated ligation probes, which allows for ligation products generated from other templated ligation probes (e.g., templated ligation probes targeting rare or low expressing analytes) to be captured or sequenced on a spatial array that may have been obscured or swamped out by a highly abundant analyte. Additionally, in a probe pool there may be one or more ligation probes that do not hybridze to just one target analyte sequence, for example there may be ligation probes in a pool of probes that hybridize to their target sequence in addition to hybridizing to off-target sequences. The ability to block these problematic probes in a pool of probes can mitigate off target binding by removing those problematic probes from hybridizing and thereby creating ligation products that could hybridization to capture probes and provide background noise and decrease resolution of the spatial assay. In some embodiments, the analytes to be silenced, minimized, or excluded herein are also refered to as analytes for decreased capture.

For example, a highly expressed gene (e.g., *SERF1A* gene) in a biological sample (e.g., a human brain tissue sample) may be a target for minimizing capture in spatial analysis. The sequences for the highly expressed gene(s) are well known to those skilled in the art and can be readily found in the database (e.g., the Genotype-Tissue Expression (GTEx) Project; or the Human Protein Atlas Project) or literature (e.g., any research or review articles releasing tissue-specific transcriptomics data). The analyte being targeted for reduction in a spatial assay can have a large quantity in a biological sample. For example, the analyte accounts for at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, or at least 50% of all analytes detected (e.g., in a single templated ligation detection) in the biological sample. In some embodiments, an analyte being targeted for reductive capture is not derived from a highly expressed gene in a biological sample. For example, a subpool of templated ligation probes can be selectively silenced to reduce the spatial analysis reads of a corresponding subset of analytes. In some embodiments, the subset of analytes includes mRNAs that share identical or substantially similar sequences, which mRNAs are translated into polypeptides having similar functional groups or protein domains. In some embodiments, the analytes include mRNAs that do not share identical or substantially similar sequences, which mRNAs are translated into proteins that do not share similar functional groups or protein domains. In some embodiments, the analytes include mRNAs that are translated into proteins that function in the same or similar biological pathways. In some embodiments, the biological pathways are associated with a pathologic disease. For example, the analytes can be derived from genes that are overexpressed or underexpressed in a particular tissue sample.

In some instances, the one or more overexpressed analytes are expressed in a cell from an organ selected from adrenal gland, bladder, bone, bone marrow, brain, bronchi of the lung, diaphragm, esophagus, eyes, fallopian tube, gallbladder, heart, hypothalamus, kidney, large intestine, liver, lungs, lymph node, mammary gland, nasal cavity, ovary, pancreas, penis, placenta, pituitary gland, prostate, skeletal muscle, skin, small intestine, spinal cord, spleen, stomach, testes, thymus gland, thyroid, trachea, tongue, urethra, uterus, vagina, or any combination thereof.

In some embodiments, the one or more analytes which might be targeted for removal or reduction during a spatial transcriptiomics assay are one or more housekeeping analytes (e.g., housekeeping genes). Non-limiting examples of housekeeping analytes that can be used in the methods described herein are as described in Eisenberg et al., Trends in Genetics, 29(10): 569-574 (2013) and Waxman et al., BMC Genomics, 8:243 (2007). In some embodiments, a housekeeping analyte can include, without limitations, glyceraldehyde-3-phosphate dehydrogenase (GAPDH), TATA-binding protein (TBP), and ribosomal proteins (RP) (e.g., 18S). In some embodiments, the method includes identifying the ratio of one or more analytes in the biological sample to a housekeeping analyte in the biological sample (e.g., in one or more cancerous regions). In some instances, the housekeeping analyte is selected from 18S ribosomal RNA, beta actin (actB), GAPDH, phosphoglycerate kinase 1 (PGK1), peptidylprolyl isomerase A (PPIA), ribosomal protein L13a (RPL13A), ribosomal protein, large, P0 (RPLP0), acidic ribosomal phosphoprotein PO (ARBP), beta-2-microglobulin (B2M), tyrosine 3-monooxygenase/tryptophan5-monooxygenase activation protein, zeta polypeptide (YWHAZ), succinate dehydrogenase complex, subunit A, flavoprotein (Fp) (SDHA), transferrin receptor (TFRC), glucuronidase, beta (GUSB), hydroxymethylbilane synthase (HMBS), hypoxanthine phosphoribosyltransferase 1 (HPRT1), TBP, or any combination thereof. In some instances, the one or more analytes are one or more non-native analytes not typically found in a cell or tissue. For example, green fluorescent protein or GFP is a fluorescent gene that is oftentimes used to track processes in a cell. GFP is introduced into a cell via a vector, wherein in within that cell of tissue that expression of GFP can be used as a marker for drug discovery, cellular pathways, etc. A skilled artisan will understand the myriad of such introduced marker genes used in the art to monitor cellular or tissue processes. The current methods for silencing or reducing the reads of the analytes are equally applicable to such marker analytes. Additionally, systems such as the Cre-Lox recombination systems for site-specific recombination (e.g., selected inversions, deletions, insertions, translocations of sequences) within cells or tissues may be in need of silencing, for example by targeting the Lox sequences for removal by using the methods described herein.

In some embodiments, the analytes targeted for reduction are a subset of RNA analytes from the entire transcriptome. In some embodiments, the analytes include an individual RNA. In some embodiments, the analytes include two or more RNAs. In some embodiments, the analytes include one or more mRNAs transcribed by one or more genes. In some embodiments, the analytes include one or more mRNA splice variants of one or more genes. In some embodiments, the analytes include non-polyadenylated RNAs in a biological sample. In some embodiments, the analytes include mRNAs having one or more single nucleotide polymorphisms (SNPs) in a biological sample.

In some embodiments, the analytes targeted for reduction in a spatial transcriptomics assay are a subset of all analytes that can be detected using a pool of templated ligation probes described herein.

In some embodiments, the analytes include 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, about 55, about 60, about 65, about 70, about 75, about 80, about 85, about 90, about 95, about 100, about 110, about 120, about 130, about 140, about 150, about 160, about 170, about 180, about 190, about 200, about 225, about 250, about 275, about 300, about 325, about 350, about 375, about 400, about 425, about 450, about 475, about 500, about 600, about 700, about 800, about 900, or about 1000 analytes.

In some embodiments, the analytes account for at least 0.1%, at least 0.2%, at least 0.3%, at least 0.4%, at least 0.5%, at least 0.6%, at least 0.7%, at least 0.8%, at least 0.9%, at least 1%, at least 2%, at least 3%, at least 4%, at least 5%, at least 6%, at least 7%, at least 8%, at least 9%, at least 10%, at least 15%, or at least 20% at least 15%, or at least 20% of the total analyte pool in a biological sample.

In some embodiments, examples of the RNA that may be targeted for reduction or exclusion from a spatial transcriptomics assay include, but are not limited to, messenger RNA (mRNA), ribosomal RNA (rRNA), mitochondrial RNA (mtRNA), transfer RNA (tRNA), microRNA (miRNA), and viral RNA. In some embodiments, the RNA molecule includes 5.8S ribosomal RNA (rRNA), 5S rRNA, transfer RNA (tRNA), microRNA (miRNA), a small nucleolar RNA (snoRNAs), Piwi-interacting RNA (piRNA), tRNA-derived small RNA (tsRNA), and small rDNA-derived RNA (srRNA), or mitochondrial RNA (mtRNA). In some embodiments, examples of the RNA include, but are not limited to, mRNA. In some embodiments, the RNA molecule includes an RNA molecule that is added (e.g., transfected) into a sample (e.g., a small interfering RNA (siRNA)). The RNA can be double-stranded RNA or single-stranded RNA. In embodiments where the RNA is double-stranded it is processed as a single-stranded RNA prior to hybridization by templated ligation probes. In some embodiments, the RNA can be circular RNA. In some embodiments, the RNA can be a bacterial rRNA (e.g., 16s rRNA or 23s rRNA). In some embodiments, the RNA that is targeted for reduction or exclusion from a spatial transcriptomic assay is from *E. coli.*

In some embodiments, the RNA molecule is rRNA. In some embodiments, the rRNA is eukaryotic rRNA. In some embodiments, the rRNA is cytoplasmic rRNA. In some embodiments, the rRNA is mitochondrial rRNA. Cytoplasmic rRNAs include, for example, 28S, 5.8S, 5S and 18S rRNAs. Mitochondrial rRNAs include, for example, 12S and 16S rRNAs. The rRNA may also be prokaryotic rRNA, which includes, for example, 5S, 16S, and 23S rRNA. The sequences for rRNAs are well known to those skilled in the art and can be readily found in sequence databases such as GenBank or may be found in the literature. For example, the sequence for the human 18S rRNA can be found in GenBank as Accession No. M10098 and the human 28S rRNA as Accession No. M11167.

In some embodiments, the RNA molecule is mitochondrial RNA. Mitochondrial RNAs include, for example, 12S rRNA (encoded by MT-RNR1), and 16S rRNA (encoded by MT-RNR2), RNAs encoding electron transport chain proteins (e.g., NADH dehydrogenase, coenzyme Q- cytochrome c reductase / cytochrome b, cytochrome c oxidase, ATP synthase, or humanin), and tRNAs (encoded by MT-TA, MT-TR, MT-TN, MT-TD, MT-TC, MT-TE, MT-TQ, MT-TG, MT-TH, MT-TI, MT-TL1, MT-TL2, MT-TK, MT-TM, MT-TF, MT-TP, MT- TS1, MT-TS2, MT-TT, MT-TW, MT-TY, or MT-TV).

In some embodiments, the RNA targeted for reduction or exclusion in spatial transcriptomics assays is transfer RNA (tRNA). In some embodiments, the RNA may be a particular mRNA. For example, it may be desirable to remove cellular transcripts that are usually present in abundance. Thus, the mRNA for reduction or exclusion from a sptail transcriptomics assay may include, but is not limited to, ACTB, GAPDH, and TUBB. Other sequences for tRNA and specific mRNA are well known to those skilled in the art and can be readily found in sequence databases such as GenBank or may be found in the literature.

In some embodiments, mRNA is not targeted for hybridization by templated ligation probes. In some embodiments, one or more templated ligation probes do not have a poly-dT that will hybridize to the poly-A tail of the ligated product described herein. In yet another particular embodiment, the templated ligation probe targets and specifically hybridizes to human 18S or human 28S rRNA.

In some embodiments, one or more RNA molecules targeted for reduction or exclusion from a spatial transcriptomics assay belong to a single species of RNA. For example, in some embodiments, the one or more RNA molecules hybridize with only ribosomal RNA molecules. In some embodiments, the one or more RNA molecules hybridize with only mitochondrial RNA molecules. In some embodiments, the RNA molecules can be a combination of two or more species of RNA. In some embodiments, an RNA molecule is an RNA fragment of one of the RNA molecules targeted for reduction or exclusion from a spatial transcriptomics assay as described herein. In some embodiments, an RNA molecule is a full-length RNA molecule of one of the RNA molecules targeted for reduction or exclusion from a spatial transcriptomics assay as described herein.

### (c) Probe(s) Targeted for Reduction or Exclusion

In some embodiments, the methods described herein include one or more probes (e.g., a first probe and a second probethat a user wishes to remove from the templated ligation process, such that the one or more probes is blocked or silenced using the methods described herein. As used herein, the term "silence" refers to a process that can block a templated ligation probe from hybridizing to its target analyte, thereby reducing or eliminating the amount of ligation product generated from the templated ligation probe and hence the target analyte detected. As a result, spatial analysis reads corresponding to the target analyte in a sample decreases relative to a reference sample to which the templated ligation probe is not silenced.

In some embodiments, blocker oligonucleotides are designed to silence one or more templated ligation probes in a pool of templated ligation probes (e.g., all templated ligation probes used for templated ligation detection). For example, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or more of all templated ligation probes in the pool can be silenced. In some instances, the pool is used in a single templated ligation detection.

In some embodiments, the templated ligation probe silencing is targeted templated ligation probe silencing. Targeted templated ligation probe silencing using the methods disclosed herein allows for silencing of a subset of templated ligation probes from the entire pool of templated ligation probes. In some embodiments, the probes for silencing target an individual analyte. In some embodiments, the probes for silencing target two or more analytes. In some embodiments, the probes target one or more mRNAs transcribed by one or more genes. In some embodiments, the probes target one or more mRNA splice variants of one or more genes. In some embodiments, the probes for silencing target non-polyadenylated RNAs in a biological sample.

In some embodiments, the methods described herein include an spanning probe for silencing. In some embodiments, the spanning probe is any one of the spanning probes described herein, where the target analyte is a target for reduction or exclusion in a spatial transcriptomics assay.

In some embodiments, the one or more probes for silencing described herein hybridize to a target analyte with an affinity that is less than 90%, less than 80%, less than 70%, less than 60%, less than 50%, less than 40%, less than 30%, less than 20%, or less than 10% as compared to that of one or more reference templated ligation probes not targeted for silencing or hybridizing to their target analyte.

In some embodiments, one or more probes could hybridize to non-target analytes. For example, the one or more probes can hybridize to analytes other than those for which the one or more probes are designed to hybridize (i.e., in an off-target manner). In some embodiments, the one or more probes hybridize to two or more analytes. In such cases, the one or more probes cannot be selectively removed from a pool of template ligation probes, e.g., by selective hybridization using bait oligonucleotides, from the pool of templated ligation probes that contains the one or more probes.

### (d) Blocker or silencing oligonucleotides

In some embodiments, the methods described herein include one or more blocker oligonucleotides (e.g., a first blocker oligonucleotide and a second blocker oligonucleotide). As used herein, the term "blocker oligonucleotide" refers to an oligonucleotide that can block one or more templated ligation probes from hybridizing to their target analyte(s). In some embodiments, a blocker oligonucleotide is an antisense oligonucleotide, which includes a sequence that is substantially complementary to a target specific sequence of one or more templated ligation probes.

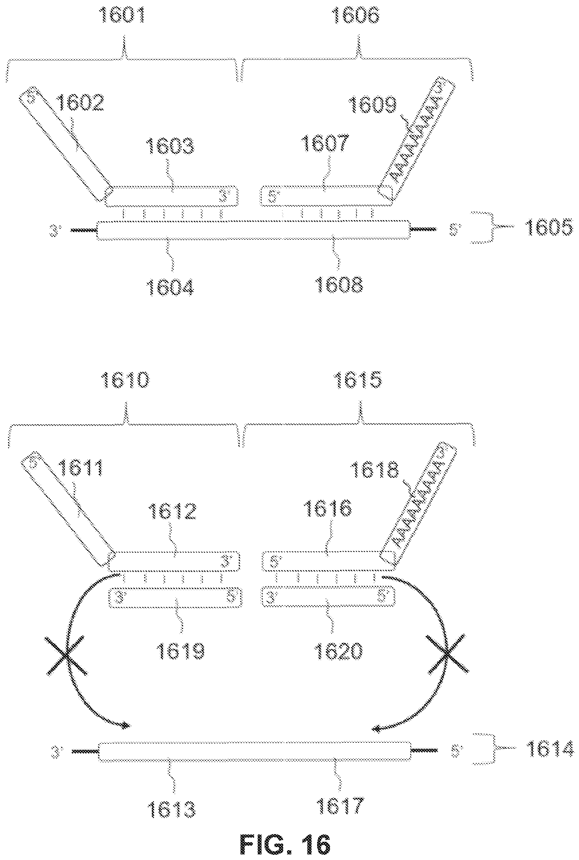

A non-limiting example of a method for identifying a location of an analyte in a biological sample where the method includes a plurality of probes (e.g., a first probe, a second probe, a first uprobe to be silenced, a second probe to be silenced, a first blocker oligonucleotide, and a second blocker oligonucleotide) can include the components as shown **FIG. 16****.** A first probe **1601** includes a functional sequence **1602** and a sequence **1603** that is substantially complementary to a first target sequence **1604** of the analyte **1605.** A second probe **1606** includes a sequence **1607** that is substantially complementary to a second target sequence **1608** of the analyte **1605** and a capture probe capture domain **1609** that is capable of hybridizing to a capture domain of a capture probe. The first probe **1601** can hybridize via its first sequence **1603** to the first target sequence **1604** of the analyte **1605,** and the second probe **1606** can hybridize via its second sequence **1607** to the second target sequence **1608** of an analyte **1605.** The pool of probes including the first probe **1601** and the second probe **1606** also includes a first probe to be silenced **1610,** a second probe to be silenced **1615,** a first blocker oligonucleotide **1619,** and a second blocker oligonucleotide **1620.** Probe **1610** includes a functional sequence **1611** and a sequence **1612** that is substantially complementary to a first target sequence **1613** of an analyte for reduction or exclusion **1614,** for example a highly expressed mRNA where blocking the capture of the highly expressed mRNA would benefit the spatial assay. The probe **1615** includes a sequence **1616** that is substantially complementary to a second target sequence **1617** of the analyte **1614** and a capture probe capture domain **1618** that is capable of binding to a capture domain of a capture probe. The first blocker oligonucleotide **1619** includes a sequence that is substantially complementary to the sequence **1612** of the first probe **1610,** as such the first blocker oligonucleotide **1619** can hybridize to the sequence **1612** and block hybridization between the sequence **1612** and the first target sequence **1613** of the analyte **1614.** Similarly, because the second blocker oligonucleotide **1620** includes a sequence that is substantially complementary to the sequence **1616** of the second probe **1615,** the second blocker oligonucleotide **1620** can hybridize to the sequence **1616** and block hybridization between the sequence **1616** and the second target sequence **1617** of the analyte **1614.**

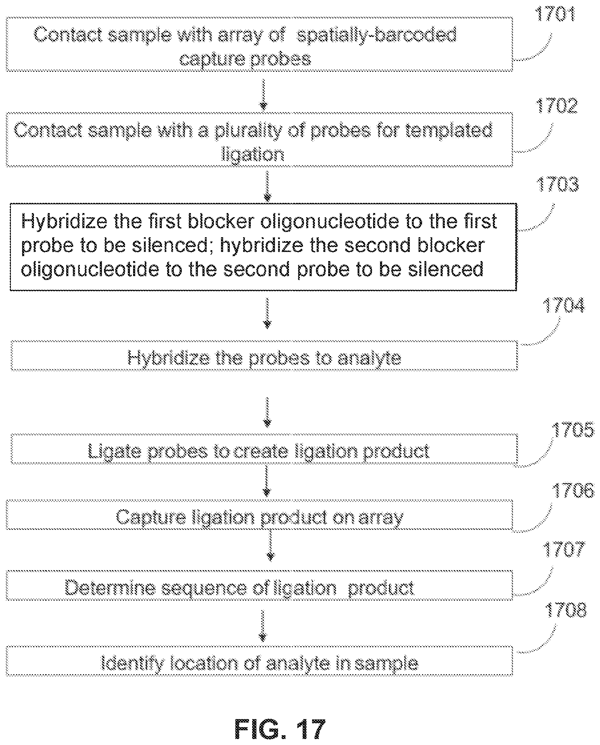

Referring to **FIG. 17****,** provided are exemplary methods for identifying a location of an analyte in a biological sample. In some embodiments, the biological samples includes a plurality of analytes including the analytes of interest and one or more analytes not of interest. In some instances, the methods include **1701** contacting a biological sample with an array of spatially-barcoded capture probes. In some instances, the array is on a substrate and the array includes a plurality of capture probes, wherein a capture probe of the plurality includes: (i) a spatial barcode and (ii) a capture domain. After placing the biological sample on the array, the biological sample **1702** is contacted with a plurality of probes including (i) a first probe and a second probe, wherein the first probe and the second probe each includes one or more sequences that are substantially complementary to sequences of the analyte of interest, and wherein the second probe includes a capture probe capture domain; (ii) a first probe to be silenced and a second probe to be silenced, wherein the first and the second probes to be silenced each includes one or more sequences that are substantially complementary to sequences of the one or more analytes not of interest; and (iii) a first blocker oligonucleotide and a second blocker oligonucleotide, wherein the first blocker oligonucleotide is substantially complementary to the first probe to be silenced, and the second blocker oligonucleotide is substantially complementary to the second probe to be silenced. The first blocker oligonucleotide and the second blocker oligonucleotide 1703 hybridize to the first and the second probes to be silenced, respectively, to block the first and second probes to be silenced from hybridizing to the one or more analytes not of interest. Then, the first probe and the second probe hybridize to complementary sequences in the analyte of interest **1704.** In some instances, steps **1702, 1703** and **1704** can happen concurrently (i.e., templated ligation probes and blocker oligonucleotides are added at the same time to the biological sample). Here, the RNA probes **1702,** the probes to be silenced **1703** and the blocking oligonucleotides **1703** are added together to the sample, such that the blocking oligonucleotides hybridize to their target probes (the first and second probes to be silenced) and the first and second probes hybridize to their analytes (the analytes of interest) **1704.** Alternatively, in some instances, step **1703** can occur prior to **1702** at which point both the unblocked and pre-blocked probes are contacted with the sample simultaneously. For example, step **1703** can occur in a tube or other milieu where the blocking oligonucleotides are added to a pool of first and second probes (e.g., a pool of probes targeting both analytes of interest and analytes not of interest) prior to contacting the sample with the pool, or a plurality of, probes **1702.** A ligation product comprising the first probe and the second probe **1705** is generated, and the ligation product is released from the analyte. The liberated ligation product can **1706** hybridize to the capture domain of a capture probe on the array. After capture, (i) all or a part of the sequence of the ligation product specifically hybridized to the capture domain, or a complement thereof, and (ii) the spatial barcode, or a complement thereof **1707** can be determined, and the spatial location of the analyte is determined by correlating the sequence of (i) and (ii) to the sample thereby identifying the location of the analyte in the biological sample **1708.**

In some embodiments, a blocker oligonucleotide includes a sequence that is about 10 nucleotides to about 100 nucleotides (e.g., a sequence of about 10 nucleotides to about 90 nucleotides, about 10 nucleotides to about 80 nucleotides, about 10 nucleotides to about 70 nucleotides, about 10 nucleotides to about 60 nucleotides, about 10 nucleotides to about 50 nucleotides, about 10 nucleotides to about 40 nucleotides, about 10 nucleotides to about 30 nucleotides, about 10 nucleotides to about 20 nucleotides, about 20 nucleotides to about 100 nucleotides, about 20 nucleotides to about 90 nucleotides, about 20 nucleotides to about 80 nucleotides, about 20 nucleotides to about 70 nucleotides, about 20 nucleotides to about 60 nucleotides, about 20 nucleotides to about 50 nucleotides, about 20 nucleotides to about 40 nucleotides, about 20 nucleotides to about 30 nucleotides, about 30 nucleotides to about 100 nucleotides, about 30 nucleotides to about 90 nucleotides, about 30 nucleotides to about 80 nucleotides, about 30 nucleotides to about 70 nucleotides, about 30 nucleotides to about 60 nucleotides, about 30 nucleotides to about 50 nucleotides, about 30 nucleotides to about 40 nucleotides, about 40 nucleotides to about 100 nucleotides, about 40 nucleotides to about 90 nucleotides, about 40 nucleotides to about 80 nucleotides, about 40 nucleotides to about 70 nucleotides, about 40 nucleotides to about 60 nucleotides, about 40 nucleotides to about 50 nucleotides, about 50 nucleotides to about 100 nucleotides, about 50 nucleotides to about 90 nucleotides, about 50 nucleotides to about 80 nucleotides, about 50 nucleotides to about 70 nucleotides, about 50 nucleotides to about 60 nucleotides, about 60 nucleotides to about 100 nucleotides, about 60 nucleotides to about 90 nucleotides, about 60 nucleotides to about 80 nucleotides, about 60 nucleotides to about 70 nucleotides, about 70 nucleotides to about 100 nucleotides, about 70 nucleotides to about 90 nucleotides, about 70 nucleotides to about 80 nucleotides, about 80 nucleotides to about 100 nucleotides, about 80 nucleotides to about 90 nucleotides, or about 90 nucleotides to about 100 nucleotides).

In some embodiments where a templated ligation probe includes a linker sequence described herein, a blocker oligonucleotide used for silencing the templated ligation probe includes a sequence that is about 10 nucleotides to about 300 nucleotides (e.g., a sequence of about 10 nucleotides to about 300 nucleotides, about 10 nucleotides to about 250 nucleotides, about 10 nucleotides to about 200 nucleotides, about 10 nucleotides to about 150 nucleotides, about 10 nucleotides to about 100 nucleotides, about 10 nucleotides to about 50 nucleotides, about 50 nucleotides to about 300 nucleotides, about 50 nucleotides to about 250 nucleotides, about 50 nucleotides to about 200 nucleotides, about 50 nucleotides to about 150 nucleotides, about 50 nucleotides to about 100 nucleotides, about 100 nucleotides to about 300 nucleotides, about 100 nucleotides to about 250 nucleotides, about 100 nucleotides to about 200 nucleotides, about 100 nucleotides to about 150 nucleotides, about 150 nucleotides to about 300 nucleotides, about 150 nucleotides to about 250 nucleotides, about 150 nucleotides to about 200 nucleotides, about 200 nucleotides to about 300 nucleotides, about 200 nucleotides to about 250 nucleotides, or about 250 nucleotides to about 300 nucleotides).

In some embodiments, the sequence of a blocker oligonucleotide that is substantially complementary to a sequence in the templated ligation probe includes a sequence that is about 5 nucleotides to about 50 nucleotides (e.g., about 5 nucleotides to about 45 nucleotides, about 5 nucleotides to about 40 nucleotides, about 5 nucleotides to about 35 nucleotides, about 5 nucleotides to about 30 nucleotides, about 5 nucleotides to about 25 nucleotides, about 5 nucleotides to about 20 nucleotides, about 5 nucleotides to about 15 nucleotides, about 5 nucleotides to about 10 nucleotides, about 10 nucleotides to about 50 nucleotides, about 10 nucleotides to about 45 nucleotides, about 10 nucleotides to about 40 nucleotides, about 10 nucleotides to about 35 nucleotides, about 10 nucleotides to about 30 nucleotides, about 10 nucleotides to about 25 nucleotides, about 10 nucleotides to about 20 nucleotides, about 10 nucleotides to about 15 nucleotides, about 15 nucleotides to about 50 nucleotides, about 15 nucleotides to about 45 nucleotides, about 15 nucleotides to about 40 nucleotides, about 15 nucleotides to about 35 nucleotides, about 15 nucleotides to about 30 nucleotides, about 15 nucleotides to about 25 nucleotides, about 15 nucleotides to about 20 nucleotides, about 20 nucleotides to about 50 nucleotides, about 20 nucleotides to about 45 nucleotides, about 20 nucleotides to about 40 nucleotides, about 20 nucleotides to about 35 nucleotides, about 20 nucleotides to about 30 nucleotides, about 20 nucleotides to about 25 nucleotides, about 25 nucleotides to about 50 nucleotides, about 25 nucleotides to about 45 nucleotides, about 25 nucleotides to about 40 nucleotides, about 25 nucleotides to about 35 nucleotides, about 25 nucleotides to about 30 nucleotides, about 30 nucleotides to about 50 nucleotides, about 30 nucleotides to about 45 nucleotides, about 30 nucleotides to about 40 nucleotides, about 30 nucleotides to about 35 nucleotides, about 35 nucleotides to about 50 nucleotides, about 35 nucleotides to about 45 nucleotides, about 35 nucleotides to about 40 nucleotides, about 40 nucleotides to about 50 nucleotides, about 40 nucleotides to about 45 nucleotides, or about 45 nucleotides to about 50 nucleotides).

In some embodiments, the blocker oligonucleotide is about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more nucleotides in length.

In some embodiments, the methods described herein comprise a plurality of blocker oligonucleotides, and the plurality of blocker nucleotides comprise at least 1, at least 2, at least 3, at least 4, at least 5, at least 6, at least 7, at least 8, at least 9, at least 10, about 15, about 20, about 25, about 30, about 35, about 40, about 45, about 50, about 75, about 100, or more blocker oligonucleotides. In some embodiments, the blocker oligonucleotides have the same sequence. In some embodiments, the blocker oligonucleotides have different sequences. In some embodiments, the plurality of blocker oligonucleotides described herein can target at least 1, at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, or more templated ligation probes.

In some embodiments, a blocker oligonucleotide does not include a phosphorylated nucleotide at the 5' end. In some embodiments, a blocker oligonucleotide (e.g., a blocker oligonucleotide targeting the LHS probe) does not include one or more (e.g., 1, 2, 3, 4, 5, 6, 7, 8, 9, or 10) ribonucleic acid bases at the 3' end.

In some instances, a blocker oligonucleotide is a DNA molecule. In some embodiments, the DNA molecule is a single-stranded DNA oligonucleotide having a sequence partially or completely complementary to a templated ligation probe and specifically hybridizes to the templated ligation probe. In some embodiments, the blocker oligonucleotide described herein includes ribonucleotides, deoxyribonucleotides, and/or synthetic nucleotides that are capable of participating in Watson-Crick type or analogous base pair interactions. In some embodiments, a blocker oligonucleotide includes a sequence that is at least 70%, at least 75%, at least 80%, at least 85%, at least 90%, at least 91%, at least 92%, at least 93%, at least 94%, at least 95%, at least 96%, at least 97%, at least 98%, at least 99%, or 100% (i.e., completely) complementary to a sequence (e.g., a sequence where the template ligation probe hybridizes to an analyte targeted for reduction or exclusion from a spatial transcriptomics assay) in the template ligation probe.

A blocker oligonucleotide can be produced by techniques known in the art. For example, in some embodiments, a blocker oligonucleotide is produced by chemical synthesis, by *in vitro* expression from recombinant nucleic acid molecules, or by *in vivo* expression from recombinant nucleic acid molecules. An RNA depletion probe may also be produced by amplification of the RNA targeted for reduction or exclusion from a spatial transcriptomics assay, e.g., by RT-PCR, asymmetric PCR, or rolling circle amplification.

In some embodiments, the methods disclosed herein include multiple blocker oligonucleotides. In some embodiments, the blocker oligonucleotides include sequences that are complementary or substantially complementary to one or more templated ligation probes. Methods provided herein may be applied to a single templated ligation probe or a plurality of templated ligation probes.

In some embodiments, a single blocker oligonucleotide hybridizes to the entire length of a templated ligation probe. In some embodiments, a blocker oligonucleotide has regions that are not complementary to a templated ligation probe, so long as such sequences do not substantially affect specific hybridization of the blocker oligonucleotide to the templated ligation probe. In some embodiments, the blocker oligonucleotides are not contiguous, such that while they may collectively hybridize across a length of the templated ligation probe, there may exist gaps between the individual blocker oligonucleotides. For example, in some embodiments, the blocker oligonucleotides that target a templated ligation probe are spaced at least one, at least two, at least 5, at least 10, at least 20, at least 30, at least 50, at least 100, at least 200, at least 300, at least 400, at least 500, at least 600, at least 700, at least 800, at least 900, or at least 1000 nucleotides apart along the length of the templated ligation probe. As such, there may be a plurality of blocker oligonucleotides that will hybridize adjacent to, or non-contiguous to, each other along the length, or partially along the length, of the templated ligation probe.

In some embodiments, a blocker oligonucleotide is associated with (e.g., conjugated to) a detectable label, an optical label, and or a label as described herein. In some instances, the detectable label is a radioisotope, a fluorescent or chemiluminescent moiety, with an enzyme or ligand, which can be used for detection or confirmation that the blocker oligonucleotide has hybridized to the target sequence. The detectable label can be directly detectable by itself (e.g., radioisotope labels or fluorescent labels) or, in the case of an enzymatic label, can be indirectly detectable, e.g., by catalyzing chemical alterations of a chemical substrate compound or composition, which chemical substrate compound or composition is directly detectable. The detectable label can be qualitatively detected (e.g., optically or spectrally), or it can be quantified using methods known in the art and/or disclosed herein. In some instances, a blocker oligonucleotide is not associated with, or conjugated to, a detectable label, an optical label, and/or a label as described herein.

In some embodiments, the methods provided herein include a pool of two or more blocker oligonucleotides. In some embodiments, the pool of blocker oligonucleotides include about 100 nM, about 200 nM, about 300 nM, about 400 nM, about 500 nM, about 600 nM, about 700 nM, about 800 nM, about 900 nM, or about 1000 nM of each blocker oligonucleotide. In some embodiments, the pool of blocker oligonucleotides include about 1 µM, about 2 µM, about 3 µM, about 4 µM, about 5 µM, about 6 µM, about 7 µM, about 8 µM, about 9 µM, or about 10 µM, or more, of each blocker oligonucleotide. In some embodiments, the concentration of a blocker oligonucleotide in a pool of blocker oligonucleotides depends on the relative level of the templated ligation probes. For example, ribosomal transcripts 18S and 28S can be highly abundant in a tissue sample. In this case, templated ligation probes targeting 18S and/or 28S can be present in the pool of templated ligation probes at a higher concentration than other templated ligation probes present in the pool. As such, a blocker oligonucleotide used for silencing the 18S and/or 28S-targeting templated ligation probes can have a higher concentration than a blocker oligonucleotide used for silencing other templated ligation probes.

### (i) Modifications to blocker oligonucleotides

In some embodiments, a blocker oligonucleotide includes one or more modifications. In some embodiments, the one or more modifications include biotin, amino-modifiers, azide, cholesteryl, alkynes, and/or thiol modifiers. Details of possible oligonucleotide modifications can be found, e.g., in Ochoa, et al. Molecules 25.20 (2020): 4659; and Verma, et al. Annual Review of Biochemistry 67.1 (1998): 99-134.

In some embodiments, the one or more modifications include a detectable label, e.g., an optical label. In some instances, the optical label is a fluorophore or a fluorescent moiety. For example, the fluorophore or the fluorescent moiety can be from a group that includes: 7-AAD (7-Aminoactinomycin D), Acridine Orange (+DNA), Acridine Orange (+RNA), Alexa Fluor^{®} 350, Alexa Fluor^{®} 430, Alexa Fluor^{®} 488, Alexa Fluor^{®} 532, Alexa Fluor^{®} 546, Alexa Fluor^{®} 555, Alexa Fluor^{®} 568, Alexa Fluor^{®} 594, Alexa Fluor^{®} 633, Alexa Fluor^{®} 647, Alexa Fluor^{®} 660, Alexa Fluor^{®} 680, Alexa Fluor^{®} 700, Alexa Fluor^{®} 750, Allophycocyanin (APC), AMCA / AMCA-X, 7-Aminoactinomycin D (7-AAD), 7- Amino-4-methylcoumarin, 6-Aminoquinoline, Aniline Blue, ANS, APC-Cy7, ATTO-TAG^{™} CBQCA, ATTO-TAG^{™} FQ, Auramine O-Feulgen, BCECF (high pH), BFP (Blue Fluorescent Protein), BFP / GFP FRET, BOBO^{™}-1 / BO-PRO^{™}-1, BOBO^{™}-3 / BO-PRO^{™}-3, BODIPY^{®} FL, BODIPY^{®} TMR, BODIPY^{®} TR-X, BODIPY^{®} 530/550, BODIPY^{®} 558/568, BODIPY^{®} 564/570, BODIPY^{®} 581/591, BODIPY^{®} 630/650-X, BODIPY^{®} 650-665-X, BTC, Calcein, Calcein Blue, Calcium Crimson^{™}, Calcium Green-1^{™}, Calcium Orange^{™}, Calcofluor^{®} White, 5-Carboxyfluoroscein (5-FAM), 5-Carboxynaphthofluoroscein, 6-Carboxyrhodamine 6G, 5-Carboxytetramethylrhodamine (5-TAMRA), Carboxy-X-rhodamine (5-ROX), Cascade Blue^{®}, Cascade Yellow^{™}, CCF2 (GeneBLAzer^{™}), CFP (Cyan Fluorescent Protein), CFP / YFP FRET, Chromomycin A3, Cl-NERF (low pH), CPM, 6-CR 6G, CTC Formazan, Cy2^{®}, Cy3^{®}, Cy3.5^{®}, Cy5^{®}, Cy5.5^{®}, Cy7^{®}, Cychrome (PE-Cy5), Dansylamine, Dansyl cadaverine, Dansylchloride, DAPI, Dapoxyl, DCFH, DHR, DiA (4-Di-16-ASP), DiD (DilC18(5)), DIDS, Dil (DilC18(3)), DiO (DiOC18(3)), DiR (DilC18(7)), Di-4 ANEPPS, Di-8 ANEPPS, DM-NERF (4.5-6.5 pH), DsRed (Red Fluorescent Protein), EBFP, ECFP, EGFP, ELF^{®} -97 alcohol, Eosin, Erythrosin, Ethidium bromide, Ethidium homodimer-1 (EthD-1), Europium (III) Chloride, 5-FAM (5-Carboxyfluorescein), Fast Blue, Fluorescein-dT phosphoramidite, FITC, Fluo-3, Fluo-4, FluorX^{®}, Fluoro-Gold^{™} (high pH), Fluoro-Gold^{™} (low pH), Fluoro-Jade, FM^{®} 1-43, Fura-2 (high calcium), Fura-2 / BCECF, Fura Red^{™} (high calcium), Fura Red^{™} / Fluo-3, GeneBLAzer^{™} (CCF2), GFP Red Shifted (rsGFP), GFP Wild Type, GFP / BFP FRET, GFP / DsRed FRET, Hoechst 33342 & 33258, 7-Hydroxy-4-methylcoumarin (pH 9), 1,5 IAEDANS, Indo-1 (high calcium), Indo-1 (low calcium), Indodicarbocyanine, Indotricarbocyanine, JC-1, 6-JOE, JOJO^{™}-1 / JO-PRO^{™}-1, LDS 751 (+DNA), LDS 751 (+RNA), LOLO^{™}-1 / LO-PRO^{™}-1, Lucifer Yellow, LysoSensor^{™} Blue (pH 5), LysoSensor^{™} Green (pH 5), LysoSensor^{™} Yellow/Blue (pH 4.2), LysoTracker^{®} Green, LysoTracker^{®} Red, LysoTracker^{®} Yellow, Mag-Fura-2, Mag-Indo-1, Magnesium Green^{™}, Marina Blue^{®}, 4-Methylumbelliferone, Mithramycin, MitoTracker^{®} Green, MitoTracker^{®} Orange, MitoTracker^{®} Red, NBD (amine), Nile Red, Oregon Green^{®} 488, Oregon Green^{®} 500, Oregon Green^{®} 514, Pacific Blue, PBF1, PE (R-phycoerythrin), PE-Cy5, PE-Cy7, PE-Texas Red, PerCP (Peridinin chlorphyll protein), PerCP-Cy5.5 (TruRed), PharRed (APC-Cy7), C-phycocyanin, R-phycocyanin, R-phycoerythrin (PE), PI (Propidium Iodide), PKH26, PKH67, POPO^{™}-1 / PO-PRO^{™}-1, POPO^{™}-3 / PO-PRO^{™}-3, Propidium Iodide (PI), PyMPO, Pyrene, Pyronin Y, Quantam Red (PE-Cy5), Quinacrine Mustard, R670 (PE-Cy5), Red 613 (PE-Texas Red) , Red Fluorescent Protein (DsRed), Resorufin, RH 414, Rhod-2, Rhodamine B, Rhodamine Green^{™}, Rhodamine Red^{™}, Rhodamine Phalloidin, Rhodamine 110, Rhodamine 123, 5-ROX (carboxy-X-rhodamine), S65A, S65C, S65L, S65T, SBFI, SITS, SNAFL^{®}-1 (high pH), SNAFL^{®}-2, SNARF^{®}-1 (high pH), SNARF^{®}-1 (low pH), Sodium Green^{™}, SpectrumAqua^{®}, SpectrumGreen^{®} #1, SpectrumGreen^{®} #2, SpectrumOrange^{®}, SpectrumRed^{®}, SYTO^{®} 11, SYTO^{®} 13, SYTO^{®} 17, SYTO^{®} 45, SYTOX^{®} Blue, SYTOX^{®} Green, SYTOX^{®} Orange, 5-TAMRA (5-Carboxytetramethylrhodamine), Tetramethylrhodamine (TRITC), Texas Red^{®} / Texas Red^{®}-X, Texas Red^{®}-X (NHS Ester), Thiadicarbocyanine, Thiazole Orange, TOTO^{®}-1 / TO-PRO^{®}-1, TOTO^{®}-3 / TO-PRO^{®}-3, TO-PRO^{®}-5, Tri-color (PE-Cy5), TRITC (Tetramethylrhodamine), TruRed (PerCP-Cy5.5), WW 781, X-Rhodamine (XRITC) , Y66F, Y66H, Y66W, YFP (Yellow Fluorescent Protein), YOYO^{®}-1 / YO-PRO^{®}-1, YOYO^{®}-3 / YO-PRO^{®}-3, 6-FAM (Fluorescein), 6-FAM (NHS Ester), 6-FAM (Azide), HEX, TAMRA (NHS Ester), Yakima Yellow, MAX, TET, TEX615, ATTO 488, ATTO 532, ATTO 550, ATTO 565, ATTO Rho101, ATTO 590, ATTO 633, ATTO 647N, TYE 563, TYE 665, TYE 705, 5' IRDye^{®} 700, 5' IRDye^{®} 800, 5' IRDye^{®} 800CW (NHS Ester), WellRED D4 Dye, WellRED D3 Dye, WellRED D2 Dye, Lightcycler^{®} 640 (NHS Ester), and/or Dy 750 (NHS Ester).

In some embodiments, a blocker oligonucleotide includes one or more, e.g., at least 1, 2, 3, 4, 5, 6, 7, 8, 9, 10, 11, 12, 13, 14, 15, 16, 17, 18, 19, 20, or more, non-natural nucleic acids. In some embodiments, a blocker oligonucleotide includes at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90%, or 100% non-natural nucleic acids. In some embodiments, a blocker oligonucleotide has a melting temperature (Tm) of about 55°C, about 56°C, about 57°C, about 58°C, about 59°C, about 60°C, about 61°C, about 62°C, about 63°C, about 64°C, about 65°C, about 65°C, about 66°C, about 67°C, about 68°C, about 69°C, about 70°C, or higher.

In some embodiments, the non-natural nucleic acid comprises one or more phosphate analogs, for example, phosphodiester analogs (e.g., phosphorothioate, boranophosphate, or phosphonate). In some embodiments, the non-natural nucleic acid comprises one or more aptamers, e.g., substituents at C-2' (e.g., F, NH2, O-methyl) or modifications to the 5-position of uracil. In some embodiments, the non-natural nucleic acid comprises one or more nucleobase analogs, e.g., 2-thioT, ψT, yT, xA, yC, xC, or xG. In some embodiments, the non-natural nucleic acid comprises substitutions of a furanose ring, e.g., threose nucleic acid (TNA), locked nucleic acid (LNA), or hexitol nucleic acid (HNA). In some embodiments, the non-natural nucleic acid comprises acyclic analogues, e.g., flexible nucleic acid (FNA), glycerol nucleic acid (GNA), or peptide nucleic acid (PNA). Detailed descriptions can be found, e.g., in Appella et al., Current Opinion in Chemical Biology, 13.5-6 (2009): 687-696.

In some embodiments, the blocker oligonucleotide described herein comprises one or more non-natural nucleic acids. In some embodiments, the non-natural nucleic acid is a locked nucleic acid (LNA). Locked nucleic acids are a type of nucleic acid analog that contains a 2'-O, 4'-C methylene bridge, which increases the affinity for complementary RNA or DNA. In some instances, compared to naturally-occurring oligonucleotides, LNAs provide enhanced stability, increased melting temperature, and binding affinity. This bridge-locked in the 3'-endo conformation restricts the flexibility of the ribofuranose ring and locks the structure into a rigid bicyclic formation. LNAs are used to increase the sensitivity and specificity of molecular biology tools such as DNA microarrays and LNA-based oligonucleotides are being developed as antisense therapies. LNA can be incorporated into the blocker oligonucleotides by standard phophoramidite chemistry.

In some embodiments, the LNA is a 2'-O,4'-C-methylene-α-1-ribofuranose (α-L-LNA) or a 2*'*-O,4*'*-C-methylene-β-d-ribofuranose (β-D-LNA). In some embodiments, the blocker oligonucleotide includes an all-LNA, a LNA mixmer (any combination of LNA and DNA residues), a LNA gapmer (with a central DNA moiety flanked by LNA-modified 5'- or 3'-end), an LNA-modified LNAzyme, or any combinations thereof. Detailed descriptions of LNA can be founds, e.g., in Grinweiler and Roland, BioDrugs 21.4 (2007): 235-243.

In some embodiments, a blocker oligonucleotide includes one or more (e.g., 1, 2, 3, 4, 5, 6, 7, 8, 9, 10, or more) modifications to its structure. For example, the modifications include one or more carbon moieties attached to the LNA. In some embodiments, the modifications include modified bases, e.g., 2'-O-methoxy-ethyl Bases (2'-MOE) (e.g., 2-MethoxyEthoxy A, 2-MethoxyEthoxy MeC, 2-MethoxyEthoxy G, and/or 2-MethoxyEthoxy T); 2'-O-Methyl RNA Bases (e.g., 2'-O-Methyl RNA Bases); Fluoro Bases (e.g., Fluoro C, Fluoro U, Fluoro A, and/or Fluoro G); 2-Aminopurine, 5-Bromo dU, deoxyUridine, 2,6-Diaminopurine (2-Amino-dA), Dideoxy-C, deoxyInosine, Hydroxymethyl dC, inverted dT, Iso-dG, Iso-dC, inverted Dideoxy-T, 3'-3'-inverted thymine, 5-Methyl dC, 5-Nitroindole, Super T (5-hydroxybutynl-2'-deoxyuridine), Super G (8-aza-7-deazaguanosine) and/or combinations thereof.

In some embodiments, a blocker oligonucleotide includes a mixture of DNA, RNA and/or LNA bases. In some embodiments, the LNA is about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides in length. In some instances, the LNA is about 5 to 20, about 10 to 20, or about 15 to 20 nucleotides in length. It is appreciated that the length of an LNA can be adjusted to modulate (e.g., increase) the melting temperature (Tm) so that it is not displaced during other methods disclosed herein (e.g., reverse transcription of the probe and/or analyte).

In some instances, the LNA is complementary to a region of a templated ligation probe targeted for reduction or exclusion. In some instances, the LNA is about 60%, 65%, 70%, 75%, 80%, 85%, 90%, 91%, 92%, 93%, 94%, 95%, 96%, 97%, 98%, 99%, or 100% complementary to a region of a templated ligation probe. Thus, in some instances, the LNA specifically binds (e.g., hybridizes) to and is complementary (e.g., 60%, 65%, 70%, 75%, 80%, 85%, 90%, 91%, 92%, 93%, 94%, 95%, 96%, 97%, 98%, 99%, or 100%) to the templated ligation probe.

In some embodiments, each LNA can increase melting temperature (Tm) of the blocker oligonucleotide by at least 1°C, at least 2°C, at least 3°C, at least 4°C, or at least 5°C as compared to a reference naturally-occurring oligonucleotide with the same sequence. In some embodiments, Tm of the blocker oligonucleotide comprising one or more LNAs is about 10%, about 20%, about 30%, about 40%, about 50%, about 60%, about 70%, about 80%, about 90%, about 100%, or higher than Tm of a reference oligonucleotide with the same sequence except that the LNA is replaced by the corresponding oligonucleotide. In some embodiments, the higher Tm of the blocker oligonucleotide comprising one or more LNAs leads to its increased binding affinity to the templated ligation probe. In some embodiments, the blocker oligonucleotide comprising one or more LNAs has a similar Tm compared to a reference oligonucleotide sharing an identical sequence without LNAs, in which case the blocker oligonucleotide is at least 1, 2, 3, 4, 5, 6, 7, 8, 9, 10, 11, 12, 13, 14, 15, 16, 17, 18, 19, 20 nucleotides shorter than the reference oligonucleotide. In some embodiments, the length of the blocker oligonucleotide, and number and/or positions of incorporated LNAs are determined according to the blocker oligonucleotide's Tm. In some embodiments, the Tm of the blocker oligonucleotide is determined according to the highest temperature used during the spatial analysis workflow (e.g., extension or amplification).

In some embodiments, the one or more LNAs within the blocker oligonucleotide can increase its binding affinity to the nucleic acid targeted for reduction or exclusion by at least 1 fold, at least 5 fold, at least 10 fold, at least 20 fold, at least 30 fold, at least 40 fold, at least 50 fold, at least 60 fold, at least 70 fold, at least 80 fold, at least 90 fold, at least 100 fold, or more as compared to the binding affinity of the same blocker oligonucleotide except that the LNA is replaced by the corresponding DNA. In some embodiments, because of the increased binding affinity to the nucleic acid, the blocker oligonucleotide does not disassociate from the templated ligation probe during the templated ligation probe hybridization step described herein.

In some embodiments, the blocker oligonucleotide includes a degenerate sequence. In some instances, the blocker oligonucleotide described herein comprises one or more (e.g., 1, 2, 3, 4, 5, 6, 7, 8, 9, 10, or more) degenerate sequences. In some embodiments, the degenerate sequence can be a degenerate nucleotide sequence. In some embodiments, the degenerate nucleotide sequence includes about or at least 1, 2, 3, 4, 5, 6, 7, 8, 9, 10, 11, 12, 13, 14, 15, 16, 17, 18, 19, 20, 25, 30, 35, 40, 45, or 50 nucleotides. In some embodiments, the degenerate nucleotides are at least or about 5%, 10%, 20%, 30%, 40%, 50%, 60%, 70%, 80%, 90%, or 100% of the blocker oligonucleotide. In some embodiments, the degenerate sequences are flanked by non-degenerate sequences on one end or on both ends. In some embodiments, the degenerate nucleotides comprise the non-natural nucleic acid (e.g., the LNA) described herein.

In some embodiments, the modified blocker nucleotides described herein (e.g., LNA-modified antisense oligonucleotides, or antisense oligonucleotides including one or more inverted thymines) can be used to silence template ligation pairs targeted to a particular gene.

### (e) Probes for Templated Ligation

The methods provided herein utilize probe pairs (or sets; the terms are interchangeable). In some instances, the probe pairs are designed so that each probe hybridizes to a sequence in an analyte that is specific to the analyte (e.g., compared to the entire genome). That is, a single probe pair can be specific to a single analyte.

In other embodiments, probes can be designed so that one of the probes of a pair is a probe that hybridizes to a specific sequence. Then, the other probe can be designed to detect a mutation of interest. Accordingly, in some instances, multiple second probes can be designed and can vary so that each binds to a specific sequence. For example, one second probe can be designed to hybridize to a wild-type sequence, and another second probe can be designed to detect a mutated sequence. Thus, in some instances, a probe set can include one first probe and two second probes (or vice versa). In some embodiments, the probe set can be silenced by blocker oligonucleotides. For example, each of the first probe and two second probes can be hybridized with a blocker oligonucleotide such that the blocked probe is unable to hybridize to the intended target analyte.

On the other hand, in some instances, probes can be designed so that they cover conserved regions of an analyte. Thus, in some instances, a probe (or probe pair) can hybridize to similar analytes in a biological sample (e.g., to detect conserved or similar analytes) or in different biological samples (e.g., across different species). In some cases, such probes can be silenced thereby reducing the spatial analysis reads of the similar analytes.

In some embodiments, probe sets cover all or nearly all of a genome (e.g., human genome). In instances where probe sets are designed to cover an entire genome (e.g., the human genome), the methods disclosed herein can detect analytes in an unbiased manner. In some instances, one probe oligonucleotide pair is designed to cover one analyte (e.g., transcript). In some instances, more than one probe oligonucleotide pair (e.g., a probe pair comprising a first probe and a second probe) is designed to hybridize to one analyte (e.g., transcript). For example, at least two, three, four, five, six, seven, eight, nine, ten, or more probe sets can be used to hybridize to a single analyte. Factors to consider when designing probes is presence of variants (e.g., SNPs, mutations) or multiple isoforms expressed by a single gene. In such cases, more than one blocker oligonucleotide pairs can be designed to silence each probe oligonucleotide pair. Alternatively, more than one blocker oligonucleotides can be designed to silence a subset of probe oligonucleotide pairs.

In some instances, the probe oligonucleotide pair does not hybridize to the entire analyte (e.g., a transcript), but instead the probe oligonucleotide pair hybridizes to a portion of the entire analyte (e.g., transcript). In such cases, a blocker oligonucleotide pair can be designed to block hybridization between the probe oligonucleotide pair and the portion of the entire analyte.

In some instances, about 5000, 10,000, 15,000, 20,000, or more probe oligonucleotide pairs are used in the methods described herein. In some instances, about 20,000 probe oligonucleotide pairs are used in the methods described herein. In some embodiments, at least 1, at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, or at least 1000 blocker oligonucleotide pairs are used in the methods described herein.

In some instances, RNA capture is targeted RNA capture. Targeted RNA capture using the methods disclosed herein allows for examination of a subset of RNA analytes from the entire transcriptome. In some embodiments, the subset of analytes includes an individual target RNA. In some embodiments, the subset of analytes includes two or more targeted RNAs. In some embodiments, the subset of analytes includes one or more mRNAs transcribed by one or more targeted genes. In some embodiments, the subset of analytes includes one or more mRNA splice variants of one or more targeted genes. In some embodiments, the subset of analytes includes non-polyadenylated RNAs in a biological sample. In some embodiments, the subset of analytes includes detection of mRNAs having one or more single nucleotide polymorphisms (SNPs) in a biological sample. In some embodiments, blocker oligonucleotides are designed to block templated ligation probes targeting the subset of RNA analytes.

In some embodiments, the subset of analytes includes mRNAs that mediate expression of a set of genes of interest. In some embodiments, the subset of analytes includes mRNAs that share identical or substantially similar sequences, which mRNAs are translated into polypeptides having similar functional groups or protein domains. In some embodiments, the subset of analytes includes mRNAs that do not share identical or substantially similar sequences, which mRNAs are translated into proteins that do not share similar functional groups or protein domains. In some embodiments, the subset of analytes includes mRNAs that are translated into proteins that function in the same or similar biological pathways. In some embodiments, the biological pathways are associated with a pathologic disease. For example, targeted RNA capture can detect genes that are overexpressed or underexpressed in cancer.

In some embodiments, the subset of analytes includes 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, about 55, about 60, about 65, about 70, about 75, about 80, about 85, about 90, about 95, about 100, about 110, about 120, about 130, about 140, about 150, about 160, about 170, about 180, about 190, about 200, about 225, about 250, about 275, about 300, about 325, about 350, about 375, about 400, about 425, about 450, about 475, about 500, about 600, about 700, about 800, about 900, or about 1000 analytes.

In some instances, the methods disclosed herein can detect the abundance and/or location of at least 5,000, 10,000, 15,000, 20,000, or more different analytes. In some instances, the methods disclosed herein can be used to selectively silence templated ligation probes targeting at least 1, at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, or at least 1000 analytes.

In some embodiments, the subset of analytes detected by targeted RNA capture methods provided herein includes a large proportion of the transcriptome of one or more cells. For example, the subset of analytes detected by targeted RNA capture methods provided herein can include at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or more of the mRNAs present in the transcriptome of one or more cells.

In some instances, the probes are DNA probes. In some instances, the probes are diribo-containing probes.

Additional embodiments of probe(s) and probe set(s) are described herein.

### (i) First Probe

In some embodiments, the methods described herein include a first probe. As used herein, a "first probe" can refer to a probe that hybridizes to all or a portion of an analyte and can be ligated to one or more additional probes (e.g., a second probe or a spanning probe). In some embodiments, "first probe" can be used interchangeably with "first probe oligonucleotide." In some embodiments, the first probe can be silenced using the methods described herein.

In some embodiments, the first probe includes ribonucleotides, deoxyribonucleotides, and/or synthetic nucleotides that are capable of participating in Watson-Crick type or analogous base pair interactions. In some embodiments, the first probe includes deoxyribonucleotides. In some embodiments, the first probe includes deoxyribonucleotides and ribonucleotides. In some embodiments, the first probe includes a deoxyribonucleic acid that hybridizes to an analyte, and includes a portion of the first probe that is not a deoxyribonucleic acid. For example, in some embodiments, the portion of the first probe that is not a deoxyribonucleic acid is a ribonucleic acid or any other non-deoxyribonucleic acid nucleic acid as described herein. In some embodiments where the first probe includes deoxyribonucleotides, hybridization of the first probe to the mRNA molecule results in a DNA:RNA hybrid. In some embodiments, the first probe includes only deoxyribonucleotides and upon hybridization of the first probe to the mRNA molecule results in a DNA:RNA hybrid.

In some embodiments, the method includes a first probe that includes one or more sequences that are substantially complementary to one or more sequences of an analyte. In some embodiments, a first probe includes a sequence that is substantially complementary to a first target sequence in the analyte. In some embodiments, the sequence of the first probe that is substantially complementary to the first target sequence in the analyte is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to the first target sequence in the analyte. In some embodiments, a first blocker oligonucleotide hybridizes to the sequence in the first probe that is substantially complementary to the first target sequence in the analyte. **In** some embodiments, the first blocker oligonucleotide hybridizes to substantially the entire sequence of the first probe.

**In** some embodiments, a first probe includes a sequence that is about 10 nucleotides to about 100 nucleotides (e.g., a sequence of about 10 nucleotides to about 90 nucleotides, about 10 nucleotides to about 80 nucleotides, about 10 nucleotides to about 70 nucleotides, about 10 nucleotides to about 60 nucleotides, about 10 nucleotides to about 50 nucleotides, about 10 nucleotides to about 40 nucleotides, about 10 nucleotides to about 30 nucleotides, about 10 nucleotides to about 20 nucleotides, about 20 nucleotides to about 100 nucleotides, about 20 nucleotides to about 90 nucleotides, about 20 nucleotides to about 80 nucleotides, about 20 nucleotides to about 70 nucleotides, about 20 nucleotides to about 60 nucleotides, about 20 nucleotides to about 50 nucleotides, about 20 nucleotides to about 40 nucleotides, about 20 nucleotides to about 30 nucleotides, about 30 nucleotides to about 100 nucleotides, about 30 nucleotides to about 90 nucleotides, about 30 nucleotides to about 80 nucleotides, about 30 nucleotides to about 70 nucleotides, about 30 nucleotides to about 60 nucleotides, about 30 nucleotides to about 50 nucleotides, about 30 nucleotides to about 40 nucleotides, about 40 nucleotides to about 100 nucleotides, about 40 nucleotides to about 90 nucleotides, about 40 nucleotides to about 80 nucleotides, about 40 nucleotides to about 70 nucleotides, about 40 nucleotides to about 60 nucleotides, about 40 nucleotides to about 50 nucleotides, about 50 nucleotides to about 100 nucleotides, about 50 nucleotides to about 90 nucleotides, about 50 nucleotides to about 80 nucleotides, about 50 nucleotides to about 70 nucleotides, about 50 nucleotides to about 60 nucleotides, about 60 nucleotides to about 100 nucleotides, about 60 nucleotides to about 90 nucleotides, about 60 nucleotides to about 80 nucleotides, about 60 nucleotides to about 70 nucleotides, about 70 nucleotides to about 100 nucleotides, about 70 nucleotides to about 90 nucleotides, about 70 nucleotides to about 80 nucleotides, about 80 nucleotides to about 100 nucleotides, about 80 nucleotides to about 90 nucleotides, or about 90 nucleotides to about 100 nucleotides).

**In** some embodiments, a sequence of the first probe that is substantially complementary to a sequence in the analyte includes a sequence that is about 5 nucleotides to about 50 nucleotides (e.g., about 5 nucleotides to about 45 nucleotides, about 5 nucleotides to about 40 nucleotides, about 5 nucleotides to about 35 nucleotides, about 5 nucleotides to about 30 nucleotides, about 5 nucleotides to about 25 nucleotides, about 5 nucleotides to about 20 nucleotides, about 5 nucleotides to about 15 nucleotides, about 5 nucleotides to about 10 nucleotides, about 10 nucleotides to about 50 nucleotides, about 10 nucleotides to about 45 nucleotides, about 10 nucleotides to about 40 nucleotides, about 10 nucleotides to about 35 nucleotides, about 10 nucleotides to about 30 nucleotides, about 10 nucleotides to about 25 nucleotides, about 10 nucleotides to about 20 nucleotides, about 10 nucleotides to about 15 nucleotides, about 15 nucleotides to about 50 nucleotides, about 15 nucleotides to about 45 nucleotides, about 15 nucleotides to about 40 nucleotides, about 15 nucleotides to about 35 nucleotides, about 15 nucleotides to about 30 nucleotides, about 15 nucleotides to about 25 nucleotides, about 15 nucleotides to about 20 nucleotides, about 20 nucleotides to about 50 nucleotides, about 20 nucleotides to about 45 nucleotides, about 20 nucleotides to about 40 nucleotides, about 20 nucleotides to about 35 nucleotides, about 20 nucleotides to about 30 nucleotides, about 20 nucleotides to about 25 nucleotides, about 25 nucleotides to about 50 nucleotides, about 25 nucleotides to about 45 nucleotides, about 25 nucleotides to about 40 nucleotides, about 25 nucleotides to about 35 nucleotides, about 25 nucleotides to about 30 nucleotides, about 30 nucleotides to about 50 nucleotides, about 30 nucleotides to about 45 nucleotides, about 30 nucleotides to about 40 nucleotides, about 30 nucleotides to about 35 nucleotides, about 35 nucleotides to about 50 nucleotides, about 35 nucleotides to about 45 nucleotides, about 35 nucleotides to about 40 nucleotides, about 40 nucleotides to about 50 nucleotides, about 40 nucleotides to about 45 nucleotides, or about 45 nucleotides to about 50 nucleotides).

In some embodiments, a first probe includes a functional sequence. In some embodiments, a functional sequence includes a primer sequence. In some embodiments, a first blocker oligonucleotide does not include a sequence that is substantially complementary to the functional sequence.

In some embodiments, a first probe includes at least two ribonucleic acid bases at the 3' end. In such cases, a second probe oligonucleotide comprises a phosphorylated nucleotide at the 5' end. In some embodiments, a first probe includes at least three, at least four, at least five, at least six, at least seven, at least eight, at least nine, or at least ten ribonucleic acid bases at the 3' end.

In some embodiments, a first probe includes an auxiliary sequence that does not hybridize to an analyte. In some embodiments, the auxiliary sequence can be used to hybridize to additional probes. In some embodiments, a first blocker oligonucleotide that can hybridize with the first probe does not include a sequence that is substantially complementary to the auxiliary sequence.

### (ii) Second Probe

In some embodiments, the methods described herein include a second probe. As used herein, a "second probe" can refer to a probe that hybridizes to all or a portion of an analyte and can be ligated to one or more additional probes (e.g., a first probe or a spanning probe). In some embodiments, "second probe" can be used interchangeably with "second probe oligonucleotide." In some embodiments, the second probe can be silenced using the methods described herein. One of skill in the art will appreciate that the order of the probes is arbitrary, and thus the contents of the first probe and/or second probe as disclosed herein are interchangeable.

In some embodiments, the second probe includes ribonucleotides, deoxyribonucleotides, and/or synthetic nucleotides that are capable of participating in Watson-Crick type or analogous base pair interactions. In some embodiments, the second probe includes deoxyribonucleotides. In some embodiments, the second probe includes deoxyribonucleotides and ribonucleotides. In some embodiments, the second probe includes a deoxyribonucleic acid that hybridizes to an analyte, and includes a portion of the second probe that is not a deoxyribonucleic acid. For example, in some embodiments, the portion of the second probe that is not a deoxyribonucleic acid is a ribonucleic acid or any other non-deoxyribonucleic acid nucleic acid as described herein. In some embodiments where the second probe includes deoxyribonucleotides, hybridization of the second probe to the mRNA molecule results in a DNA:RNA hybrid. In some embodiments, the second probe includes only deoxyribonucleotides and upon hybridization of the first probe to the mRNA molecule results in a DNA:RNA hybrid.

In some embodiments, the method includes a second probe that includes one or more sequences that are substantially complementary to one or more sequences of an analyte. In some embodiments, a second probe includes a sequence that is substantially complementary to a second target sequence in the analyte. In some embodiments, the sequence of the second probe that is substantially complementary to the second target sequence in the analyte is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to the second target sequence in the analyte. In some embodiments, a second blocker oligonucleotide hybridizes to the sequence in second first probe that is substantially complementary to the second target sequence in the analyte. In some embodiments, the second blocker oligonucleotide hybridizes to substantially the entire sequence of the second probe.

**In** some embodiments, a second probe includes a sequence that is about 10 nucleotides to about 100 nucleotides (e.g., a sequence of about 10 nucleotides to about 90 nucleotides, about 10 nucleotides to about 80 nucleotides, about 10 nucleotides to about 70 nucleotides, about 10 nucleotides to about 60 nucleotides, about 10 nucleotides to about 50 nucleotides, about 10 nucleotides to about 40 nucleotides, about 10 nucleotides to about 30 nucleotides, about 10 nucleotides to about 20 nucleotides, about 20 nucleotides to about 100 nucleotides, about 20 nucleotides to about 90 nucleotides, about 20 nucleotides to about 80 nucleotides, about 20 nucleotides to about 70 nucleotides, about 20 nucleotides to about 60 nucleotides, about 20 nucleotides to about 50 nucleotides, about 20 nucleotides to about 40 nucleotides, about 20 nucleotides to about 30 nucleotides, about 30 nucleotides to about 100 nucleotides, about 30 nucleotides to about 90 nucleotides, about 30 nucleotides to about 80 nucleotides, about 30 nucleotides to about 70 nucleotides, about 30 nucleotides to about 60 nucleotides, about 30 nucleotides to about 50 nucleotides, about 30 nucleotides to about 40 nucleotides, about 40 nucleotides to about 100 nucleotides, about 40 nucleotides to about 90 nucleotides, about 40 nucleotides to about 80 nucleotides, about 40 nucleotides to about 70 nucleotides, about 40 nucleotides to about 60 nucleotides, about 40 nucleotides to about 50 nucleotides, about 50 nucleotides to about 100 nucleotides, about 50 nucleotides to about 90 nucleotides, about 50 nucleotides to about 80 nucleotides, about 50 nucleotides to about 70 nucleotides, about 50 nucleotides to about 60 nucleotides, about 60 nucleotides to about 100 nucleotides, about 60 nucleotides to about 90 nucleotides, about 60 nucleotides to about 80 nucleotides, about 60 nucleotides to about 70 nucleotides, about 70 nucleotides to about 100 nucleotides, about 70 nucleotides to about 90 nucleotides, about 70 nucleotides to about 80 nucleotides, about 80 nucleotides to about 100 nucleotides, about 80 nucleotides to about 90 nucleotides, or about 90 nucleotides to about 100 nucleotides).

In some embodiments, a sequence of the second probe that is substantially complementary to a sequence in the analyte includes a sequence that is about 5 nucleotides to about 50 nucleotides (e.g., about 5 nucleotides to about 45 nucleotides, about 5 nucleotides to about 40 nucleotides, about 5 nucleotides to about 35 nucleotides, about 5 nucleotides to about 30 nucleotides, about 5 nucleotides to about 25 nucleotides, about 5 nucleotides to about 20 nucleotides, about 5 nucleotides to about 15 nucleotides, about 5 nucleotides to about 10 nucleotides, about 10 nucleotides to about 50 nucleotides, about 10 nucleotides to about 45 nucleotides, about 10 nucleotides to about 40 nucleotides, about 10 nucleotides to about 35 nucleotides, about 10 nucleotides to about 30 nucleotides, about 10 nucleotides to about 25 nucleotides, about 10 nucleotides to about 20 nucleotides, about 10 nucleotides to about 15 nucleotides, about 15 nucleotides to about 50 nucleotides, about 15 nucleotides to about 45 nucleotides, about 15 nucleotides to about 40 nucleotides, about 15 nucleotides to about 35 nucleotides, about 15 nucleotides to about 30 nucleotides, about 15 nucleotides to about 25 nucleotides, about 15 nucleotides to about 20 nucleotides, about 20 nucleotides to about 50 nucleotides, about 20 nucleotides to about 45 nucleotides, about 20 nucleotides to about 40 nucleotides, about 20 nucleotides to about 35 nucleotides, about 20 nucleotides to about 30 nucleotides, about 20 nucleotides to about 25 nucleotides, about 25 nucleotides to about 50 nucleotides, about 25 nucleotides to about 45 nucleotides, about 25 nucleotides to about 40 nucleotides, about 25 nucleotides to about 35 nucleotides, about 25 nucleotides to about 30 nucleotides, about 30 nucleotides to about 50 nucleotides, about 30 nucleotides to about 45 nucleotides, about 30 nucleotides to about 40 nucleotides, about 30 nucleotides to about 35 nucleotides, about 35 nucleotides to about 50 nucleotides, about 35 nucleotides to about 45 nucleotides, about 35 nucleotides to about 40 nucleotides, about 40 nucleotides to about 50 nucleotides, about 40 nucleotides to about 45 nucleotides, or about 45 nucleotides to about 50 nucleotides).

In some embodiments, a second probe includes a capture probe capture domain sequence. As used herein, a "capture probe capture domain" is a sequence, domain, or moiety that can bind specifically to a capture domain of a capture probe. In some embodiments, "capture domain capture domain" can be used interchangeably with "capture probe binding domain." In some embodiments, a second probe includes a sequence from 5' to 3': a sequence that is substantially complementary to a sequence in the analyte and a capture probe capture domain. In some embodiments, a second blocker oligonucleotide does not include a sequence that is substantially complementary to capture probe capture domain.

In some embodiments, a capture probe capture domain includes a poly(A) sequence. In some embodiments, the capture probe capture domain includes a poly-uridine sequence, a poly-thymidine sequence, or both. In some embodiments, the capture probe capture domain includes a random sequence (e.g., a random hexamer or octamer). In some embodiments, the capture probe capture domain is complementary to a capture domain in a capture probe that detects a particular target(s) of interest. In some embodiments, a capture probe capture domain blocking moiety that interacts with the capture probe capture domain is provided. In some embodiments, a capture probe capture domain blocking moiety includes a sequence that is complementary or substantially complementary to a capture probe capture domain. In some embodiments, a capture probe capture domain blocking moiety prevents the capture probe capture domain from binding the capture probe when present. In some embodiments, a capture probe capture domain blocking moiety is removed prior to binding the capture probe capture domain (e.g., present in a ligated probe) to a capture probe. In some embodiments, a capture probe capture domain blocking moiety includes a poly-uridine sequence, a poly-thymidine sequence, or both. In some embodiments, the capture probe capture domain sequence includes ribonucleotides, deoxyribonucleotides, and/or synthetic nucleotides that are capable of participating in Watson-Crick type or analogous base pair interactions. In some embodiments, the capture probe binding domain sequence includes at least 10, 11, 12, 13, 14, 15, 16, 17, 18, 19, or 20 nucleotides. In some embodiments, the capture probe binding domain sequence includes at least 25, 30, or 35 nucleotides.

In some embodiments, a second probe includes a phosphorylated nucleotide at the 5' end. The phosphorylated nucleotide at the 5' end can be used in a ligation reaction to ligate the second probe to the first probe.

In some embodiments, a second probe includes an auxiliary sequence that does not hybridize to an analyte. In some embodiments, the auxiliary sequence can be used to hybridize to additional probes. In some embodiments, a second blocker oligonucleotide that can hybridize with the second probe does not include a sequence that is substantially complementary to the auxiliary sequence.

### (iii) Multiple Probes

In some embodiments, the methods of target RNA capture as disclosed herein include multiple probe oligonucleotides. In some embodiments, the methods include 2, 3, 4, or more probe oligonucleotides. In some embodiments, each of the probe oligonucleotides includes ribonucleotides, deoxyribonucleotides, and/or synthetic nucleotides that are capable of participating in Watson-Crick type or analogous base pair interactions. In some embodiments, each of the probe oligonucleotides includes deoxyribonucleotides. In some embodiments, each of the probe oligonucleotides includes deoxyribonucleotides and ribonucleotides. In some embodiments, blocker oligonucleotides can be designed to silence one or more of the multiple probe oligonucleotides.

In some instances, the methods include a first probe and multiple second probes (or vice versa) are used, with the multiple second probes hybridizing to different sequences (e.g., wild-type versus mutant sequence, different isoforms, splice variants) in order to identify the sequence of an analyte. It is appreciated that this method can be utilized to detect single mutations (e.g., point mutations, SNPs, splice variants, etc.) or can multi-nucleotide mutations (e.g., insertions, deletions, etc.). In some embodiments, the first probe and multiple second probes can be silenced by blocker oligonucleotides such that each of the first probe and multiple second probes can be hybridized with a blocker oligonucleotide.

Methods provided herein may be applied to a single nucleic acid molecule or a plurality of nucleic acid molecules. A method of analyzing a sample comprising a nucleic acid molecule may comprise providing a plurality of nucleic acid molecules (e.g., RNA molecules), where each nucleic acid molecule comprises a first target region (e.g., a first target sequence) and a second target region (e.g., a second target sequence), a plurality of first probe oligonucleotides, and a plurality of second probe oligonucleotides. In some cases, one or more target regions of nucleic acid molecules of the plurality of nucleic acid molecules may comprise the same sequence. The first and second target regions (e.g., the first and second target sequences) of a nucleic acid molecule of the plurality of nucleic acid molecules may be adjacent to one another. In some embodiments, the methods described herein may also include a plurality of first blocker oligonucleotides and a plurality of second oligonucleotides. In some cases, a first blocker oligonucleotide of the plurality of first blocker oligonucleotides can be used to block a first probe of the plurality of first probe oligonucleotides hybridizing to the one or more target regions of nucleic acid molecules. In some cases, a second blocker oligonucleotide of the plurality of second blocker oligonucleotides can be used to block a second probe of the plurality of second probe oligonucleotides hybridizing to the one or more target regions of nucleic acid molecules.

### (iv) First Probe Having a Linker Sequence

Also provided herein are methods for identifying a location of an analyte in a biological sample where the method includes a first probe that includes a linker and a second probe. Using a pair of probes where the first probe includes a linker sequence enables greater flexibility in designing templated ligation probes, primarily by increasing the sequences within the analyte that can be used as optional target sequences. In some embodiments, the first probe having a linker sequence can be silenced using one or more blocker oligonucleotides described herein.

As used herein, a "linker sequence" can refer to one or more nucleic acids sequences on a probe (e.g., a first probe, a second probe, or a spanning probe that are disposed between sequences that hybridize to an analyte, sequences that link together the analyte specific sequences of a probe). In some embodiments, a linker includes a sequence that is not substantially complementary to either the sequence of the target analyte or to the analyte specific sequences of a first probe, a second probe, or a spanning probe. In some embodiments, the linker sequence includes ribonucleotides, deoxyribonucleotides, and/or synthetic nucleotides, where the sequence within the linker is not substantially complementary to the target analyte or the analyte specific sequences of a first probe, a second probe, or a spanning probe. In some embodiments where a first and/or a second probe include a linker sequence, a blocker oligonucleotide includes a sequence that is substantially complementary to the linker sequence. In some embodiments, a blocker oligonucleotide does not include a sequence that is substantially complementary to the linker sequence.

In some embodiments where a first and/or a second probe include a linker sequence, the linker sequence can include a total of about 10 nucleotides to about 100 nucleotides, or any of the subranges described herein.

In some embodiments, a linker sequence includes a barcode sequence that serves as a proxy for identifying the analyte. In some embodiments, the barcode sequence is a sequence that is at least 70% identical (e.g., at least 75% identical, at least 80% identical, at least 85% identical, at least 90% identical, at least 95% identical, or at least 99% identical) to a sequence in the analyte. In some embodiments where a linker sequence includes a barcode sequence, the barcode sequence is located 5' to the linker sequence. In some embodiments where a linker sequence includes a barcode sequence, the barcode sequence is located 3' to the linker sequence. In some embodiments, the barcode sequence is disposed between two linker sequences. In such cases, the two linker sequences flanking the barcode sequence can be considered to be a part of the same linker sequence. In some embodiments where a linker sequence includes a barcode sequence, a blocker oligonucleotide includes a sequence that is substantially complementary to the barcode sequence. In some embodiments where a linker sequence includes a barcode sequence, a blocker oligonucleotide does not include a sequence that is substantially complementary to the barcode sequence.

In some embodiments where a first and/or a second probe include a linker sequence, the linker sequence can include ribonucleotides, deoxyribonucleotides, and/or synthetic nucleotides.

A non-limiting example of a method for identifying a location of an analyte in a biological sample includes a first probe that includes a linker sequence and a second probe comprising: (a) contacting the biological sample with a substrate including a plurality of capture probes, wherein a capture probe of the plurality of capture probes includes a capture domain and a spatial barcode; (b) contacting the biological sample with a first probe and a second probe, wherein a portion of the first probe and a portion of the second probe are substantially complementary to adjacent sequences of the analyte, wherein the first probe includes: (i) a first sequence that is substantially complementary to a first target sequence of the analyte; (ii) a linker sequence; (iii) a second sequence that is substantially complementary to a second target sequence of the analyte; and wherein the second probe includes a sequence that is substantially complementary to a third target sequence of the analyte and a capture probe capture domain that is capable of binding to a capture domain of a capture probe; (c) hybridizing the first probe and the second probe to the analyte; (d) ligating the first probe and the second probe, thereby creating a ligation product; (e) releasing the ligation product from the analyte; (f) hybridizing the capture probe binding domain to a capture domain; and (g) determining (i) all or a part of the sequence of the ligation product specifically bound to the capture domain, or a complement thereof, and (ii) all or a part of the sequence of the spatial barcode, or a complement thereof, and using the determined sequence of (i) and (ii) to identify the location of the analyte in the biological sample. Here, blocker oligonucleotides can be added to the sample concurrently with the first probe and the second probe, such that the blocker oligonucleotides can hybridize to probes targeted reduction or exclusion, thereby blocking their hybridization to the target analyte (e.g., an analyte targeted for reduction or exclusion from a spatial transcriptomics assay).

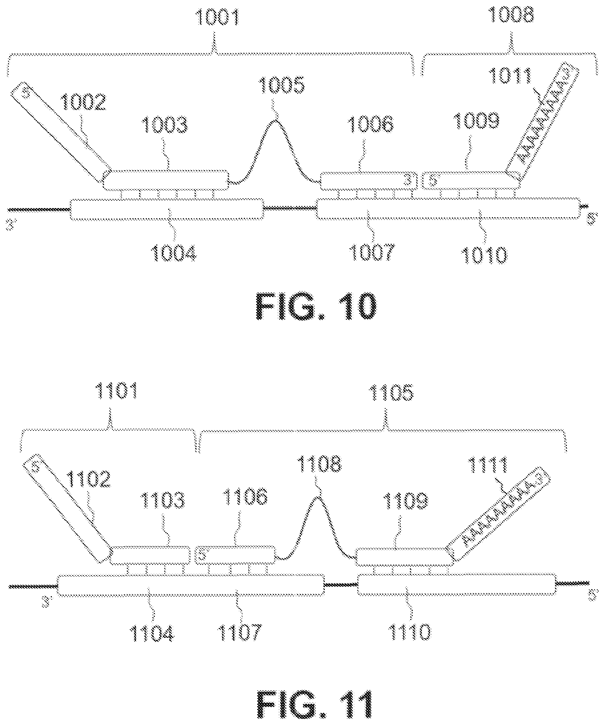

A non-limiting example of a method for identifying a location of an analyte in a biological sample where the method includes a first probe that includes a linker and a second probe can include the components as shown **FIG. 10****.** A first probe **1001** includes a functional sequence **1002,** a first sequence **1003** that is substantially complementary to a first target sequence **1004** of the analyte, a linker sequence **1005;** and a second sequence **1006** that is substantially complementary to a second target sequence **1007** of the analyte. A second probe **1008** includes a sequence **1009** that is substantially complementary to a third target sequence **1010** of the analyte and a capture probe capture domain **1011** that is capable of binding to a capture domain of a capture probe.

In situations when the analyte is an analyte targeted for reduction or exclusion from a spatial transcriptomics assay, a first blocker oligonucleotide can be designed to hybridize with the first sequence **1003** for the first probe **1001;** and/or a second blocker oligonucleotide can be designed to hybridize with the second sequence **1006** of the first probe **1001;** and/or a third blocker oligonucleotide can be designed to hybridize with the sequence **1009** of the second probe **1008.** Alternatively, when the analyte is an analyte targeted for reduction or exclusion, a first blocker oligonucleotide can be designed to hybridize with a sequence including the first sequence **1003,** optionally the linker sequence **1005,** and the second sequence **1006;** and a second blocker oligonucleotide can be designed to hybridize with the sequence **1009** of the second probe **1008.** Alternatively, when the analyte is an analyte targeted for reduction or exclusion, a first blocker oligonucleotide can be designed to hybridize with the first sequence **1003** of the first probe **1001;** and/or a second blocker oligonucleotide can be designed to hybridize with the second sequence **1006** (of the first probe **1001)** and the sequence **1009** (of the second probe **1008).** Alternatively, when the analyte is an analyte targeted for reduction or exclusion, a single blocker oligonucleotide can be designed to hybridize with the first sequence **1003** (of the first probe **1001),** optionally the linker sequence **1005,** the second sequence **1006** (of the first probe **1001),** and the sequence **1009** (of the second probe **1008).** In all the cases above, the blocker oligonucleotide(s) can block the first probe **1001** and/or the second probe **1008** from hybridizing to the analyte targeted for reduction or exclusion. Without proper hybridization, negligible amounts of ligation product may be generated from the first probe **1001** and the second probe **1008.** As a result, the first probe **1001** and the second probe **1008** are silenced by the blocker oligonucleotide(s).

### 1) First probe

In some embodiments where first probe includes a linker sequence, the first probe includes a first sequence that is substantially complementary to a first target sequence of the analyte, a linker sequence, and a second sequence that is substantially complementary to second target sequence of the analyte. In some embodiments, a first probe includes from 5' to 3': a first sequence that is substantially complementary to a first target sequence of the analyte, a linker sequence, and a second sequence that is substantially complementary to second target sequence of the analyte.

In some embodiments where a first probe includes a linker sequence, the first probe includes a functional sequence. In some embodiments, a first probe includes a functional sequence, a first sequence that is substantially complementary to a first target sequence of the analyte, a linker sequence, and a second sequence that is substantially complementary to second target sequence of the analyte. In some embodiments, the functional sequence includes a primer sequence. In some embodiments, a blocker oligonucleotide used to silence the first probe includes a sequence that is substantially complementary to the functional sequence. In some embodiments, the blocker oligonucleotide does not include a sequence that is substantially complementary to the functional sequence. In some embodiments, a blocker oligonucleotide used to silence the first probe includes a sequence that is substantially complementary to the first sequence. In some embodiments, the blocker oligonucleotide does not include a sequence that is substantially complementary to the first sequence. In some embodiments, the blocker oligonucleotide includes a sequence that is substantially complementary to the linker sequence. In some embodiments, the blocker oligonucleotide does not include a sequence that is substantially complementary to the linker sequence. In some embodiments, the blocker oligonucleotide includes a sequence that is substantially complementary to the second sequence. In some embodiments, the blocker oligonucleotide does not include a sequence that is substantially complementary to the second sequence.

In some embodiments where a first probe includes a linker sequence, a first probe includes at least two ribonucleic acid bases at the 3' end. In some embodiments, a first probe includes at least three, at least four, at least five, at least six, at least seven, at least eight, at least nine, or at least ten ribonucleic acid bases at the 3' end.

In some embodiments where a first probe includes a linker, the first probe includes a sequence that is about 10 nucleotides to about 300 nucleotides (e.g., a sequence of about 10 nucleotides to about 300 nucleotides, about 10 nucleotides to about 250 nucleotides, about 10 nucleotides to about 200 nucleotides, about 10 nucleotides to about 150 nucleotides, about 10 nucleotides to about 100 nucleotides, about 10 nucleotides to about 50 nucleotides, about 50 nucleotides to about 300 nucleotides, about 50 nucleotides to about 250 nucleotides, about 50 nucleotides to about 200 nucleotides, about 50 nucleotides to about 150 nucleotides, about 50 nucleotides to about 100 nucleotides, about 100 nucleotides to about 300 nucleotides, about 100 nucleotides to about 250 nucleotides, about 100 nucleotides to about 200 nucleotides, about 100 nucleotides to about 150 nucleotides, about 150 nucleotides to about 300 nucleotides, about 150 nucleotides to about 250 nucleotides, about 150 nucleotides to about 200 nucleotides, about 200 nucleotides to about 300 nucleotides, about 200 nucleotides to about 250 nucleotides, or about 250 nucleotides to about 300 nucleotides).

In some embodiments where a first probe includes a linker sequence, the first probe includes a first sequence that is substantially complementary to a first target sequence of the analyte. In some embodiments, the first sequence of the first probe is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to the first target sequence in the analyte. In some embodiments, the first sequence of the first probe that is substantially complementary to a first target sequence can include a sequence that is about 5 nucleotides to about 50 nucleotides, or any of the subranges described herein.

In some embodiments where a first probe includes a linker, the first probe includes a second sequence that is substantially complementary to a second target sequence of the analyte. In some embodiments, the second sequence of the first probe is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to the second target sequence in the analyte. In some embodiments, the second sequence of the first probe that is substantially complementary to a second target sequence can include a sequence that is about 5 nucleotides to about 50 nucleotides, or any of the subranges described herein.

In some embodiments, a blocker oligonucleotide used for silencing the first probe includes a sequence that is about 5 nucleotides to about 50 nucleotides, or any of the subranges described herein.

In some embodiments, a first probe that includes a linker sequence includes ribonucleotides, deoxyribonucleotides, and/or synthetic nucleotides that are capable of participating in Watson-Crick type or analogous base pair interactions. In some embodiments, the first probe that includes a linker sequence includes deoxyribonucleotides. In some embodiments, the first probe that includes a linker sequence includes deoxyribonucleotides and ribonucleotides. In some embodiments where the first probe that includes a linker sequence includes deoxyribonucleotides, hybridization of the first probe to the mRNA molecule results in a DNA:RNA hybrid. In some embodiments, the first probe that includes a linker sequence includes only deoxyribonucleotides and upon hybridization of the first probe to the mRNA molecule results in a DNA:RNA hybrid.

### 2) Second probe

In some embodiments where a first probe includes a linker sequence, a second probe includes a sequence that is substantially complementary to a third target sequence of the analyte and a capture probe capture domain that is capable of binding to a capture domain of a capture probe. In some embodiments where a first probe includes a linker, a second probe includes a sequence that is about 10 nucleotides to about 100 nucleotides, or any of the subranges described herein. In some embodiments, a blocker oligonucleotide used to silence the second probe includes a sequence that is substantially complementary to a sequence (e.g., a sequence that is substantially complementary to the third target sequence of the analyte) of the second probe.

In some embodiments where a first probe includes a linker, the sequence of the second probe is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to the third target sequence in the analyte. In some embodiments, the sequence of the second probe that is substantially complementary to a third target sequence can include a sequence that is about 5 nucleotides to about 50 nucleotides, or any of the subranges described herein. In some embodiments, a blocker oligonucleotide used for silencing the second probe includes a sequence that is about 5 nucleotides to about 50 nucleotides, or any of the subranges described herein.

In some embodiments where a first probe includes a linker sequence, a first target sequence is not adjacent to a second target sequence. For example, the first target sequence and second target sequences are located on different exons of the same mRNA molecule. In another example, the first target sequence and the second target sequence are located on the same exon of the same mRNA molecule but are not adjacent. In some instances, the first probe and the second probe hybridize to sequences that at least about 1, 2, 3, 4, 5, 6, 7, 8, 9, 10, 11, 12, 13, 14, 15, 16, 17, 18, 19, 20, 21, 22, 23, 24, 25, or more nucleotides apart. In such cases, a single blocker oligonucleotide can be designed to silence both the first probe and the second probe. For example, the single blocker oligonucleotide can include at least about 1, 2, 3, 4, 5, 6, 7, 8, 9, 10, 11, 12, 13, 14, 15, 16, 17, 18, 19, 20, 21, 22, 23, 24, 25, or more nucleotides between two sequences that are substantially complementary to the first probe and the second probe, respectively. Alternatively, two or more blocker oligonucleotides can be designed to silence the first probe and the second probe.

In some embodiments where a first probe includes a linker sequence, a second target sequence is directly adjacent to a third target sequence. In such cases, either a single blocker oligonucleotide or multiple blocker nucleotides can be used to silence the first probe and the second probe.

In some embodiments where a first probe includes a linker sequence, the second probe includes ribonucleotides, deoxyribonucleotides, and/or synthetic nucleotides that are capable of participating in Watson-Crick type or analogous base pair interactions. In some embodiments, the second probe includes deoxyribonucleotides. In some embodiments, the second probe includes deoxyribonucleotides and ribonucleotides. In some embodiments where the second probe includes deoxyribonucleotides, hybridization of the second probe to the mRNA molecule results in a DNA:RNA hybrid. In some embodiments, the second probe includes only deoxyribonucleotides and upon hybridization of the second probe to the mRNA molecule results in a DNA:RNA hybrid.

### (v) Second Probe Having a Linker

Also provided herein are methods for identifying a location of an analyte in a biological sample where the method includes a first probe and a second probe that includes a linker sequence. Using a pair of probes where the second probe includes a linker sequence enables greater flexibility in designing templated ligation probes, primarily by increasing the sequences within the analyte that can be used as optional target sequences. In some embodiments, the second probe having a linker sequence can be silenced using one or more blocker oligonucleotides described herein.

A non-limiting example of a method for identifying a location of an analyte in a biological sample where the method includes a first probe and a second probe that includes a linker includes: (a) contacting the biological sample with a substrate including a plurality of capture probes, wherein a capture probe of the plurality of capture probes includes a capture domain and a spatial barcode; (b) contacting the biological sample with a first probe and a second probe, wherein a portion of the first probe and a portion of the second probe are substantially complementary to adjacent sequences of the analyte, wherein the first probe includes a sequence that is substantially complementary to a first target sequence of the analyte, wherein the second probe includes:(i) a first sequence that is substantially complementary to a second target sequence of the analyte; (ii) a linker sequence; (iii) a second sequence that is substantially complementary to a third target sequence of the analyte; and (iv) a capture probe binding domain that is capable of binding to a capture domain of a capture probe; (c) hybridizing the first probe and the second probe to the analyte; (d) ligating the first probe and the second probe, thereby creating a ligation product; (e) releasing the ligation product from the analyte; (f) hybridizing the capture probe binding domain to a capture domain; and (g) determining (i) all or a part of the sequence of the ligation product specifically bound to the capture domain, or a complement thereof, and (ii) all or a part of the sequence of the spatial barcode, or a complement thereof, and using the determined sequence of (i) and (ii) to identify the location of the analyte in the biological sample. Here, blocker oligonucleotides can be added to the sample concurrently with the first probe and the second probe, such that the blocker oligonucleotides can hybridize to probes targeted reduction or exclusion, thereby blocking their hybridization to the target analyte (e.g., an analyte targeted for reduction or exclusion from a spatial transcriptomics assay).

A non-limiting example of a method for identifying a location of an analyte in a biological sample where the method includes a first probe and a second probe that includes a linker can include the components as shown **FIG. 11****.** A first probe **1101** includes a functional sequence **1102** and a sequence **1103** that is substantially complementary to a first target sequence **1104** of the analyte. A second probe **1105** includes a first sequence **1106** that is substantially complementary to a second target sequence **1107** of the analyte, a linker sequence **1108;** and a second sequence **1109** that is substantially complementary to a third target sequence **1110** of the analyte, and a capture probe capture domain **1111** that is capable of binding to a capture domain of a capture probe.

In situations when the analyte is an analyte targeted for reduction or exclusion from a spatial transcriptomics assay, a first blocker oligonucleotide can be designed to hybridize with the sequence **1103** of the first probe **1101;** and/or a second blocker oligonucleotide can be designed to hybridize with the first sequence **1106** of the second probe **1105;** and/or a third blocker oligonucleotide can be designed to hybridize with the second sequence **1109** of the second probe **1105.** Alternatively, when the analyte is an analyte targeted for reduction or exclusion, a first blocker oligonucleotide can be designed to hybridize with the sequence **1103** of the first probe **1101;** and/or a second blocker oligonucleotide can be designed to hybridize with a sequence including the first sequence **1106,** optionally the linker sequence **1108,** and the second sequence **1109** of the second probe **1105.** Alternatively, when the analyte is an analyte targeted for reduction or exclusion, a first blocker oligonucleotide can be designed to hybridize with the sequence **1103** (of the first probe **1101)** and the first sequence **1106** (of the second probe **1105);** and a second blocker oligonucleotide can be designed to hybridize with the second sequence **1109** of the second probe **1105.** Alternatively, when the analyte is an analyte targeted for reduction or exclusion, a single blocker oligonucleotide can be designed to hybridize with the sequence **1103** (of the first probe **1101),** the first sequence **1106** (of the second probe **1105),** optionally the linker sequence **1108,** and the sequence **1109** (of the second probe **1105).** In all the cases above, the blocker oligonucleotide(s) can block the first probe **1101** and/or the second probe **1105** from hybridizing to the analyte targeted for reduction or exclusion. Without proper hybridization, negligible amounts of ligation product may be generated from the first probe **1101** and/or the second probe **1105.** As a result, the first probe **1101** and/or the second probe **1105** are silenced by the blocker oligonucleotide(s).

### 1) First Probe

In some embodiments where a second probe includes a linker sequence, a first probe includes a sequence that is substantially complementary to a first target sequence of the analyte.

In some embodiments where a second probe includes a linker sequence, a first probe includes a functional sequence. In some embodiments, a first probe includes a functional sequence and a sequence that is substantially complementary to a first target sequence of the analyte. In some embodiments, the functional sequence includes a primer sequence. In some embodiments, the first probe oligonucleotide includes from 5' to 3': a functional sequence, and a sequence that is substantially complementary to a first target sequence. In some embodiments, a blocker oligonucleotide used to silence the first probe includes a sequence that is substantially complementary to the functional sequence. In some embodiments, the blocker oligonucleotide does not include a sequence that is substantially complementary to the functional sequence.

In some embodiments where a linker is on a second probe, a first probe includes a sequence that is about 10 nucleotides to about 100 nucleotides, or any of the subranges described herein. In some embodiments, a blocker oligonucleotide used to silence the first probe includes a sequence that is substantially complementary to a sequence (e.g., a sequence that is substantially complementary to the first target sequence of the analyte) of the first probe.

In some embodiments where the second probe includes a linker sequence, a sequence of a first probe that is substantially complementary to a first target sequence of the analyte is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to the first target sequence in the analyte. In some embodiments, the sequence that is substantially complementary to a first target sequence can include a sequence that is about 5 nucleotides to about 50 nucleotides, or any of the subranges described herein. In some embodiments, a blocker oligonucleotide used for silencing the first probe includes a sequence that is about 5 nucleotides to about 50 nucleotides, or any of the subranges described herein.

In some embodiments where a second probe includes a linker sequence, a first probe includes at least two ribonucleic acid bases at the 3' end. In some embodiments, a first probe includes at least three, at least four, at least five, at least six, at least seven, at least eight, at least nine, or at least ten ribonucleic acid bases at the 3' end.

In some embodiments where a second probe includes a linker sequence, the first probe includes ribonucleotides, deoxyribonucleotides, and/or synthetic nucleotides that are capable of participating in Watson-Crick type or analogous base pair interactions. In some embodiments, the first probe includes deoxyribonucleotides. In some embodiments, the first probe includes deoxyribonucleotides and ribonucleotides. In some embodiments where the first probe includes deoxyribonucleotides, hybridization of the first probe to the mRNA molecule results in a DNA:RNA hybrid. In some embodiments, the first probe includes only deoxyribonucleotides and upon hybridization of the first probe to the mRNA molecule results in a DNA:RNA hybrid.

### 2) Second Probe

In some embodiments where a linker is on a second probe, the second probe includes (i) a first sequence that is substantially complementary to a second target sequence of the analyte; (ii) a linker sequence (e.g., any of the exemplary linker sequences described herein); (iii) a second sequence that is substantially complementary to third target sequence of the analyte; and (iv) a capture probe capture domain (e.g., any of the exemplary capture probe capture domains described herein) that is capable of binding to a capture domain of a capture probe. In some embodiments, a blocker oligonucleotide used to silence the second probe includes a sequence that is substantially complementary to the first sequence. In some embodiments, the blocker oligonucleotide does not include a sequence that is substantially complementary to the first sequence. In some embodiments, the blocker oligonucleotide includes a sequence that is substantially complementary to the linker sequence. In some embodiments, the blocker oligonucleotide does not include a sequence that is substantially complementary to the linker sequence. In some embodiments, the blocker oligonucleotide includes a sequence that is substantially complementary to the second sequence. In some embodiments, the blocker oligonucleotide does not include a sequence that is substantially complementary to the second sequence. In some embodiments, the blocker oligonucleotide includes a sequence that is substantially complementary to the capture probe capture domain. In some embodiments, a blocker oligonucleotide used to silence the second probe does not include a sequence that is substantially complementary to the capture probe capture domain.

In some embodiments where a second probe includes a linker sequence, the second probe includes a sequence that is about 10 nucleotides to about 300 nucleotides, or any of the subranges described herein.

In some embodiments where a second probe includes a linker sequence, the second probe includes a first sequence that is substantially complementary to a second target sequence of the analyte. In some embodiments, the first sequence of the second probe is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to the second target sequence in the analyte. In some embodiments, the first sequence of the second probe that is substantially complementary to a second target sequence can include a sequence that is about 5 nucleotides to about 50 nucleotides, or any of the subranges described herein.

In some embodiments where a second probe includes a linker sequence, the second probe includes a second sequence that is substantially complementary to a third target sequence of the analyte. In some embodiments, the second sequence of the second probe is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to the third target sequence in the analyte. In some embodiments, the second sequence of the second probe that is substantially complementary to a third target sequence can include a sequence that is about 5 nucleotides to about 50 nucleotides, or any of the subranges described herein. In some embodiments, a blocker oligonucleotide used for silencing the second probe includes a sequence that is about 5 nucleotides to about 50 nucleotides, or any of the subranges described herein.

In some embodiments where a second probe includes a linker sequence, a second target sequence is not adjacent to a third target sequence in the mRNA molecule. For example, the second target sequence and third target sequence are located on different exons of the same mRNA molecule. In another example, the second target sequence and the third target sequence are located on the same exon of the same mRNA molecule but are not adjacent. In such cases, a single blocker oligonucleotide can be designed to silence both the first probe and the second probe. For example, the single blocker oligonucleotide can include one or more nucleotides between two sequences that are substantially complementary to the first probe and the second probe, respectively. Alternatively, two or more blocker oligonucleotides can be designed to silence the first probe and the second probe.

In some embodiments where a second probe includes a linker sequence, a first target sequence is directly adjacent to a second target sequence. In such cases, either a single blocker oligonucleotide or multiple blocker nucleotides can be used to silence the first probe and the second probe.

In some embodiments, a second probe that includes a linker sequence includes ribonucleotides, deoxyribonucleotides, and/or synthetic nucleotides that are capable of participating in Watson-Crick type or analogous base pair interactions. In some embodiments, the second probe that includes a linker sequence includes deoxyribonucleotides. In some embodiments, the second probe that includes a linker sequence includes deoxyribonucleotides and ribonucleotides. In some embodiments where the second probe that includes a linker sequence includes deoxyribonucleotides, hybridization of the second probe to the mRNA molecule results in a DNA:RNA hybrid. In some embodiments, the second probe that includes a linker sequence includes only deoxyribonucleotides and upon hybridization of the second probe to the mRNA molecule results in a DNA:RNA hybrid.

### (vi) Probe Combination with Linkers on the First Probe and the Second Probe

Also provided herein are methods for identifying a location of an analyte in a biological sample where the method includes a first probe that includes a linker sequence and a second probe that includes a linker sequence. Using a pair of probes where the first probe and second probe each include a linker sequence enables greater flexibility in designing templated ligation probes, primarily by increasing the sequences within the analyte that can be used as optional target sequences. In some embodiments, the first probe and the second probe can each be silenced using one or more blocker oligonucleotides described herein. In some embodiments, the one or more blocker oligonucleotides include a sequence that is substantially complementary to the linker sequence. In some embodiments, the one or more blocker oligonucleotides do not include a sequence that is substantially complementary to the linker sequence.

### (f) Probe Combinations including a First Probe, a Second Probe and a Spanning Probe

Also provided herein are methods for identifying a location of an analyte in a biological sample where the method includes a first probe, a spanning probe, and a second probe. Using a spanning probe enables greater flexibility in designing templated ligation probes, primarily by increasing the sequences within the analyte that can be used as optional target sequences. In some cases, using a spanning probe can also be used to interrogate the variants (e.g., splice variants) that span greater distances that can be interrogated using a first or second probe with a linker sequence. In some embodiments, the first probe, the spanning probe, and the second probe can be silenced by blocker oligonucleotides. For example, each of these probes can be hybridized with a blocker oligonucleotide.

A non-limiting example of a method for identifying a location of an analyte in a biological sample where the method includes a first probe, a spanning probe, and a second probe, includes: (a) contacting the biological sample with a substrate including a plurality of capture probes, wherein a capture probe of the plurality of capture probes includes a capture domain and a spatial barcode; (b) contacting the biological sample with a first probe, a second probe, and one or more spanning probes, wherein the first probe is substantially complementary to a first portion of the analyte, wherein the second probe is substantially complementary to a second portion of the analyte and further includes a capture probe binding domain, and wherein the spanning probe includes: (i) a first sequence that is substantially complementary to a first target sequence of the analyte, and (ii) a second sequence that is substantially complementary to a second target sequence of the analyte; (c) hybridizing the first probe, the second probe, and the spanning probe to the analyte; (d) ligating the first probe, the one or more spanning probes, and the second probe, thereby creating a ligation product that is substantially complementary to the analyte; (e) releasing the ligation product from the analyte; (f) hybridizing the capture probe binding domain to a capture domain; and (g) determining (i) all or a part of the sequence of the ligation product specifically bound to the capture domain, or a complement thereof, and (ii) all or a part of the sequence of the spatial barcode, or a complement thereof, and using the determined sequence of (i) and (ii) to identify the location of the analyte in the biological sample. Here, blocker oligonucleotides can be added to the sample concurrently with the first probe, the second probe, and the one or more spanning probes, such that the blocker oligonucleotides can hybridize to probes targeted for reduction or exclusion, thereby blocking their hybridization to the target analyte (e.g., an analyte targeted for reduction or exclusion from a spatial transcriptomics assay).

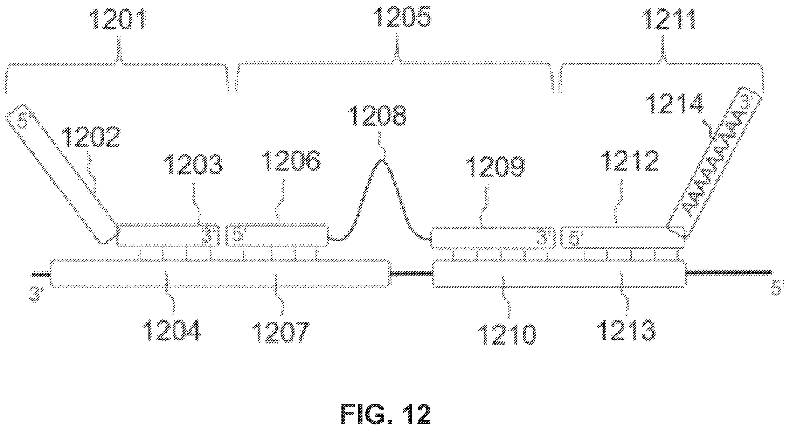

A non-limiting example of a method for identifying a location of an analyte in a biological sample where the method includes a first probe, a second probe, and a spanning probe can include the components as shown FIG. 12. A first probe **1201** includes a functional sequence **1202,** a sequence **1203** that is substantially complementary to a first portion **1204 of** the analyte. A spanning probe **1205** includes a first sequence **1206** that is substantially complementary to a first target sequence **1207** of the analyte, a linker sequence **1208,** and a second sequence **1209** that is substantially complementary to a second target sequence **1210** of the analyte. A second probe **1211** includes a sequence **1212** that is substantially complementary to a second portion **1213** of the analyte and a capture probe capture domain **1214.** In situations when the analyte is an analyte targeted reduction or exclusion, a first blocker oligonucleotide can be designed to hybridize with the sequence **1203** of the first probe **1201;** and/or a second blocker oligonucleotide can be designed to hybridize with the first sequence **1206** of the spanning probe **1205;** and/or a third blocker oligonucleotide can be designed to hybridize with the second sequence **1209** of the spanning probe **1205;** and/or a fourth oligonucleotide can be designed to hybridize with the sequence **1212** of the second probe **1211.** Alternatively, when the analyte is an analyte targeted reduction or exclusion, a first blocker oligonucleotide can be designed to hybridize with the sequence **1203** of the first probe **1201;** and/or a second blocker oligonucleotide can be designed to hybridize with a sequence including the first sequence **1206,** optionally the linker sequence **1208,** and the second sequence **1209** of the spanning probe **1205;** and/or a third blocker oligonucleotide can be designed to hybridize with the sequence **1212** of the second probe **1211.** Alternatively, when the analyte is an analyte targeted reduction or exclusion, a first blocker oligonucleotide is designed to hybridize with the sequence **1203** (of the first probe **1201)** and the first sequence **1206** (of the spanning probe **1205);** and/or a second blocker oligonucleotide is designed to hybridize with the second sequence **1209** (of the spanning probe **1205)** and the sequence **1212** of the second probe **1211.** In all the cases above, the blocker oligonucleotide(s) can block the first probe **1201,** and/or the spanning probe **1205,** and/or the second probe **1211** from hybridizing to the analyte targeted reduction or exclusion. Without proper hybridization, negligible amounts of ligation product may be generated from these probes. As a result, the first probe **1201,** the spanning probe **1205,** and the second probe **1211** are silenced by the blocker oligonucleotide(s).

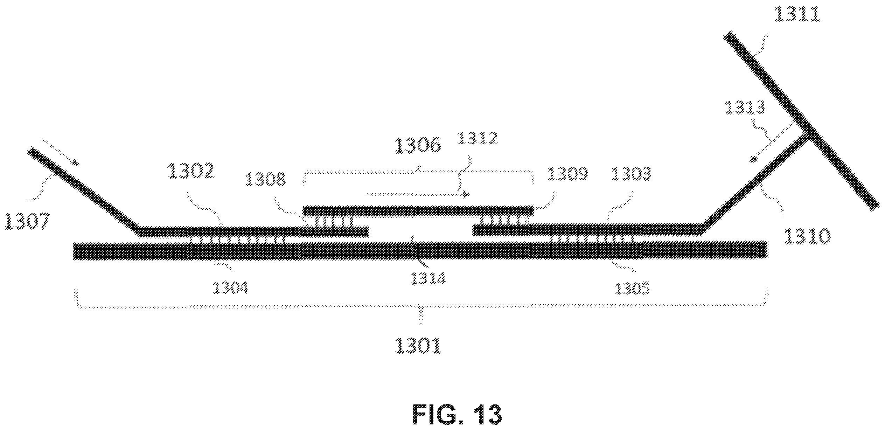

Another non-limiting example of this method is shown in **FIG. 13****.** A biological sample including an analyte **1301** is contacted with a first probe **1302** and a second probe **1303.** The first probe **1302** and the second probe **1303** hybridize to the analyte at a first target sequence **1304** and a second target sequence **1305,** respectively. The first probe and the second probe include free ends **1307-1310.** As shown in **FIG. 13****,** the first and second target sequences are not directly adjacent in the analyte. After hybridization, unbound first and second probes are washed away. Then, a third oligonucleotide **1306** hybridizes to the first and the second probes at **1308** and **1309,** respectively. After hybridization, the first probe is extended **1312** and a ligation product is created that includes the first probe sequence and the second probe sequence. Alternatively, instead of extending the first probe, the third oligonucleotide is used to "bind" the first probe and the second probe together. In such cases, the first probe and the second probe bound together by the third oligonucleotide can be referred to as a ligation product. The ligation product then is contacted with a substrate **1311,** and the ligation product is bound to a capture probe **1313** of the substrate **1311** on the array at distinct spatial positions. In some embodiments, the biological sample is contacted with the substrate **1311** prior to being contacted with the first probe and the second probe.

### (i) First Probe

In some embodiments where the method includes a spanning probe, a first probe includes a sequence that is substantially complementary to a first portion of the analyte. In some embodiments, a sequence of the first probe is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to a first portion of the analyte. In some embodiments, the sequence of the first probe that is substantially complementary to a first portion of the analyte can include a sequence that is about 5 nucleotides to about 50 nucleotides, or any of the subranges described herein. In some embodiments, a blocker oligonucleotide used for silencing the first probe includes a sequence that is about 5 nucleotides to about 50 nucleotides, or any of the subranges described herein.

In some embodiments, the first probe includes a functional sequence. In some embodiments, the functional sequence is a primer sequence. In some embodiments, a blocker oligonucleotide used to silence the first probe includes a sequence that is substantially complementary to the functional sequence. In some embodiments, the blocker oligonucleotide does not include a sequence that is substantially complementary to the functional sequence.

In some embodiments, a blocker oligonucleotide used to silence the first probe includes a sequence that is substantially complementary to a sequence (e.g., a sequence that is substantially complementary to the first portion of the analyte) of the first probe.

In some embodiments where the method includes a spanning probe, a first probe includes at least two ribonucleic acid bases at the 3' end. In some embodiments, a first probe includes at least three, at least four, at least five, at least six, at least seven, at least eight, at least nine, or at least ten ribonucleic acid bases at the 3' end.

In some embodiments where the method includes a spanning probe, a first probe includes from 5' to 3': a functional sequence, a sequence that is substantially complementary to a first portion of the analyte, and two or more ribonucleic acid bases.

In some embodiments where the method includes a spanning probe, a first probe includes ribonucleotides, deoxyribonucleotides, and/or synthetic nucleotides that are capable of participating in Watson-Crick type or analogous base pair interactions. In some embodiments, the first probe includes deoxyribonucleotides. In some embodiments, the first probe includes deoxyribonucleotides and ribonucleotides. In some embodiments where the first probe includes deoxyribonucleotides, hybridization of the first probe to the mRNA molecule results in a DNA:RNA hybrid. In some embodiments, the first probe includes only deoxyribonucleotides and upon hybridization of the first probe to the mRNA molecule results in a DNA:RNA hybrid.

### (ii) Spanning Probe

In some embodiments where the method includes a spanning probe, the spanning probe includes a first sequence that is substantially complementary to a first target sequence of the analyte, and a second sequence that is substantially complementary to a second target sequence of the analyte. In some embodiments, the spanning probe includes a first sequence that is substantially complementary to a first target sequence of the analyte, a functional sequence, and a second sequence that is substantially complementary to a second target sequence of the analyte. In some embodiments, a spanning probe includes from 5' to 3': a first sequence, a functional sequence, and a second sequence. In some embodiments, the functional sequence is a linker sequence. The linker sequence can include a total of about 10 nucleotides to about 100 nucleotides, or any of the subranges described herein. In some embodiments, a blocker oligonucleotide used to silence the spanning probe includes a sequence that is substantially complementary to the first sequence. In some embodiments, the blocker oligonucleotide does not include a sequence that is substantially complementary to the first sequence. In some embodiments, a blocker oligonucleotide used to silence the spanning probe includes a sequence that is substantially complementary to the functional sequence. In some embodiments, the blocker oligonucleotide does not include a sequence that is substantially complementary to the functional sequence. In some embodiments, the blocker oligonucleotide includes a sequence that is substantially complementary to the second sequence. In some embodiments, the blocker oligonucleotide does not include a sequence that is substantially complementary to the second sequence.

In some embodiments, the functional sequence includes a barcode sequence. In some embodiments, the spanning probe can include a linker and a barcode sequence. In such cases, linker sequences can flank the barcode, the barcode can be 5' to a linker sequence, or the barcode can be 3' to a linker sequence. In some embodiments, a barcode sequence is flanked by a 5' linker sequence (e.g., any of the exemplary linker sequences described herein) and a 3' linker sequence (e.g., any of the exemplary linker sequences described herein). In some embodiments where a functional sequence includes a barcode sequence, a blocker oligonucleotide used for silencing the spanning probe includes a sequence that is substantially complementary to the barcode sequence. In some embodiments where a functional sequence includes a barcode sequence, a blocker oligonucleotide used for silencing the spanning probe does not include a sequence that is substantially complementary to the barcode sequence.

In some embodiments, the spanning probe includes from 5' to 3': a first sequence, a 5' linker sequence, a barcode, a 3' linker sequence, and a second sequence.

In some embodiments, the spanning probes includes a sequence that is about 10 nucleotides to about 300 nucleotides or any of the subranges described herein. In some embodiments, a blocker oligonucleotide used for silencing the spanning probe includes a sequence that is about 10 nucleotides to about 300 nucleotides, or any of the subranges described herein.

In some embodiments, a first sequence of the spanning probe is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to the first target sequence of the analyte. In some embodiments, a second sequence of the spanning probe is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to the second target sequence of the analyte.

In some embodiments, the first sequence of the spanning probe and the second sequence of the spanning probe are substantially complementary to sequences within the same exon.

In some embodiments, the first target sequence of the analyte and the second target of the analyte are located within the same exon. In such cases, the first target sequence and the second target sequence are not directly adjacent.

In some embodiments, the first sequence of the spanning probe and the second sequence of the spanning probe are substantially complementary to sequences within the different exons of the same gene. In some embodiments, the first target sequence of the analyte and the second target sequence of the analyte are located on different exons of the same gene.

In some embodiments, the spanning probe includes ribonucleotides, deoxyribonucleotides, and/or synthetic nucleotides that are capable of participating in Watson-Crick type or analogous base pair interactions. In some embodiments, the spanning probe includes deoxyribonucleotides. In some embodiments, the spanning probe includes deoxyribonucleotides and ribonucleotides. In some embodiments where the spanning probe includes deoxyribonucleotides, hybridization of the spanning probe to the mRNA molecule results in a DNA:RNA hybrid. In some embodiments, the spanning probe includes only deoxyribonucleotides and upon hybridization of the spanning probe to the mRNA molecule results in a DNA:RNA hybrid.

### (iii) Second probe

In some embodiments where the method includes a spanning probe, a second probe includes a sequence that is substantially complementary to a second portion of the analyte and a capture probe capture domain (e.g., any of the exemplary capture probe capture domains described herein). In some embodiments, a blocker oligonucleotide used to silence the second probe includes a sequence that is substantially complementary to the capture probe capture domain. In some embodiments, the blocker oligonucleotide does not include a sequence that is substantially complementary to the capture probe capture domain.

In some embodiments, a blocker oligonucleotide used to silence the second probe includes a sequence that is substantially complementary to a sequence (e.g., a sequence that is substantially complementary to the second portion of the analyte) of the second probe.

In some embodiments where the method includes a spanning probe, a sequence of the second probe is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to a second portion of the analyte. In some embodiments, the sequence of the second probe that is substantially complementary to a second portion of the analyte can include a sequence that is about 5 nucleotides to about 50 nucleotides, or any of the subranges described herein. In some embodiments, a blocker oligonucleotide used for silencing the second probe includes a sequence that is about 5 nucleotides to about 50 nucleotides, or any of the subranges described herein.

In some embodiments where the method includes a spanning probe, the first portion of the analyte is directly adjacent to the first target sequence, and/or the second portion of the analyte is directly adjacent to the second target sequence. In such cases, the sequence of the first probe is ligated to the first sequence of the spanning probe, and the sequence of the second probe is ligated to the second sequence of the spanning probe. In some embodiments, the spanning probe includes at least two ribonucleic acid bases at the 3' end, the first probe includes at least two ribonucleic acids at the 3' end, or both. In some embodiments, the spanning probe includes a phosphorylated nucleotide at the 5' end, the second probe includes a phosphorylated nucleotide at the 5' end, or both. In some embodiments where the first portion of the analyte is directly adjacent to the first target sequence, a single blocker oligonucleotide can be designed to silence both the first probe and the spanning probe. Alternatively, two or more blocker oligonucleotides can be designed to silence the first probe and the spanning probe. In some embodiments where the second portion of the analyte is directly adjacent to the second target sequence, a single blocker oligonucleotide can be designed to silence both the spanning probe and the second probe. Alternatively, two or more blocker oligonucleotides can be designed to silence the spanning probe and the second probe.

In some embodiments where the method includes a spanning probe, a second probe includes ribonucleotides, deoxyribonucleotides, and/or synthetic nucleotides that are capable of participating in Watson-Crick type or analogous base pair interactions. In some embodiments, the second probe includes deoxyribonucleotides. In some embodiments, the second probe includes deoxyribonucleotides and ribonucleotides. In some embodiments where the second probe includes deoxyribonucleotides, hybridization of the second probe to the mRNA molecule results in a DNA:RNA hybrid. In some embodiments, the second probe includes only deoxyribonucleotides and upon hybridization of the second probe to the mRNA molecule results in a DNA:RNA hybrid.

### (iv) Probe Combinations including a First Probe, a Second Probe, and Multiple Spanning Probes

Also provided herein are methods for identifying a location of an analyte in a biological sample where the method includes a first probe, at least two spanning probes, and a second probe. Using two or more spanning probes enables greater flexibility in designing templated ligation probes, primarily by increasing the sequences within the analyte that can be used as optional target sequences. In some cases, using two or more spanning probe can also be used to interrogate the variants (e.g., splice variants) that span greater distances that can be interrogated using one spanning probe. In some embodiments, the first probe, the at least two spanning probes, and the second probe can be silenced by at least 1, at least 2, at least 3, at least 4, at least 5, at least 6, at least 7, at least 8, at least 9, at least 10, or more blocker oligonucleotides. For example, each of these probes can be hybridized with a blocker oligonucleotide.

A non-limiting example of a method for identifying a location of an analyte in a biological sample where the method includes a first probe, two or more spanning probes, and a second probe, includes: (a) contacting the biological sample with a substrate including a plurality of capture probes, wherein a capture probe of the plurality of capture probes includes a capture domain and a spatial barcode; (b) contacting the biological sample with a first probe, a second probe, and two spanning probes, wherein the first probe is substantially complementary to a first portion of the analyte, wherein the second probe is substantially complementary to a second portion of the analyte and further includes a capture probe binding domain, and wherein the first spanning probe includes: (i) a first sequence that is substantially complementary to a first target sequence of the analyte, and (ii) a second sequence that is substantially complementary to a second target sequence of the analyte; and the second spanning probe includes (i) a third sequence that is substantially complementary to a third target sequence of the analyte, and (ii) a fourth sequence that is substantially complementary to a fourth target sequence of the analyte; (c) hybridizing the first probe, the second probe, and the spanning probes to the analyte; (d) ligating the first probe, the one or more spanning probes, and the second probe, thereby creating a ligation product that is substantially complementary to the analyte; (e) releasing the ligation product from the analyte; (f) hybridizing the capture probe binding domain to a capture domain; and (g) determining (i) all or a part of the sequence of the ligation product specifically bound to the capture domain, or a complement thereof, and (ii) all or a part of the sequence of the spatial barcode, or a complement thereof, and using the determined sequence of (i) and (ii) to identify the location of the analyte in the biological sample. Here, blocker oligonucleotides can be added to the sample concurrently with the first probe, the two spanning probes, and the second probe, such that the blocker oligonucleotides can hybridize to probes targeted reduction or exclusion, thereby blocking their hybridization to the target analyte (e.g., an analyte targeted for reduction or exclusion from a spatial transcriptomics assay).

In some embodiments, the methods that include one or more spanning probes include at least two, at least three, at least four, at least five, or more spanning probes. In such cases, the one or more spanning probes includes (i) a third sequence that is substantially complementary to a third target sequence of the analyte, and (ii) a fourth sequence that is substantially complementary to a fourth target sequence of the analyte. In some instances, all or a part of each spanning probe can be hybridized by one or more blocker oligonucleotides.

In some embodiments where the method includes two (or more) spanning probes, the first target sequence is located in a first exon, the second target sequence is located in a second exon, and the third target sequence and the fourth target sequence are located in a third exon. In some embodiments where the method includes two (or more) spanning probes, the first target sequence is located in a first exon, the second target sequence is located in a second exon, and the third target sequence is located in a third exon, and the fourth target sequence is located in a fourth exon. In some embodiments where the method includes two (or more) spanning probes, the first target sequence and the second target sequences are located in a first exon, and the third target sequence and the fourth target sequence are located in a second exon. In some embodiments where the method includes two (or more) spanning probes, the first target sequence and the second target sequences are located in a first exon, and the third target sequence is located in a second exon, and the fourth target sequence is located in a third exon.

In some embodiments where the first portion of the analyte is directly adjacent to the first target sequence, a single blocker oligonucleotide can be designed to silence both the first probe and the first spanning probe. Alternatively, two or more blocker oligonucleotides can be designed to silence the first probe and the first spanning probe. In some embodiments where the second target sequence of the analyte is directly adjacent to the third target sequence of the analyte, a single blocker oligonucleotide can be designed to silence both the first and the second spanning probes. Alternatively, two or more blocker oligonucleotides can be designed to silence the first and the second spanning probes. In some embodiments where the fourth target sequence of the analyte is directly adjacent to the second portion of the analyte, a single blocker oligonucleotide can be designed to silence both the second spanning probe and the second probe. Alternatively, two or more blocker oligonucleotides can be designed to silence the second spanning probe and the second probe. While not intending to be bound by any theory, it is believed that blocker oligonucleotides can be designed as described herein to silence a probe set including a first probe, two or more spanning probes, and a second probe.

In some embodiments, where the methods include two (or more) spanning probes, the method includes ligating: the first probe to the spanning probe, the spanning probe to the one or more additional spanning probes, and the one or more additional spanning probes spanning oligonucleotide to the second probe, thereby creating a ligation product that includes one or more sequences that are substantially complementary to the analyte. In some embodiments, where the methods include two (or more) spanning probes, the method includes ligating: the first probe to the one or more additional spanning probes, the one or more additional spanning probes to the spanning probe, and the spanning probe to the second probe, thereby creating a ligation product that includes one or more sequences that are substantially complementary to the analyte.

In some embodiments, each additional spanning probe can include a functional sequence (e.g., any of the functional sequence described herein). For example, each additional spanning probe can include a linker sequence (e.g., any of the exemplary linker sequences described herein). In another example, each additional spanning probe can include a barcode sequence (e.g., any of the exemplary barcode sequences described herein) and a linker sequence (e.g., any of the linker sequences described herein). In some embodiments where an additional spanning probe includes a barcode and a linker, a linker sequences can flank the barcode, the barcode can be 5' to a linker sequence, or the barcode can be 3' to a linker sequence. In some embodiments, a barcode sequence is flanked by a 5' linker sequence (e.g., any of the exemplary linker sequences described herein) and a 3' linker sequence (e.g., any of the exemplary linker sequences described herein). In some embodiments, an additional spanning probe can include from 5' to 3': a first sequence, a 5' linker sequence, a barcode, a 3' linker sequence, and a second sequence. In some embodiments, the functional sequence, linker sequence, and/or barcode sequence of each additional spanning probe can be hybridized in order to silence the additional spanning probe.

### (g) Pre-Hybridization Methods

### (i) Imaging and Staining

Prior to addition of the probes, in some instances, biological samples can be stained using a wide variety of stains and staining techniques. In some instances, the biological sample is a section on a slide (e.g., a 10 µm section). In some instances, the biological sample is dried after placement onto a glass slide. In some instances, the biological sample is dried at 42°C. In some instances, drying occurs for about 1 hour, about 2, hours, about 3 hours, or until the sections become transparent. In some instances, the biological sample can be dried overnight (e.g., in a desiccator at room temperature).

In some embodiments, a sample can be stained using any number of biological stains, including but not limited to, acridine orange, Bismarck brown, carmine, coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, or safranin. In some instances, the methods disclosed herein include imaging the biological sample. In some instances, imaging the sample occurs prior to deaminating the biological sample. In some instances, the sample can be stained using known staining techniques, including Can-Grunwald, Giemsa, hematoxylin and eosin (H&E), Jenner's, Leishman, Masson's trichrome, Papanicolaou, Romanowsky, silver, Sudan, Wright's, and/or Periodic Acid Schiff (PAS) staining techniques. PAS staining is typically performed after formalin or acetone fixation. In some instances, the stain is an H&E stain.

In some embodiments, the biological sample can be stained using a detectable label (e.g., radioisotopes, fluorophores, chemiluminescent compounds, bioluminescent compounds, and dyes) as described elsewhere herein. In some embodiments, a biological sample is stained using only one type of stain or one technique. In some embodiments, staining includes biological staining techniques such as H&E staining. In some embodiments, staining includes identifying analytes using fluorescently-conjugated antibodies. In some embodiments, a biological sample is stained using two or more different types of stains, or two or more different staining techniques. For example, a biological sample can be prepared by staining and imaging using one technique (e.g., H&E staining and brightfield imaging), followed by staining and imaging using another technique (e.g., IHC/IF staining and fluorescence microscopy) for the same biological sample.

In some embodiments, biological samples can be destained. Methods of destaining or discoloring a biological sample are known in the art, and generally depend on the nature of the stain(s) applied to the sample. For example, H&E staining can be destained by washing the sample in HCl, or any other acid (e.g., selenic acid, sulfuric acid, hydroiodic acid, benzoic acid, carbonic acid, malic acid, phosphoric acid, oxalic acid, succinic acid, salicylic acid, tartaric acid, sulfurous acid, trichloroacetic acid, hydrobromic acid, hydrochloric acid, nitric acid, orthophosphoric acid, arsenic acid, selenous acid, chromic acid, citric acid, hydrofluoric acid, nitrous acid, isocyanic acid, formic acid, hydrogen selenide, molybdic acid, lactic acid, acetic acid, carbonic acid, hydrogen sulfide, or combinations thereof). In some embodiments, destaining can include 1, 2, 3, 4, 5, or more washes in an acid (e.g., HCl). In some embodiments, destaining can include adding HCl to a downstream solution (e.g., permeabilization solution). In some embodiments, destaining can include dissolving an enzyme used in the disclosed methods (e.g., pepsin) in an acid (e.g., HCl) solution. In some embodiments, after destaining hematoxylin with an acid, other reagents can be added to the destaining solution to raise the pH for use in other applications. For example, SDS can be added to an acid destaining solution in order to raise the pH as compared to the acid destaining solution alone. As another example, in some embodiments, one or more immunofluorescence stains are applied to the sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., J. Histochem. Cytochem. 2017; 65(8): 431-444, Lin et al., Nat Commun. 2015; 6:8390, Pirici et al., J. Histochem. Cytochem. 2009; 57:567-75, and Glass et al., J. Histochem. Cytochem. 2009; 57:899-905.

In some embodiments, immunofluorescence or immunohistochemistry protocols (direct and indirect staining techniques) can be performed as a part of, or in addition to, the exemplary spatial workflows presented herein. For example, tissue sections can be fixed according to methods described herein. The biological sample can be transferred to an array (e.g., capture probe array), wherein analytes (e.g., proteins) are probed using immunofluorescence protocols. For example, the sample can be rehydrated, blocked, and permeabilized (3X SSC, 2% BSA, 0.1% Triton X, 1 U/µl RNAse inhibitor for 10 minutes at 4°C) before being stained with fluorescent primary antibodies (1:100 in 3XSSC, 2% BSA, 0.1% Triton X, 1 U/µl RNAse inhibitor for 30 minutes at 4°C). The biological sample can be washed, coverslipped (in glycerol + 1 U/µl RNAse inhibitor), imaged (e.g., using a confocal microscope or other apparatus capable of fluorescent detection), washed, and processed according to analyte capture or spatial workflows described herein.

In some instances, a glycerol solution and a cover slip can be added to the sample. In some instances, the glycerol solution can include a counterstain (e.g., DAPI).

As used herein, an antigen retrieval buffer can improve antibody capture in IF/IHC protocols. An exemplary protocol for antigen retrieval can be preheating the antigen retrieval buffer (e.g., to 95°C), immersing the biological sample in the heated antigen retrieval buffer for a predetermined time, and then removing the biological sample from the antigen retrieval buffer and washing the biological sample.

In some embodiments, optimizing permeabilization can be useful for identifying intracellular analytes. Permeabilization optimization can include selection of permeabilization agents, concentration of permeabilization agents, and permeabilization duration. Tissue permeabilization is discussed elsewhere herein.

In some embodiments, blocking an array and/or a biological sample in preparation of labeling the biological sample decreases nonspecific binding of the antibodies to the array and/or biological sample (decreases background). Some embodiments provide for blocking buffers/blocking solutions that can be applied before and/or during application of the label, wherein the blocking buffer can include a blocking agent, and optionally a surfactant and/or a salt solution. In some embodiments, a blocking agent can be bovine serum albumin (BSA), serum, gelatin (e.g., fish gelatin), milk (e.g., non-fat dry milk), casein, polyethylene glycol (PEG), polyvinyl alcohol (PVA), or polyvinylpyrrolidone (PVP), biotin blocking reagent, a peroxidase blocking reagent, levamisole, Carnoy's solution, glycine, lysine, sodium borohydride, pontamine sky blue, Sudan Black, trypan blue, FITC blocking agent, and/or acetic acid. The blocking buffer/blocking solution can be applied to the array and/or biological sample prior to and/or during labeling (e.g., application of fluorophore-conjugated antibodies) to the biological sample.

### (ii) Preparation of Sample for Application of Probes

In some instances, the biological sample is deparaffinized. Deparaffinization can be achieved using any method known in the art. For example, in some instances, the biological samples is treated with a series of washes that include xylene and various concentrations of ethanol. In some instances, methods of deparaffinization include treatment of xylene (e.g., three washes at 5 minutes each). In some instances, the methods further include treatment with ethanol (e.g., 100% ethanol, two washes 10 minutes each; 95% ethanol, two washes 10 minutes each; 70% ethanol, two washes 10 minutes each; 50% ethanol, two washes 10 minutes each). In some instances, after ethanol washes, the biological sample can be washed with deionized water (e.g., two washes for 5 minutes each). It is appreciated that one skilled in the art can adjust these methods to optimize deparaffinization.

In some instances, the biological sample is decrosslinked. In some instances, the biological sample is decrosslinked in a solution containing TE buffer (comprising Tris and EDTA). In some instances, the TE buffer is basic (e.g., at a pH of about 9). In some instances, decrosslinking occurs at about 50°C to about 80°C. In some instances, decrosslinking occurs at about 70°C. In some instances, decrosslinking occurs for about 1 hour at 70°C. Just prior to decrosslinking, the biological sample can be treated with an acid (e.g., 0.1M HCl for about 1 minute). After the decrosslinking step, the biological sample can be washed (e.g., with 1x PBST).

In some instances, the methods of preparing a biological sample for probe application include permeabilizing the sample. In some instances, the biological sample is permeabilized using a phosphate buffer. In some instances, the phosphate buffer is PBS (e.g., 1x PBS). In some instances, the phosphate buffer is PBST (e.g., 1x PBST). In some instances, the permeabilization step is performed multiple times (e.g., 3 times at 5 minutes each).

In some instances, the methods of preparing a biological sample for probe application include steps of equilibrating and blocking the biological sample. In some instances, equilibrating is performed using a pre-hybridization (pre-Hyb) buffer. In some instances, the pre-Hyb buffer is RNase-free. In some instances, the pre-Hyb buffer contains no bovine serum albumin (BSA), solutions like Denhardt's, or other potentially nuclease-contaminated biological materials.

In some instances, the equilibrating step is performed multiple times (e.g., 2 times at 5 minutes each; 3 times at 5 minutes each). In some instances, the biological sample is blocked with a blocking buffer. In some instances, the blocking buffer includes a carrier such as tRNA, for example yeast tRNA such as from brewer's yeast (e.g., at a final concentration of 10-20 µg/mL). In some instances, blocking can be performed for 5, 10, 15, 20, 25, or 30 minutes.

Any of the foregoing steps can be optimized for performance. For example, one can vary the temperature. In some instances, the pre-hybridization methods are performed at room temperature. In some instances, the pre-hybridization methods are performed at 4°C (in some instances, varying the timeframes provided herein).

### (h) Hybridizing the Probes

In some embodiments, the methods provided herein include hybridizing a first probe oligonucleotide and a second probe oligonucleotide (e.g., a probe pair). In some instances, the first and second probe oligonucleotides each include sequences that are substantially complementary to one or more sequences (e.g., one or more target sequences) of an analyte of interest. In some embodiments, the first probe and the second probe bind to complementary sequences that are completely adjacent (i.e., no gap of nucleotides) to one another or are on the same transcript.

In some instances, the methods include hybridization of probe sets, wherein the probe pairs are in a medium at a concentration of about 1 to about 100 nM. In some instances, the concentration of the probe pairs is about 1, 2, 3, 4, 5, 6, 7, 8, 9, 10, 15, 20, 25, 30, 35, 40, 45, 50, 60, 70, 80, 90, 100, 200, 300, 400, or 500 nM. In some instances, the concentration of the probe pairs is 5 nM. In some instances, the probe sets are diluted in a hybridization (Hyb) buffer. In some instances, the probe sets are at a concentration of 5 nM in Hyb buffer.

In some instances, probe hybridization occurs at about 50°C. In some instances, the temperature of probe hybridization ranges from about 30°C to about 75°C, from about 35°C to about 70°C, or from about 40°C to about 65°C. In some embodiments, the temperature is about 30°C, about 31°C, about 32°C, about 33°C, about 34°C, about 35°C, about 36°C, about 37°C, about 38°C, about 39°C, about 40°C, about 41°C, about 42°C, about 43°C, about 44°C, about 45°C, about 46°C, about 47°C, about 48°C, about 49°C, about 50°C, about 51°C, about 52°C, about 53°C, about 54°C, about 55°C, about 56°C, about 57°C, about 58°C, about 59°C, about 60°C, about 61°C, about 62°C, about 63°C, about 64°C, about 65°C, about 66°C, about 67°C, about 68°C, about 69°C, or about 70°C. In some instances, probe hybridization occurs for about 30 minutes, about 1 hour, about 2 hours, about 2.5 hours, about 3 hours, or more. In some instances, probe hybridization occurs for about 2.5 hours at 50°C.

In some instances, the hybridization buffer includes SSC (e.g., 1x SSC) or SSPE. In some instances, the hybridization buffer includes formamide or ethylene carbonate. In some instances, the hybridization buffer includes one or more salts, like Mg salt for example MgCl₂, Na salt for example NaCl, Mn salt for example MnCl₂. In some instances, the hybridization buffer includes Denhardt's solution, dextran sulfate, ficoll, PEG or other hybridization rate accelerators. In some instances, the hybridization buffer includes a carrier such as yeast tRNA, salmon sperm DNA, and/or lambda phage DNA. In some instances, the hybridization buffer includes one or more blockers. In some instances, the hybridization buffer includes RNase inhibitor(s). In some instances, the hybridization buffer can include BSA, sequence specific blockers, non-specific blockers, EDTA, RNase inhibitor(s), betaine, TMAC, or DMSO. In some instances, a hybridization buffer can further include detergents such as Tween, Triton-X 100, sarkosyl, and SDS. In some instances, the hybridization buffer includes nuclease-free water, DEPC water.

In some embodiments, the complementary sequences to which the first probe oligonucleotide and the second probe oligonucleotide bind are 1, 2, 3, 4, 5, 6, 7, 8, 9, 10, about 15, about 20, about 25, about 30, about 35, about 40, about 45, about 50, about 55, about 60, about 65, about 70, about 75, about 80, about 85, about 90, about 95, about 100, about 125, about 150, about 175, about 200, about 250, about 300, about 350, about 400, about 450, about 500, about 600, about 700, about 800, about 900, or about 1000 nucleotides away from each other. Gaps between the probe oligonucleotides may first be filled prior to ligation, using, for example, Mu polymerase, DNA polymerase, RNA polymerase, reverse transcriptase, VENT polymerase, Taq polymerase, and/or any combinations, derivatives, and variants (e.g., engineered mutants) thereof. In some embodiments, when the first and second probe oligonucleotides are separated from each other by one or more nucleotides, nucleotides are ligated between the first and second probe oligonucleotides. In some embodiments, when the first and second probe oligonucleotides are separated from each other by one or more nucleotides, deoxyribonucleotides are ligated between the first and second probe oligonucleotides.

In some instances, after hybridization, the biological sample is washed with a post-hybridization wash buffer. In some instances, the post-hybridization wash buffer includes one or more of SSC, yeast tRNA, formamide, ethylene carbonate, and nuclease-free water.

### (i) Hybridizing Temperatures

In some embodiments, the method described utilizes oligonucleotides that include deoxyribonucleic acids (instead of strictly utilizing ribonucleotides) at the site of ligation. Utilizing deoxyribonucleic acids in the methods described herein create a more uniform efficiency that can be readily-controlled and flexible for various applications.

In a non-limiting example, the methods disclosed herein include contacting a biological sample with a plurality of oligonucleotides (e.g., probes) including a first oligonucleotide (e.g., a first probe) and a second oligonucleotide (e.g., a second probe), wherein the first oligonucleotide (e.g., the first probe) and the second oligonucleotide (e.g., the second probe) are complementary to a first sequence present in an analyte and a second sequence present in the analyte, respectively; hybridizing the first oligonucleotide (e.g., the first probe) and the second oligonucleotide (e.g., the second probe) to the analyte at a first temperature; hybridizing the first oligonucleotide (e.g., the first probe) and the second oligonucleotide (e.g., the second probe) to a third oligonucleotide (e.g., a splint oligonucleotide) at a second temperature such that the first oligonucleotide (e.g., the first probe) and the second oligonucleotide (e.g., the second probe) abut each other; ligating the first oligonucleotide (e.g., the first probe) to the second oligonucleotide (e.g., the second probe) to create a ligation product; contacting the biological sample with a substrate, wherein a capture probe is immobilized on the substrate, wherein the capture probe includes a spatial barcode and a capture domain; allowing the ligation product to specifically bind to the capture domain; and determining (i) all or a part of the sequence of the ligation product specifically bound to the capture domain, or a complement thereof, and (ii) all or a part of the sequence of the spatial barcode, or a complement thereof, and using the determined sequence of (i) and (ii) to identify the location of the analyte in the biological sample; wherein the first oligonucleotide (e.g., the first probe), the second oligonucleotide (e.g., the second probe), and the third oligonucleotide are DNA oligonucleotides, and wherein the first temperature is a higher temperature than the second temperature.

In some embodiments, the first oligonucleotide (e.g., the first probe) and the second oligonucleotide (e.g., the second probe) hybridize to an analyte at a first temperature. In some embodiments, the first temperature ranges from about 50°C to about 75°C, from about 55°C to about 70°C, or from about 60°C to about 65°C. In some embodiments, the first temperature is about 55°C, about 56°C, about 57°C, about 58°C, about 59°C, about 60°C, about 61°C, about 62°C, about 63°C, about 64°C, about 65°C, about 66°C, about 67°C, about 68°C, about 69°C, or about 70°C.

In some embodiments, after the step of hybridizing the first oligonucleotide (e.g., the first probe) and the second oligonucleotide (e.g., the second probe) to the analyte, a wash step is performed to remove unbound oligonucleotides (e.g., probes). The wash step can be performed using any of the wash methods and solutions described herein.

In some embodiments, after the step of hybridizing the first oligonucleotide (e.g., the first probe) and the second oligonucleotide (e.g., the second probe) to the analyte, a third oligonucleotide (e.g., a splint oligonucleotide) is added to the analyte. In some embodiments, the third oligonucleotide is an oligonucleotide. In some embodiments, the third oligonucleotide is a DNA oligonucleotide.

In some embodiments, the third oligonucleotide includes a sequence that is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to a portion of the first probe oligonucleotide (e.g., a portion of the first probe that is not hybridized to the analyte (e.g., an auxiliary sequence)). In some embodiments, the third oligonucleotide includes a sequence that is 100% complementary to a portion of the first oligonucleotide (e.g., the first probe). In some embodiments, the third oligonucleotide includes a sequence that is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to a portion of the second probe oligonucleotide (e.g., a portion of the second probe that is not hybridized to the analyte (e.g., an auxiliary sequence)). In some embodiments, the third oligonucleotide includes a sequence that is 100% complementary to a portion of the second oligonucleotide (e.g., the second probe). In some embodiments, the third oligonucleotide hybridizes to the first oligonucleotide (e.g., the first probe) at the complementary portion. In some embodiments, the third oligonucleotide hybridizes to the second oligonucleotide (e.g., the second probe) at the complementary portion.

In some embodiments, the third oligonucleotide hybridizes to the first oligonucleotide (e.g., the first probe) and to the second oligonucleotide (e.g., the second probe) at a second temperature. In some embodiments, the second temperature is lower than the first temperature at which the first and second oligonucleotides (e.g., the first and second probes) bind the analyte. In some embodiments, the second temperature ranges from about 15°C to about 35°C, from about 20°C to about 30°C, or from about 25°C to about 30°C. In some embodiments, the first temperature is about 15°C, about 16°C, about 17°C, about 18°C, about 19°C, about 20°C, about 21°C, about 22°C, about 23°C, about 24°C, about 25°C, about 26°C, about 27°C, about 28°C, about 29°C, about 30°C, about 31°C, about 32°C, about 33°C, about 34°C, or about 35°C. Methods including a third, or splint, oligonucleotide have been described in U.S. Patent Pub. No. 2019/0055594A1.

In some embodiments, after the step of hybridizing the third oligonucleotide to the analyte, a wash step is performed to remove unbound third oligonucleotides. The wash step can be performed using any of the wash methods and solutions described herein. In some embodiments, after the washing step, the first and second oligonucleotides (e.g., the first and second probes) are bound to (e.g., hybridized to) the analyte, and the third oligonucleotide is bound to (e.g., hybridized to) the first and second oligonucleotides (e.g., at portions of the first and second probes that are not bound to the analyte).

In some embodiments, the first oligonucleotide (e.g., the first probe), the second oligonucleotide (e.g., the second probe), and the third oligonucleotide are added to the biological sample at the same time. Then, in some embodiments, the temperature is adjusted to the first temperature to allow the first oligonucleotide (e.g., the first probe) and the second oligonucleotide (e.g., the second probe) to hybridize to the analyte in the biological sample. Next, the temperature is adjusted to the second temperature to allow the third oligonucleotide to hybridize to the first oligonucleotide and the second oligonucleotide.

In some embodiments where a third oligonucleotide hybridizes to a first probe and a second probe that are hybridized to targets sequences that are not directly adjacent in the analyte, the third oligonucleotide is extended to fill the gap between the first probe and the second probe. In some instances, a polymerase (e.g., a DNA polymerase) can extend one of the probes (e.g., the first probe) prior to ligation. For example, as shown in **FIG. 13****,** the first probe **1302** is extended **1312** to fill the gap **1314** between the first probe **1302** and the second probe **1303.**

In some embodiments, a ligation step is performed. Ligation can be performed using any of the methods described herein. In some embodiments, the step includes ligation of the first oligonucleotide (e.g., the first probe) and the second oligonucleotide (e.g., the second probe), forming a ligation product. In some embodiments, the third oligonucleotide serves as an oligonucleotide splint to facilitate ligation of the first oligonucleotide (e.g., the first probe) and the second oligonucleotide (e.g., the second probe). In some embodiments, ligation is chemical ligation. In some embodiments, ligation is enzymatic ligation. In some embodiments, the ligase is a T4 RNA ligase (Rnl2), a splintR ligase, a single stranded DNA ligase, or a T4 DNA ligase.

### (ii) Hybridization Buffer

In some embodiments, a first probe and a second probe are hybridized to the analyte in a hybridization buffer. In some instances, the hybridization buffer contains formamide. In other instances the hybridization buffer is formamide free. Formamide is not human friendly and it is a known health hazard. Chemically, it can oxidize over time, thereby impacting reagent shelf life and, most importantly, reagent efficacy. As such, the methods described herein can include formamide-free buffers, including formamide-free hybridization buffer.

In some embodiments, the formamide-free hybridization buffer is a saline-sodium citrate (SSC) hybridization buffer. In some embodiment, the SSC is present in the SSC hybridization buffer from about 1x SSC to about 6x SSC (e.g., about 1x SSC to about 5x SSC, about 1x SSC to about 4x SSC, about 1x SSC to about 3x SSC, about 1x SSC to about 2x SSC, about 2x SSC to about 6x SSC, about 2x SSC to about 5x SSC, about 2x SSC to about 4x SSC, about 2x SSC to about 3x SSC, about 3x SSC to about 5x SSC, about 3x SSC to about 4x SSC, about 4x SSC to about 6x SSC, about 4x SSC to about 6x SSC, about 4x SSC to about 5x SSC, or about 5x SSC to about 6x SSC). In some embodiments, the SSC is present in the SSC hybridization buffer from about 2x SSC to about 4x SSC. In some embodiments, SSPE hybridization buffer can be used.

In some embodiments, the SSC hybridization buffer comprises a solvent. In some embodiments, the solvent comprises ethylene carbonate instead of formamide (2020, Kalinka et al., Scientia Agricola 78(4):e20190315).

In some embodiments, the SSC hybridization buffer is at a temperature from about 40°C to about 60°C (e.g., about 40°C to about 55°C, about 40°C to about 50°C, about 40°C to about 45°C, about 45°C to about 60°C, about 45°C to about 55°C, about 45°C to about 50°C, about 50°C to about 60°C, about 50°C to about 55°C, or about 55°C to about 60°C). In some embodiments, the SSC hybridization buffer is at temperature from about 45°C to about 55°C, or any of the subranges described herein. In some embodiments, the SSC hybridization buffer is at a temperature of about 40°C, about 41°C, about 42°C, about 43°C, about 44°C, about 45°C, about 46°C, about 47°C, about 48°C, about 49°C, about 50°C, about 51°C, about 52°C, about 53°C, about 54°C, about 55°C, about 56°C, about 57°C, about 58°C, about 59°C, or about 60°C. In some embodiments, the SSC hybridization buffer is at a temperature of about 50°C.

In some embodiments, the SSC hybridization buffer further comprises one or more of a carrier, a crowder, or an additive. Non-limiting examples of a carrier that can be included in the hybridization buffer include: yeast tRNA, salmon sperm DNA, lambda phage DNA, glycogen, and cholesterol. Non-limiting examples of a molecular crowder that can be included in the hybridization buffer include: Ficoll, dextran, Denhardt's solution, and PEG. Non-limiting examples of additives that can be included in the hybridization buffer include: binding blockers, RNase inhibitors, Tm adjustors and adjuvants for relaxing secondary nucleic acid structures (e.g., betaine, TMAC, and DMSO). Further, a hybridization buffer can include detergents such as SDS, Tween, Triton-X 100, and sarkosyl (e.g., N-Lauroylsarcosine sodium salt). A skilled artisan would understand that a buffer for hybridization of nucleic acids could include many different compounds that could enhance the hybridization reaction.

### (iii) Hybridization between probe targeted for reduction or exclusion and blocker oligonucleotide

In some embodiments, one or more blocker oligonucleotides can be added to the hybridization buffer described herein. In some embodiments, the methods provided herein include hybridization of a first blocker oligonucleotide to a first probe targeted for reduction or exclusion, and hybridization of a second blocker oligonucleotide to a second probe targeted for reduction or exclusion. In some instances, the first and the second probe targeted for reduction or exclusion are within the same templated ligation probe pool that includes the first probe and the second probe.

In some instances, the methods include hybridization of blocker oligonucleotide pairs to probe pairs targeted for reduction or exclusion, where the blocker oligonucleotide pairs are in a medium at a concentration of about 1 nM to about 1 µM. In some instances, the concentration of the blocker oligonucleotide pairs is about 0.1, 0.2, 0.3, 0.4, 0.5, 0.6, 0.7, 0.8, 0.9, or 1 nM. In some instances, the concentration of the blocker oligonucleotide pairs is about 1, 2, 3, 4, 5, 6, 7, 8, 9, 10, 15, 20, 25, 30, 35, 40, 45, 50, 60, 70, 80, 90, 100, 200, 300, 400, 500, 600, 700, 800, 900, or 1000 nM. In some embodiments, the blocker oligonucleotides are diluted in a hybridization (Hyb) buffer.

In some instances, the concentration of a blocker oligonucleotide is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 2-fold, at least 3-fold, at least 4-fold, at least 5-fold, at least 6-fold, at least 7-fold, at least 8-fold, at least 9-folds, at least 10-fold as compared to that of an templated ligation probe targeted for reduction or exclusion.

### 1) Concurrent hybridization

In some embodiments, one or more blocker oligonucleotides (e.g., a first blocker oligonucleotide and a second blocker oligonucleotide) are concurrently added to a biological sample with a pool of templated ligation probes that include one or more templated ligation probes targeted for reduction or exclusion (e.g., a first and a second probe targeted for reduction or exclusion). In some instances, one or more blocker oligonucleotides are mixed with a pool of templated ligation probes, and the mixture is applied to a biological sample. In both cases, the hybridization of templated ligation probes with target analytes and the hybridization of blocker oligonucleotides with templated ligation probes targeted for reduction or exclusion occur at substantially the same time. For instance, blocker oligonucleotides can be added to a hybridization buffer (including a pool of templated ligation probes), and the hybridization buffer is applied to a biological sample. During the subsequent templated ligation probe hybridization step, the blocker oligonucleotides hybridize to their target templated ligation probes, thereby silencing such probes. In the same step (or within substantially the same period of time), those unsilenced templated ligation probes hybridize to their target analytes (e.g., analytes of interest). Hybridizing blocker oligonucleotides with templated ligation probes targeted for reduction or exclusion at the templated ligation probe hybridization step allows for less hybridization steps to be performed in templated ligation, as compared to a multi-step hybridization protocol.

### 2) Multi-step hybridization

In some embodiments, one or more blocker oligonucleotides (e.g., a first blocker oligonucleotide and a second blocker oligonucleotide) are hybridized to templated ligation probes targeted for reduction or exclusion (e.g., a first and a second probe targeted for reduction or exclusion) before the templated ligation probe hybridization step. For example, blocker oligonucleotides can be mixed with a pool of templated ligation probes, and the mixture is hybridized in a hybridization buffer at a first hybridization temperature, such that those templated ligation probes are silenced. Next, the hybridization buffer is applied to a biological sample, and the unsilenced templated ligation probes within the templated ligation probe pool hybridize to their target analytes (e.g., analytes of interest) at a second hybridization temperature.

In some embodiments, the hybridization buffer can be any one of the hybridization buffer described herein. In some embodiments, the first and the second hybridization temperatures are the same. In some embodiments, the first and the second hybridization temperatures are different. In some embodiments, the first and the second hybridization temperatures can be selected from any of the hybridization temperatures described herein.

### (i) Washing

In some embodiments, the methods disclosed herein also include a wash step. The wash step removes any unbound probes and/or unbound blocker oligonucleotides. Wash steps could be performed between any of the steps in the methods disclosed herein. For example, a wash step can be performed after adding probes and/or blocker oligonucleotides to the biological sample. As such, free/unbound probes and/or blocker oligonucleotides are washed away, leaving only probes that have hybridized to an analyte and/or blocker oligonucleotides that have hybridized to a templated ligation probe targeted for reduction or exclusion. In some instances, multiple (i.e., at least 2, 3, 4, 5, or more) wash steps occur between the methods disclosed herein. Wash steps can be performed at times (e.g., 1, 2, 3, 4, or 5 minutes) and temperatures (e.g., room temperature; 4°C known in the art and determined by a person of skill in the art.

In some instances, wash steps are performed using a wash buffer. In some instances, the wash buffer includes SSC (e.g., 1x SSC). In some instances, the wash buffer includes PBS (e.g., 1x PBS). In some instances, the wash buffer includes PBST (e.g., 1x PBST). In some instances, the wash buffer can also include formamide or be formamide free.

### (i) Wash Buffer

In some embodiments, after ligating a first probe and a second probe, the one or more unhybridized first probes, one or more unhybridized second probes, or both, are removed from the array. In some embodiments, after ligating a first probe, one or more spanning probes, and a second probe, the one or more unhybridized first, second, and/or spanning probes, are removed from the array. In some embodiments, after ligating a first probe, a second probe, and a third oligonucleotide, the one or more unhybridized first probes, one or more unhybridized second probes, or one or more third oligonucleotides, or all the above, are removed from the array.

In some embodiments, a pre-hybridization buffer is used to wash the sample. In some embodiments, a phosphate buffer is used. In some embodiments, multiple wash steps are performed to remove unbound oligonucleotides.

In some embodiments, removing includes washing the one or more unhybridized probes (e.g., a first probe, a second probe, a spanning probe, additional spanning probes, and a third oligonucleotide) and one or more unhybridized blocker oligonucleotides (e.g., any of the blocker oligonucleotides described herein) from the array in a formamide-free wash buffer.

In some embodiments, the formamide-free wash buffer is an SSC wash buffer. In some embodiments, SSC is present in the SSC wash buffer from about 0.01x SSC to about 1x SSC (e.g., about 0.01x SSC to about 0.5x SSC, 0.01x SSC to about 0.1x SSC, about 0.01x SSC to about 0.05x SSC, about 0.05x SSC to about 1x SSC, about 0.05x SSC to about 0.5x SSC, about 0.05x SSC to about 0.1x SSC, about 0.1x SSC to about 1x SSC, about 0.1x SSC to about 0.5x SSC, or about 0.5x SSC to about 1x SSC). In some embodiments, SSC is present in the SSC wash buffer at about 0.01x SSC, about 0.02x SSC, about 0.03x SSC, about 0.04x SSC, about 0.05x SSC, about 0.06x SSC, about 0.07x SSC, about 0.08x SSC, about 0.09x SSC, about 0.1x SSC, about 0.2x SSC, about 0.3x SSC, about 0.4x SSC, about 0.5x SSC, about 0.6x SSC, about 0.7x SSC, about 0.8x SSC, about 0.9x SSC, or about 0.1x SSC. In some embodiments, SSC is present in the SSC wash buffer at about 0.1x SSC.

In some embodiments, the SSC wash buffer comprises a detergent. In some embodiments, the detergent comprises sodium dodecyl sulfate (SDS). In some embodiments, SDS is present in the SSC wash buffer from about 0.01% (v/v) to about 0.5% (v/v) (e.g., about 0.01% (v/v) to about 0.4% (v/v), about 0.01% (v/v) to about 0.3% (v/v), about 0.01% (v/v) to about 0.2% (v/v), about 0.01% (v/v) to about 0.1% (v/v), about 0.05% (v/v) to about 0.5% (v/v), about 0.05% (v/v) to about 0.4% (v/v), about 0.05% (v/v) to about 0.3% (v/v), about 0.05% (v/v) to about 0.2% (v/v), about 0.05% (v/v) to about 0.1% (v/v), about 0.1% (v/v) to about 0.5% (v/v), about 0.1% (v/v) to about 0.4% (v/v), about 0.1% (v/v) to about 0.3% (v/v), about 0.1% (v/v) to about 0.2% (v/v), about 0.2% (v/v) to about 0.5% (v/v), about 0.2% (v/v) to about 0.4% (v/v), about 0.2% (v/v) to about 0.3% (v/v), about 0.3% (v/v) to about 0.5% (v/v), about 0.3% (v/v) to about 0.4% (v/v), or about 0.4% (v/v) to about 0.5% (v/v)). In some embodiments, the SDS is present the SSC wash buffer at about 0.01% (v/v), about 0.02% (v/v), about 0.03% (v/v), about 0.04% (v/v), about 0.05% (v/v), about 0.06% (v/v), about 0.07% (v/v), about 0.08% (v/v), about 0.09% (v/v), about 0.10% (v/v), about 0.2% (v/v), about 0.3% (v/v), about 0.4% (v/v), or about 0.5% (v/v), In some embodiments, the SDS is present in the SSC wash buffer at about 0.1% (v/v). In some embodiments, sarkosyl may be present in the SSC wash buffer.

In some embodiments, the SSC wash buffer is at a temperature from about 50°C to about 70°C (e.g., about 50°C to about 65°C, about 50°C to about 60°C, about 50°C to about 55°C, about 55°C to about 70°C, about 55°C to about 65°C, about 55°C to about 60°C, about 60°C to about 70°C, about 60°C to about 65°C, or about 65°C to about 70°C). In some embodiments, the SSC wash buffer is at a temperature from about 55°C to about 65°C. In some embodiments, the SSC wash buffer is at a temperature about 50°C, about 51°C, about 52°C, about 53°C, about 54°C, about 55°C, about 56°C, about 57°C, about 58°C, about 59°C, about 60°C, about 61°C, about 62°C, about 63°C, about 64°C, about 65°C, about 66°C, about 67°C, about 68°C, about 69°C, or about 70°C. In some embodiments, the SSC wash buffer is at a temperature of about 60°C.

In some embodiments, the method includes releasing the ligation product, where releasing is performed after the array is washed to remove the one or more unhybridized first and second probes.

### (j) Ligation

In some embodiments, after hybridization of the probe oligonucleotides (e.g., a first probe, a second probe, a spanning probe, additional spanning probes, and/or a third oligonucleotide) to the analyte, the probe (e.g., a first probe, a second probe, a spanning probe, additional spanning probes, and/or a third oligonucleotide) can be ligated together, creating a single ligation product that includes one or more sequences that are complementary to the analyte. Ligation can be performed enzymatically or chemically, as described herein.

In some instances, the ligation is an enzymatic ligation reaction, using a ligase (e.g., T4 RNA ligase (Rnl2), a PBCV-1 ligase, a Chlorella virus DNA ligase, a single stranded DNA ligase, or a T4 DNA ligase). See, e.g., Zhang et al.; RNA Biol. 2017; 14(1): 36-44, for a description of KOD ligase. Following the enzymatic ligation reaction, the probes (e.g., a first probe, a second probe, a spanning probe, additional spanning probes, and/or a third oligonucleotide) may be considered ligated.

In some embodiments, a polymerase catalyzes synthesis of a complementary strand of the ligation product, creating a double-stranded ligation product. In some instances, the polymerase is DNA polymerase. In some embodiments, the polymerase has 5' to 3' polymerase activity. In some embodiments, the polymerase has 3' to 5' exonuclease activity for proofreading. In some embodiments, the polymerase has 5' to 3' polymerase activity and 3' to 5' exonuclease activity for proofreading.

In some embodiments, the probe (e.g., a first probe, a second probe, a spanning probe, additional spanning probes, and/or a third oligonucleotide) may each comprise a reactive moiety such that, upon hybridization to the target and exposure to appropriate ligation conditions, the probe oligonucleotides may ligate to one another. In some embodiments, probe oligonucleotides that include a reactive moiety are ligated chemically. For example, a first probe capable of hybridizing to a first target region (e.g., a first target sequence or a first portion) of a nucleic acid molecule may comprise a first reactive moiety, and a second probe oligonucleotide capable of hybridizing to a second target region (e.g., a second target sequence or a second portion) of the nucleic acid molecule may comprise a second reactive moiety. When the first and second probes are hybridized to the first and second target regions (e.g., first and second target sequences) of the nucleic acid molecule, the first and second reactive moieties may be adjacent to one another. A reactive moiety of a probe may be selected from the non-limiting group consisting of azides, alkynes, nitrones (e.g., 1,3-nitrones), strained alkenes (e.g., trans-cycloalkenes such as cyclooctenes or oxanorbomadiene), tetrazines, tetrazoles, iodides, thioates (e.g., phorphorothioate), acids, amines, and phosphates. For example, the first reactive moiety of a first probe may comprise an azide moiety, and a second reactive moiety of a second probe may comprise an alkyne moiety. The first and second reactive moieties may react to form a linking moiety. A reaction between the first and second reactive moieties may be, for example, a cycloaddition reaction such as a strain-promoted azide-alkyne cycloaddition, a copper-catalyzed azide-alkyne cycloaddition, a strain-promoted alkyne-nitrone cycloaddition, a Diels-Alder reaction, a [3+2] cycloaddition, a [4+2] cycloaddition, or a [4+1] cycloaddition; a thiol-ene reaction; a nucleophilic substation reaction; or another reaction. In some cases, reaction between the first and second reactive moieties may yield a triazole moiety or an isoxazoline moiety. A reaction between the first and second reactive moieties may involve subjecting the reactive moieties to suitable conditions such as a suitable temperature, pH, or pressure and providing one or more reagents or catalysts for the reaction. For example, a reaction between the first and second reactive moieties may be catalyzed by a copper catalyst, a ruthenium catalyst, or a strained species such as a difluorooctyne, dibenzylcyclooctyne, or biarylazacyclooctynone. Reaction between a first reactive moiety of a first probe hybridized to a first target region (e.g., a first target sequence or first portion) of the nucleic acid molecule and a second reactive moiety of a third probe oligonucleotide hybridized to a second target region (e.g., a first target sequence or a first portion) of the nucleic acid molecule may link the first probe and the second probe to provide a ligated probe. Upon linking, the first and second probe may be considered ligated. Accordingly, reaction of the first and second reactive moieties may comprise a chemical ligation reaction such as a copper-catalyzed 5' azide to 3' alkyne "click" chemistry reaction to form a triazole linkage between two probe oligonucleotides. In other non-limiting examples, an iodide moiety may be chemically ligated to a phosphorothioate moiety to form a phosphorothioate bond, an acid may be ligated to an amine to form an amide bond, and/or a phosphate and amine may be ligated to form a phosphoramidate bond.

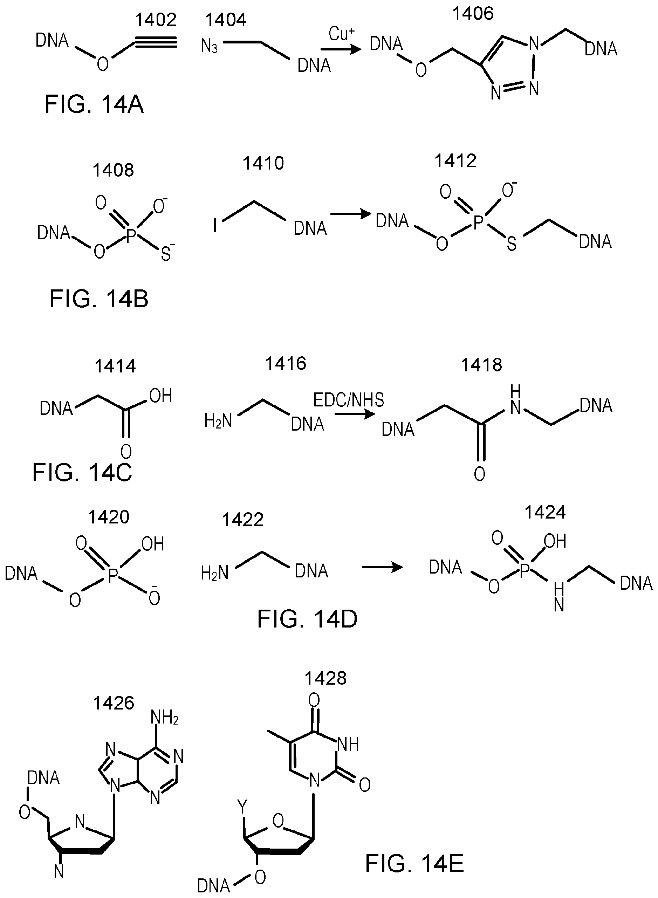

**FIGs. 14A-14E** illustrates examples of representative reactions. **FIG. 14A** shows a chemical ligation reaction of an alkyne moiety **1402** and an azide moiety **1204** reacting under copper-mediated cycloaddition to form a triazole linkage **1406.** **FIG. 14B** shows a chemical ligation reaction of a phosphorothioate group **1408** with an iodide group **1410** to form a phosphorothioate linkage **1412.** **FIG. 14C** shows a chemical ligation reaction of an acid **1414** and amine **1416** to form an amide linkage **1418.** **FIG. 14D** shows a chemical ligation reaction of a phosphate moiety **1420** and an amine moiety **1422** to form a phosphoramidate linkage **1424.** **FIG. 14E** shows a conjugation reaction of two species **1426** and **1428.**

In some instances, ligation is performed in a ligation buffer. In instances where probe ligation is performed on diribo-containing probes, the ligation buffer can include T4 RNA Ligase Buffer 2, enzyme (e.g., RNL2 ligase), and nuclease free water. In instances where probe ligation is performed on DNA probes, the ligation buffer can include Tris-HCl pH7.5, MnCl₂, ATP, DTT, surrogate fluid, enzyme (e.g., PBCV-1 ligase, ligase from a Chlorella virus), and nuclease-free water.

In some embodiments, the ligation buffer includes additional reagents. In some instances, the ligation buffer includes adenosine triphosphate (ATP) is added during the ligation reaction. DNA ligase-catalyzed sealing of nicked DNA substrates is first activated through ATP hydrolysis, resulting in covalent addition of an AMP group to the enzyme. After binding to a nicked site in a DNA duplex, the ligase transfers the AMP to the phosphorylated 5'-end at the nick, forming a 5'-5' pyrophosphate bond. Finally, the ligase catalyzes an attack on this pyrophosphate bond by the OH group at the 3'-end of the nick, thereby sealing it, whereafter ligase and AMP are released. If the ligase detaches from the substrate before the 3' attack, e.g. because of premature AMP reloading of the enzyme, then the 5' AMP is left at the 5'-end, blocking further ligation attempts. In some instances, ATP is added at a concentration of about 1µM, about 10 µM, about 100 µM, about 1000 µM, or about 10000 µM during the ligation reaction.

In some embodiments, cofactors that aid in joining of the probe oligonucleotides are added during the ligation process. In some instances, the cofactors include magnesium ions (Mg²⁺). In some instances, the cofactors include manganese ions (Mn²⁺). In some instances, Mg²⁺ is added in the form of MgCl₂. In some instances, Mn²⁺ is added in the form of MnCl₂. In some instances, the concentration of MgCl₂ is at about 1 mM, at about 10 mM, at about 100 mM, or at about 1000 mM. In some instances, the concentration of MnCl₂ is at about 1 mM, at about 10 mM, at about 100 mM, or at about 1000 mM.

In some embodiments, the ligation product includes a capture probe capture domain, which can bind to a capture probe (e.g., a capture probe immobilized, directly or indirectly, on a substrate). In some embodiments, methods provided herein include contacting a biological sample with a substrate, wherein the capture probe is affixed to the substrate (e.g., immobilized to the substrate, directly or indirectly). In some embodiments, the capture probe capture domain of the ligated probe specifically binds to the capture domain.

After ligation, in some instances, the biological sample is washed with a post-ligation wash buffer. In some instances, the post-ligation wash buffer includes one or more of SSC (e.g., 1x SSC), ethylene carbonate or formamide, and nuclease free water. In some instances, the biological sample is washed at this stage at about 50°C to about 70°C. In some instances, the biological sample is washed at about 60°C.

### (i) Ligation Including Pre-Adenylated 5' Phosphate on Second Probe

Provided herein are methods for determining a location of a target nucleic acid in a biological sample that include: (a) contacting the biological sample with a substrate comprising a plurality of capture probes, where a capture probe of the plurality of capture probes comprises a capture domain and a spatial barcode; (b) hybridizing a target nucleic acid in the biological sample with a first probe and a second probe, where the first probe comprises, from 3' to 5', a sequence substantially complementary to the capture domain and a sequence that is substantially complementary to a first sequence in the target nucleic acid and has a pre-adenylated phosphate group at its 5' end; the second probe comprises a sequence substantially complementary to a second sequence in the target nucleic acid; (c) generating a ligation product by ligating a 3' end of the second probe to the 5' end of the first probe using a ligase that does not require adenosine triphosphate for ligase activity; (d) releasing the ligation product from the target nucleic acid and binding the capture domain of the ligation product specifically to the capture domain of capture probe; and (e) determining (i) all or a part of a sequence corresponding to the ligation product, or a complement thereof, and (ii) all or a part of a sequence corresponding to the spatial barcode, or a complement thereof, and using the determined sequences of (i) and (ii) to identify the location of the target nucleic acid in the biological sample.

In some instances, the ligase that does not require adenosine triphosphate for ligase activity (e.g., thermostable 5' AppDNA/RNA Ligase, truncated T4 RNA Ligase 2 (trRnl2), truncated T4 RNA Ligase 2 K227Q, truncated T4 RNA Ligase 2 KQ, Chlorella Virus PBCV-1 DNA Ligase, and combinations thereof). See, e.g., Nichols et al., "RNA Ligases," Curr. Protocol. Molec. Biol. 84(1):3.15.1-.4 (2008); Viollet et al., "T4 RNA Ligase 2 Truncated Active Site Mutants: Improved Tools for RNA Analysis," BMC Biotechnol. 11: 72 (2011); and Ho et al., "Bacteriophage T4 RNA Ligase 2 (gp24.1) Exemplifies a Family of RNA Ligases Found in All Phylogenetic Domains," PNAS 99(20):12709-14 (2002) for a description of T4 RNA Ligases and truncated T4 RNA Ligases. Thermostable 5' AppDNA/RNA Ligase is an enzyme belonging to the Ligase family that catalyzes the ligation of the 3' end of ssRNA or ssDNA to a 5'-adenylated ssDNA or 5'-adenylated ssRNA. Truncated T4 RNA Ligase 2 is an enzyme belonging to the Ligase family that catalyzes the ligation of dsRNA nicks and ssRNA to ssRNA. It can also ligate the 3' end of RNA or DNA to a 5'-pDNA when annealed to an RNA complement, and the 3' end of RNA to a 5'-pRNA when annealed to a DNA complement, with reduced efficiency. Truncated T4 RNA Ligase 2 K227Q is an enzyme belonging to the Ligase family that catalyzes the ligation of the 3' end of ssRNA to 5' adenylated ssDNA and 5' adenylated ssRNA. It has a reduction of side products as compared to truncated T4 RNA Ligase 2. Truncated T4 RNA Ligase 2 KQ is an enzyme belonging to the Ligase family that catalyzes the ligation of the 3' end of ssRNA to 5' adenylated ssDNA and 5' adenylated ssRNA. It is a preferred choice for ligation of ssRNA to preadenylated adapters and has a reduction of side products as compared to truncated T4 RNA Ligase 2.

In some embodiments, the T4 RNA Ligase comprises a K227Q mutation. *See* Viollet et al., "T4 RNA Ligase 2 Truncated Active Site Mutants: Improved Tools for RNA Analysis," BMC Biotechnol. 11.

In some instances, cofactors that aid in ligation of the first and second probe are added during ligation. In some instances, the cofactors include magnesium ions (Mg²⁺). In some instances, the cofactors include manganese ions (Mn²⁺). In some instances, Mg²⁺ is added in the form of MgCl₂. In some instances, Mn²⁺ is added in the form of MnCl₂. In some instances, the concentration of MgCl₂ is at about 1 mM to about 10 mM. In some instances, the concentration of MnCl₂ is at about 1 mM to about 10 mM.

In some instances, the ligation occurs at a pH in the range of about 6.5 to about 9.0, about 6.5 to about 8.0, or about 7.5 to about 8.0.

### (k) Sandwich Processes

In some instances, the methods provided herein include aligning (i.e., sandwiching) a first substrate having the biological sample with a second substrate that includes a plurality of capture probes, thereby "sandwiching" the biological sample between the two substrates. Upon interaction of the biological sample with the substrate having a plurality of probes (in either instance), the location and abundance of an analyte in a biological sample can be determined, as provided herein. This methods includes an advantage in that steps provided herein prior to analyte or analyte-derived molecule by the capture probe, most-if not all-steps can be performed on a substrate that does not have capture probes, thereby providing a method that is cost effective. Details of the methods can be found, e.g., in WO2022140028A1.

### (l) Permeabilization and Releasing the Ligation Product

In some embodiments, the methods provided herein include a permeabilizing step. In some embodiments, permeabilization occurs using a protease. In some embodiments, the protease is an endopeptidase. Endopeptidases that can be used include but are not limited to trypsin, chymotrypsin, elastase, thermolysin, pepsin, clostripan, glutamyl endopeptidase (GluC), ArgC, peptidyl-asp endopeptidase (ApsN), endopeptidase LysC and endopeptidase LysN. In some embodiments, the endopeptidase is pepsin. In some embodiments, after creating a ligation product (e.g., by ligating a first probe and a second probe that are hybridized to adjacent sequences in the analyte), the biological sample is permeabilized. In some embodiments, the biological sample is permeabilized contemporaneously with or prior to contacting the biological sample with a first probe and a second probe, hybridizing the first probe and the second probe to the analyte, generating a ligation product by ligating the first probe and the second probe, and releasing the ligated product from the analyte.

In some embodiments, methods provided herein include permeabilization of the biological sample such that the capture probe can more easily bind to the captured ligated probe (i.e., compared to no permeabilization). In some embodiments, reverse transcription (RT) reagents can be added to permeabilized biological samples. Incubation with the RT reagents can produce spatially-barcoded full-length nucleic acids from the captured analytes (e.g., polyadenylated mRNA).

In some instances, the permeabilization step includes application of a permeabilization buffer to the biological sample. In some instances, the permeabilization buffer includes a buffer (e.g., Tris pH 7.5), MgCl₂, sarkosyl detergent (e.g., sodium lauroyl sarcosinate), enzyme (e.g., proteinase K, and nuclease free water. In some instances, the permeabilization step is performed at 37°C. In some instances, the permeabilization step is performed for about 20 minutes to 2 hours (e.g., about 20 minutes, about 30 minutes, about 40 minutes, about 50 minutes, about 1 hour, about 1.5 hours, or about 2 hours). In some instances, the releasing step is performed for about 40 minutes.

In some embodiments, after generating a ligation product, the ligation product is released from the analyte. In some embodiments, a ligation product is released from the analyte using an endoribonuclease. In some embodiments, the endoribonuclease is RNase H, RNase A, RNase C, or RNase I. In some embodiments, the endoribonuclease is RNase H. RNase H is an endoribonuclease that specifically hydrolyzes the phosphodiester bonds of RNA, when hybridized to DNA. RNase H is part of a conserved family of ribonucleases which are present in many different organisms. There are two primary classes of RNase H: RNase H1 and RNase H2. Retroviral RNase H enzymes are similar to the prokaryotic RNase H1. All of these enzymes share the characteristic that they are able to cleave the RNA component of an RNA:DNA heteroduplex. In some embodiments, the RNase H is RNase H1, RNase H2, or RNase H1 and RNase H2. In some embodiments, the RNase H includes but is not limited to RNase HII from *Pyrococcus furiosus*, RNase HII from *Pyrococcus horikoshi*, RNase HI from *Thermococcus litoralis*, RNase HI from *Thermus thermophilus*, RNAse HI from *E. coli*, or RNase HII from *E. coli.*

In some instances, the releasing step is performed using a releasing buffer. In some instances, the release buffer includes one or more of a buffer (e.g., Tris pH 7.5), enzyme (e.g., RNAse H) and nuclease-free water. In some instances, the releasing step is performed at 37°C. In some instances, the releasing step is performed for about 20 minutes to 2 hours (e.g., about 20 minutes, about 30 minutes, about 40 minutes, about 50 minutes, about 1 hour, about 1.5 hours, or about 2 hours). In some instances, the releasing step is performed for about 30 minutes.

In some instances, the releasing step occurs before the permeabilization step. In some instances, the releasing step occurs after the permeabilization step. In some instances, the releasing step occurs at the same time as the permeabilization step (e.g., in the same buffer).

### (m) Blocking probes

In some embodiments, a capture probe capture domain is blocked prior to adding a second probe oligonucleotide to a biological sample. This prevents the capture probe capture domain from prematurely hybridizing to the capture domain.

In some embodiments, a blocking probe is used to block or modify the free 3' end of the capture probe capture domain. In some embodiments, a blocking probe can be hybridized to the capture probe capture domain of the second probe to mask the free 3' end of the capture probe capture domain. In some embodiments, a blocking probe can be a hairpin probe or partially double stranded probe. In some embodiments, the free 3' end of the capture probe capture domain of the second probe can be blocked by chemical modification, e.g., addition of an azidomethyl group as a chemically reversible capping moiety such that the capture probes do not include a free 3' end. Blocking or modifying the capture probe capture domain, particularly at the free 3' end of the capture probe capture domain, prior to contacting second probe with the substrate, prevents hybridization of the second probe to the capture domain (e.g., prevents the capture of a poly(A) of a capture probe capture domain to a poly(T) capture domain). In some embodiments, a blocking probe can be referred to as a capture probe capture domain blocking moiety.

In some embodiments, the blocking probes can be reversibly removed. For example, blocking probes can be applied to block the free 3' end of either or both the capture probe capture domain and/or the capture probes. Blocking interaction between the capture probe capture domain and the capture probe on the substrate can reduce non-specific capture to the capture probes. After the second probe hybridizes to the analyte and is ligated to a first probe, one or more spanning probes, or a third oligonucleotide, the blocking probes can be removed from the 3' end of the capture probe capture domain and/or the capture probe, and the ligation product can migrate to and become bound by the capture probes on the substrate. In some embodiments, the removal includes denaturing the blocking probe from capture probe capture domain and/or capture probe. In some embodiments, the removal includes removing a chemically reversible capping moiety. In some embodiments, the removal includes digesting the blocking probe with an RNase (e.g., RNase H).

In some embodiments, the blocking probes are oligo (dT) blocking probes. In some embodiments, the oligo (dT) blocking probes can have a length of 15-30 nucleotides. In some embodiments, the oligo (dT) blocking probes can have a length of 10-50 nucleotides, e.g., 10-50, 10-45, 10-40, 10-35, 10-30, 10-25, 10-20, 10-15, 15-50, 15-45, 15-40, 15-35, 15-30, 15-25, 15-20, 20-50, 20-45, 20-40, 20-35, 20-30, 20-25, 25-50, 25-45, 25-40, 25-35, 25-30, 30-50, 30-45, 30-40, 30-35, 35-50, 35-45, 35-40, 40-50, 40-45, or 45-50 nucleotides. In some embodiments, the analyte capture agents can be blocked at different temperatures (e.g., 4°C and 37°C).

### (n) Biological Samples

Methods disclosed herein can be performed on any type of sample. In some embodiments, the sample is a fresh tissue. In some embodiments, the sample is a frozen sample. In some embodiments, the sample was previously frozen. In some embodiments, the sample is a formalin-fixed, paraffin embedded (FFPE) sample.

Subjects from which biological samples can be obtained can be healthy or asymptomatic individuals, individuals that have or are suspected of having a disease (e.g., cancer) or a pre-disposition to a disease, and/or individuals that are in need of therapy or suspected of needing therapy. In some instances, the biological sample can include one or more diseased cells. A diseased cell can have altered metabolic properties, gene expression, protein expression, and/or morphologic features. Examples of diseases include inflammatory disorders, metabolic disorders, nervous system disorders, and cancer. In some instances, the biological sample includes cancer or tumor cells. Cancer cells can be derived from solid tumors, hematological malignancies, cell lines, or obtained as circulating tumor cells. In some instances, the biological sample is a heterogeneous sample. In some instances, the biological sample is a heterogeneous sample that includes tumor or cancer cells and/or stromal cells,
In some instances, the cancer is breast cancer. In some instances, the breast cancer is triple positive breast cancer (TPBC). In some instances, the breast cancer is triple negative breast cancer (TNBC).

In some instances, the cancer is colorectal cancer. In some instances, the cancer is ovarian cancer. In certain embodiments, the cancer is squamous cell cancer, small-cell lung cancer, non-small cell lung cancer, gastrointestinal cancer, Hodgkin's or non-Hodgkin's lymphoma, pancreatic cancer, glioblastoma, glioma, cervical cancer, ovarian cancer, liver cancer, bladder cancer, breast cancer, colon cancer, colorectal cancer, endometrial carcinoma, myeloma, salivary gland carcinoma, kidney cancer, basal cell carcinoma, melanoma, prostate cancer, vulval cancer, thyroid cancer, testicular cancer, esophageal cancer, or a type of head or neck cancer. In certain embodiments, the cancer treated is desmoplastic melanoma, inflammatory breast cancer, thymoma, rectal cancer, anal cancer, or surgically treatable or non-surgically treatable brain stem glioma. In some embodiments, the subject is a human.

FFPE samples generally are heavily cross-linked and fragmented, and therefore this type of sample allows for limited RNA recovery using conventional detection techniques. In certain embodiments, methods of targeted RNA capture provided herein are less affected by RNA degradation associated with FFPE fixation than other methods (e.g., methods that take advantage of oligo-dT capture and reverse transcription of mRNA). In certain embodiments, methods provided herein enable sensitive measurement of specific genes of interest that otherwise might be missed with a whole transcriptomic approach.

In some instances, FFPE samples are stained (e.g., using H&E). The methods disclosed herein are compatible with H&E will allow for morphological context overlaid with transcriptomic analysis. However, depending on the need some samples may be stained with only a nuclear stain, such as staining a sample with only hematoxylin and not eosin, when location of a cell nucleus is needed.

In some embodiments, a biological sample (e.g. tissue section) can be fixed with methanol, stained with hematoxylin and eosin, and imaged. In some embodiments, fixing, staining, and imaging occurs before one or more probes are hybridized to the sample. Some embodiments of any of the workflows described herein can further include a destaining step (e.g., a hematoxylin and eosin destaining step), after imaging of the sample and prior to permeabilizing the sample. For example, destaining can be performed by performing one or more (e.g., one, two, three, four, or five) washing steps (e.g., one or more (e.g., one, two, three, four, or five) washing steps performed using a buffer including HCl). The images can be used to map spatial gene expression patterns back to the biological sample. A permeabilization enzyme can be used to permeabilize the biological sample directly on the slide.

In some embodiments, the FFPE sample is deparaffinized, permeabilized, equilibrated, and blocked before target probe oligonucleotides are added. In some embodiments, deparaffinization using xylenes. In some embodiments, deparaffinization includes multiple washes with xylenes. In some embodiments, deparaffinization includes multiple washes with xylenes followed by removal of xylenes using multiple rounds of graded alcohol followed by washing the sample with water. In some aspects, the water is deionized water. In some embodiments, equilibrating and blocking includes incubating the sample in a pre-Hyb buffer. In some embodiments, the pre-Hyb buffer includes yeast tRNA. In some embodiments, permeabilizing a sample includes washing the sample with a phosphate buffer. In some embodiments, the buffer is PBS. In some embodiments, the buffer is PBST.

### (o) Determining the Sequence of the Ligation Product

After a target analyte proxy such as a ligation product has hybridized or otherwise been associated with a capture probe according to any of the methods described above in connection with the general spatial cell-based analytical methodology, the barcoded constructs that result from hybridization/association are analyzed.

In some embodiments, after contacting a biological sample with a substrate that includes capture probes, a removal step can optionally be performed to remove all or a portion of the biological sample from the substrate. In some embodiments, the removal step includes enzymatic and/or chemical degradation of cells of the biological sample. For example, the removal step can include treating the biological sample with an enzyme (e.g., a proteinase, e.g., proteinase K) to remove at least a portion of the biological sample from the substrate. In some embodiments, the removal step can include ablation of the tissue (e.g., laser ablation).

In some embodiments, a biological sample is not removed from the substrate. For example, the biological sample is not removed from the substrate prior to releasing a capture probe (e.g., a capture probe bound to an analyte) from the substrate. In some embodiments, such releasing comprises cleavage of the capture probe from the substrate (e.g., via a cleavage domain). In some embodiments, such releasing does not comprise releasing the capture probe from the substrate (e.g., a copy of the capture probe bound to an analyte can be made and the copy can be released from the substrate, e.g., via denaturation). In some embodiments, the biological sample is not removed from the substrate prior to analysis of an analyte bound to a capture probe after it is released from the substrate. In some embodiments, the biological sample remains on the substrate during removal of a capture probe from the substrate and/or analysis of an analyte bound to the capture probe after it is released from the substrate. In some embodiments, the biological sample remains on the substrate during removal (e.g., via denaturation) of a copy of the capture probe (e.g., complement). In some embodiments, analysis of an analyte bound to capture probe from the substrate can be performed without subjecting the biological sample to enzymatic and/or chemical degradation of the cells (e.g., permeabilized cells) or ablation of the tissue (e.g., laser ablation).

In some embodiments, at least a portion of the biological sample is not removed from the substrate. For example, a portion of the biological sample can remain on the substrate prior to releasing a capture probe (e.g., a capture prove bound to an analyte) from the substrate and/or analyzing an analyte bound to a capture probe released from the substrate. In some embodiments, at least a portion of the biological sample is not subjected to enzymatic and/or chemical degradation of the cells (e.g., permeabilized cells) or ablation of the tissue (e.g., laser ablation) prior to analysis of an analyte bound to a capture probe from the substrate.

In some embodiments, the method further includes subjecting a region of interest in the biological sample to spatial transcriptomic analysis. In some embodiments, one or more of the capture probes includes a capture domain. In some embodiments, one or more of the capture probes comprises a unique molecular identifier (UMI). In some embodiments, one or more of the capture probes comprises a cleavage domain. In some embodiments, the cleavage domain comprises a sequence recognized and cleaved by uracil-DNA glycosylase, apurinic/apyrimidinic (AP) endonuclease (APE1), Uracil-specific excision reagent (USER), and/or an endonuclease VIII. In some embodiments, one or more capture probes do not comprise a cleavage domain and is not cleaved from the array.

In some embodiments, a capture probe can be extended (an "extended capture probe," e.g., as described herein) in order to create a copy of the ligated probe. This process involves synthesis of a complementary strand of the ligated probe, e.g., generating a full length complement of the ligation product. In some embodiments, the capture probe is extended using one or more DNA polymerases.

In some embodiments, a capture domain of a capture probe includes a primer for producing the complementary strand of a ligated probe hybridized to the capture probe, e.g., a primer for DNA polymerase. The nucleic acid, e.g., DNA and/or ligated probes or complement thereof, molecules generated by the extension reaction incorporate the sequence of the capture probe. The extension of the capture probe, e.g., a DNA polymerase and/or reverse transcription reaction, can be performed using a variety of suitable enzymes and protocols.

In some embodiments, a full-length DNA molecule is generated. In some embodiments, a "full-length" DNA molecule refers to the whole of the captured ligated product. For example, the full length DNA molecule refers to the whole of the ligation product released following RNase H digestion of the RNA from the RNA:DNA duplex.

In some embodiments, double-stranded extended capture probes (now containing the ligated probe) are treated to remove any unextended capture probes prior to amplification and/or analysis, e.g., sequence analysis. This can be achieved by a variety of methods, e.g., using an enzyme to degrade the unextended probes, such as an exonuclease enzyme, or purification columns.

In some embodiments, extended capture probes containing the complement of the ligated probe are amplified to yield quantities that are sufficient for analysis, e.g., via DNA sequencing. In some embodiments, the extended ligation products (e.g., the capture probe plus the ligated probe) act as a template for the amplification reaction (e.g., a polymerase chain reaction).

In some embodiments, the amplification reaction incorporates an affinity group onto the extended ligation product using a primer including the affinity group. In some embodiments, the primer includes an affinity group and the extended ligation product includes the affinity group. The affinity group can correspond to any of the affinity groups described previously.

In some embodiments, the extended ligation products that include the affinity group can be coupled to a substrate specific for the affinity group. In some embodiments, the substrate can include an antibody or antibody fragment. In some embodiments, the substrate includes avidin or streptavidin and the affinity group includes biotin. In some embodiments, the substrate includes maltose and the affinity group includes maltose-binding protein. In some embodiments, the substrate includes maltose-binding protein and the affinity group includes maltose. In some embodiments, amplifying the extended ligation products can function to release the products from the substrate, insofar as copies of the extended ligation products are not immobilized on the substrate.

In some embodiments, the extended ligation product or complement or amplicon thereof is released. The step of releasing from the surface of the substrate can be achieved in a number of ways. In some embodiments, an extended ligation product or a complement thereof is released from the array by nucleic acid cleavage and/or by denaturation (e.g., by heating to denature a double-stranded molecule).

In some embodiments, the extended ligation product or complement or amplicon thereof is released from the array by physical means. For example, where the extended ligation product is indirectly immobilized on the array substrate, e.g., via hybridization to a surface probe, it can be sufficient to disrupt the interaction between the extended ligation product and the surface probe. Methods for disrupting the interaction between nucleic acid molecules include denaturing double stranded nucleic acid molecules are known in the art. A straightforward method for releasing the DNA molecules (i.e., of stripping the array of extended products) is to use a solution that interferes with the hydrogen bonds of the double stranded molecules. In some embodiments, the extended capture product is released by an applying heated solution, such as water or buffer, of at least 85°C, e.g., at least 90, 91, 92, 93, 94, 95, 96, 97, 98, or 99°C. In some embodiments, a solution including salts, surfactants, etc. that can further destabilize the interaction between the nucleic acid molecules is added to release the extended ligation product from the substrate.

In some embodiments, probes complementary to the extended ligation product can be contacted with the substrate. In some embodiments, the biological sample can be in contact with the substrate when the probes are contacted with the substrate. In some embodiments, the biological sample can be removed from the substrate prior to contacting the substrate with probes. In some embodiments, the probes can be labeled with a detectable label (e.g., any of the detectable labels described herein). In some embodiments, probes that do not specially bind (e.g., hybridize) to an extended ligation product can be washed away. In some embodiments, probes complementary to the extended ligation product can be detected on the substrate (e.g., imaging, any of the detection methods described herein).

In some embodiments, probes complementary to an extended ligation products can be about 4 nucleotides to about 100 nucleotides long. In some embodiments, probes (e.g., detectable probes) can be about 10 nucleotides to about 90 nucleotides long. In some embodiments, probes (e.g., detectable probes) can be about 20 nucleotides to about 80 nucleotides long. In some embodiments, probes (e.g., detectable probes) can be about 30 nucleotides to about 60 nucleotides long. In some embodiments, probes (e.g., detectable probes) can be about 40 nucleotides to about 50 nucleotides long. In some embodiments, probes (e.g., detectable probes) can be about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, about 60, about 61, about 62, about 63, about 64, about 65, about 66, about 67, about 68, about 69, about 70, about 71, about 72, about 73, about 74, about 75, about 76, about 77, about 78, about 79, about 80, about 81, about 82, about 83, about 84, about 85, about 86, about 87, about 88, about 89, about 90, about 91, about 92, about 93, about 94, about 95, about 96, about 97, about 98, and about 99 nucleotides long.

In some embodiments, about 1 to about 100 probes can be contacted to the substrate and specifically bind (e.g., hybridize) to an extended ligation product. In some embodiments, about 1 to about 10 probes can be contacted to the substrate and specifically bind (e.g., hybridize) to an extended ligation product. In some embodiments, about 10 to about 100 probes can be contacted to the substrate and specifically bind (e.g., hybridize) to an extended ligation product. In some embodiments, about 20 to about 90 probes can be contacted to the substrate. In some embodiments, about 30 to about 80 probes (e.g., detectable probes) can be contacted to the substrate. In some embodiments, about 40 to about 70 probes can be contacted to the substrate. In some embodiments, about 50 to about 60 probes can be contacted to the substrate. In some embodiments, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, about 60, about 61, about 62, about 63, about 64, about 65, about 66, about 67, about 68, about 69, about 70, about 71, about 72, about 73, about 74, about 75, about 76, about 77, about 78, about 79, about 80, about 81, about 82, about 83, about 84, about 85, about 86, about 87, about 88, about 89, about 90, about 91, about 92, about 93, about 94, about 95, about 96, about 97, about 98, and about 99 probes can be contacted to the substrate and specifically bind (e.g., hybridize) to an extended ligation product.

In some embodiments, the probes can be complementary to a ligation product of a single analyte (e.g., a single gene). In some embodiments, the probes can be complementary to one or more analyte ligation products (e.g., analytes in a family of genes). In some embodiments, the probes (e.g., detectable probes) can be directed to ligation products for a panel of genes associated with a disease (e.g., cancer, Alzheimer's disease, Parkinson's disease).

Sequencing of polynucleotides can be performed by various systems. More generally, sequencing can be performed using nucleic acid amplification, polymerase chain reaction (PCR) (e.g., digital PCR and droplet digital PCR (ddPCR), quantitative PCR, real time PCR, multiplex PCR, PCR-based single plex methods, emulsion PCR), and/or isothermal amplification. Non-limiting examples of methods for sequencing genetic material include, but are not limited to, DNA hybridization methods (e.g., Southern blotting), restriction enzyme digestion methods, Sanger sequencing methods, next-generation sequencing methods (e.g., single-molecule real-time sequencing, nanopore sequencing, and Polony sequencing), ligation methods, and microarray methods.

### (p) Kits and Compositions

In some embodiments, also provided herein are kits and compositions that include one or more reagents to detect one or more analytes described herein. In some instances, the kit or composition includes a substrate comprising a plurality of capture probes comprising a spatial barcode and the capture domain. In some instances, the kit or composition includes a plurality of probes (e.g., a first probe, a second probe, one or more spanning probes, and/or a third oligonucleotide).

A non-limiting example of a kit or composition used to perform any of the methods described herein includes: (a) a substrate comprising a plurality of capture probes comprising a spatial barcode and a capture domain; (b) a system comprising: a plurality of first probes and second probes, wherein a first probe and a second probe each comprises sequences that are substantially complementary to an analyte, and wherein the second probe comprises a capture probe capture domain; (c) a system comprising: a plurality of first blocker oligonucleotides and a plurality of second blocker oligonucleotides, wherein a first blocker oligonucleotide of the plurality of first blocker oligonucleotides is substantially complementary to sequences of a first probe targeted for reduction or exclusion in the plurality of first probes, and a second blocker oligonucleotide of the plurality of second blocker oligonucleotides is substantially complementary to sequences of a second probe targeted for reduction or exclusion in the plurality of second probes, wherein the first and the second probe targeted for reduction or exclusion each comprises sequences that are substantially complementary to sequences of an analyte targeted for reduction or exclusion; and (d) instructions for performing the method as described herein.

Another non-limiting example of a kit or composition used to perform any of the methods described herein includes: (a) an array comprising a plurality of capture probes; (b) a plurality of probes comprising a first probe and a second, wherein the first probe and the second probe are substantially complementary to adjacent sequences of an analyte, wherein the second probe comprises (i) a capture probe binding domain that is capable of binding to a capture domain of the capture probe and (ii) a linker sequence; (c) a plurality of enzymes comprising a ribonuclease and a ligase; (d) a plurality of blocker oligonucleotides comprising a first blocker oligonucleotide and a second blocker oligonucleotide, wherein the first blocker oligonucleotide is substantially complementary to sequences of a first probe targeted for reduction or exclusion in the plurality of probes, and the second blocker oligonucleotide is substantially complementary to sequences of a second probe targeted for reduction or exclusion in the plurality of probes, wherein the first and the second probe targeted for reduction or exclusion each comprises sequences that are substantially complementary to sequences of an analyte targeted for reduction or exclusion; and (e) instructions for performing the method as described herein.

Another non-limiting example of a kit or composition used to perform any of the methods described herein includes: (a) an array comprising a plurality of capture probes; (b) a plurality of probes comprising a first probe and a second probe, wherein the first probe and the second probe are substantially complementary to adjacent sequences of an analyte, wherein the first probe includes a linker sequence, wherein the second probe comprises a capture probe binding domain that is capable of binding to a capture domain of the capture probe; (c) a plurality of enzymes comprising a ribonuclease and a ligase; (d) a plurality of blocker oligonucleotides comprising a first blocker oligonucleotide and a second blocker oligonucleotide, wherein the first blocker oligonucleotide is substantially complementary to sequences of a first probe targeted for reduction or exclusion in the plurality of probes, and the second blocker oligonucleotide is substantially complementary to sequences of a second probe targeted for reduction or exclusion in the plurality of probes, wherein the first and the second probe targeted for reduction or exclusion each comprises sequences that are substantially complementary to sequences of an analyte targeted for reduction or exclusion; and (e) instructions for performing the method as described herein.

In some embodiments of any of the kits or composition described herein, the kit or composition includes a second probe that includes a preadenylated phosphate group at its 5' end and a first probe comprising at least two ribonucleic acid bases at the 3' end.

Another non-limiting example of a kit or composition used to perform any of the methods described herein includes: (a) a spatial array comprising a plurality of capture probes, wherein a capture probe of the plurality of capture probes comprises a spatial barcode and a capture domain; (b) a biological sample on the spatial array, wherein the biological sample comprises a plurality of analytes comprising an analyte and one or more analytes for decreased capture, (c) a first probe and a second probe that are ligated together, wherein the first probe and the second probe each comprises one or more sequences that are substantially complementary to sequences of the analyte, and wherein either the first probe or the second probe comprises a capture probe capture domain that is hybridized to the capture domain of the capture probe of the spatial array; and (d) a third probe and a fourth probe targeting the one or more analytes for decreased capture, each comprising one or more sequences that are substantially complementary to sequences of the one or more analytes for decreased capture, wherein a first blocker oligonucleotide with sequence complementary to the third probe is hybridized to the third probe, and a second blocker oligonucleotide with sequence complementary to the fourth probe is hybridized to the fourth probe. In some embodiments, the third probe comprises a capture probe capture domain, and the sequence of the first blocker oligonucleotide is substantially complementary to the sequence of the third probe excluding the capture probe capture domain; or the fourth probe comprises a capture probe capture domain, and the sequence of the second blocker oligonucleotide is substantially complementary to the sequence of the fourth probe excluding the capture probe capture domain. In some embodiments, the composition further comprises an RNase H enzyme. In some embodiments, the composition further comprises a ligase. In some embodiments, the first probe or the second probe that does not comprise the capture probe capture domain comprises a functional domain. In some embodiments, the third and fourth probes described herein refer to probes targeted for reduction or exclusion described herein. In some embodiments, the third and fourth probes described herein refer to a probe pair targeting one or more analytes for decreased capture described herein.

### EXAMPLES

The invention is further described in the following examples, which do not limit the scope of the invention which is set out in the appended set of claims.

### Example 1. Spatial gene expression analysis of FFPE-fixed samples using templated ligation

As an overview, a non-limiting example of templated ligation on an FFPE-fixed sample was performed as described in **FIG. 15****.** FFPE-fixed samples were deparaffinized, stained (e.g., H&E stain), and imaged **1501.** Samples were destained (e.g., using HCl) and decrosslinked **1502.** Following decrosslinking, samples treated with pre-hybridization buffer (e.g., hybridization buffer without the first and second probes), probes were added to the sample, probes hybridized, and samples were washed **1503.** Ligase was added to the samples to ligate hybridized probes to generate a ligation product and samples were then washed **1504.** Probes were released from the analyte by contacting the biological sample with RNAse H **1505.** Samples were permeabilized to facilitate capture of the ligation product by the capture probes on the substrate **1506.** Ligation products that hybridized to the capture probes were extended **1507.** The extended capture probes were denatured **1508.** Denatured, extended capture probes were indexed and the amplified libraries were subjected to quality control **1509** before being sequenced.

In a non-limiting example, mouse brain and mouse kidney FFPE sections on standard slides (for sandwich conditions) or gene expression (GEx) slides (for non-sandwich control conditions) are deparaffinized, H&E stained, and imaged. Next, the tissue samples are hematoxylin-destained with three HCl solution washes. The sections are then decrosslinked and the tissues are incubated in 1x PBS-Tween for 15 minutes. Individual probe oligonucleotides (e.g., a first probe oligonucleotide, a second probe oligonucleotide) of probe pairs are hybridized to adjacent sequences of an analyte (e.g., an RNA molecule) in the mouse brain tissue. The template ligation probe oligonucleotides are then ligated together, thereby creating a connected probe (e.g., a ligation product). The connected probe (e.g., a ligation product) includes a capture probe binding domain. The probes are designed to hybridize to part of the mouse transcriptome (e.g., using 5000 total probe pairs) or to hybridize to each transcript in the mouse transcriptome. After ligation of the template ligation probe oligonucleotides, the connected probes are released from the tissue using various conditions: (A) sandwich process with RNase in the reagent medium; (B) sandwich process with RNAse + Proteinase K in the reagent medium; or (C) without sandwich methods (i.e., a control). For sandwich conditions A and B, the tissue mounted standard slides are aligned with a GEx slide and permeabilized in the sandwich configuration. For the non-sandwich condition C, the tissue mounted on the GEx slides are permeabilized directly on the GEx array. Following permeabilization, the capture probes are extended, sequencing libraries are prepared and sequenced, and the results are analyzed computationally.

### Example 2. Silencing templated ligation probes targeting SERF1A gene transcripts using blocker oligonucleotides

Experiments were performed to block or silence a templated ligation probe pair targeting *SERF1A* gene transcripts within a pool of templated ligation probes (having about 20,000 templated ligation probe pairs in total). A human brain tissue sample, known for high expression levels of *SERF1* (e.g., *SERF1A* and *SERF1B*) genes, was used for templated ligation detection. Two antisense oligonucleotides (acting as the first and second blocker oligonucleotides as described herein) were designed, which included sequences substantially complementary to the both the first and second probes targeting *SERF1A* gene transcripts, respectively. Locked nucleic acids (LNAs) were also introduced into both antisense oligonucleotides. Both the unmodified and the LNA-modified antisense oligonucleotides were diluted to 100 µM.

Three conditions were tested in a randomized experiment. In the first condition, 5 µl each of the two unmodified antisense oligonucleotides were added to the sample concurrently with the pool of templated ligation probes, followed by probe hybridization and subsequent templated ligation steps. In the second condition, 5 µl of one unmodified antisense oligonucleotide and 5 µl of one LNA-modified antisense oligonucleotide were added to the sample concurrently with the pool of templated ligation probes, followed by probe hybridization and subsequent templated ligation steps. In the third condition, 5 µl each of the two LNA-modified antisense oligonucleotides were added to the sample concurrently with the pool of templated ligation probes, and the hybridization buffer was heated to 100°C then gradually cooled. Probe hybridization was performed, followed by subsequent templated ligation steps.

As shown in **FIGs. 18A-18D****,** the templated-ligation-based spatial gene expression analysis results showed that when the unmodified antisense oligonucleotides (the first condition; **FIG. 18B**) or the combination of unmodified plus LNA-modified antisense oligonucleotides (the second condition; **FIG. 18D**) were applied to the samples, the expression level of detected *SERF1A* gene transcripts dramatically decreased as compared to that of control samples (two replicates, **FIGs. 18A** **and** **18C**). Samples used in the two control replicates were not treated with any antisense oligonucleotides.

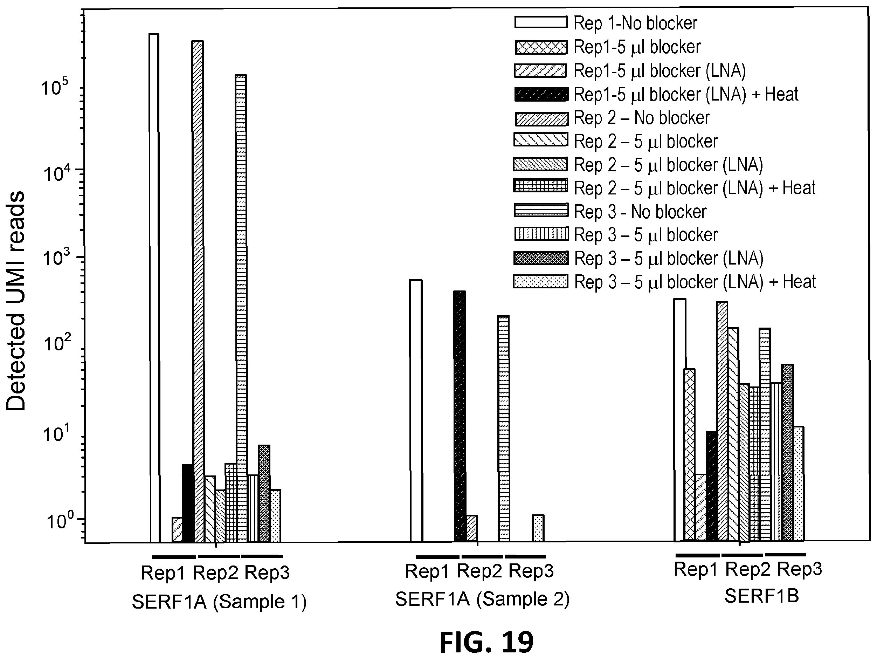

Silencing of the templated ligation probe pair targeting *SERF1A* gene transcripts were further confirmed using two human brain tissue samples, and the UMI reads of *SERF1A* gene were detected in triplicate for each sample. As shown in **FIG. 19****,** each sample was either (1) untreated (shown as "No blocker"); (2) treated with 5 µl each of the two unmodified antisense oligonucleotides (the first condition; shown as "5 µl blocker"); (3) treated with a combination of 5 µl unmodified antisense oligonucleotide and 5 µl LNA-modified antisense oligonucleotide (the second condition; shown as "5 µl blocker (LNA)"); or (4) treated with 5 µl each of the two LNA-modified antisense oligonucleotides, followed by heating and gradual cooling (the third condition; shown as "5 µl blocker (LNA) + Heat"). Consistent results were obtained as compared to **FIGs. 18A-18D****,** showing that the number of UMI reads of *SERF1A* gene was significantly decreased when the antisense oligonucleotides were applied. The results indicate that the antisense oligonucleotides significantly silenced their target templated ligation probes within the templated ligation probe pool, thereby reducing hybridization of the *SERF1A* templated ligation probes to the *SERF1A* target transcript and thus the UMI reads of *SERF1A.* The UMI reads of *SERF1B* were also determined using the same samples. As shown in **FIG. 19****,** the number of UMI reads related to *SERF1B* e was not altered as significantly as compared to that of *SERF1A.* As such, the template ligation probe pair targeting *SERF1B* gene transcripts was not silenced, indicating that the antisense oligonucleotides used herein were specific to the template ligation probes targeting the *SERF1A* transcript in the probe pool.

Additional spatial analysis parameters were determined using the untreated sample (shown as "Control") or samples treated under the three conditions (i.e., the first condition, shown as "5 µl''; the second condition, shown as "5 µl LNA"; and the third condition, shown as "5 µl LNA + Heat").

The library metrics of the templated-ligation-based spatial gene expression results for *SERF1A* was compared between different treatment groups. As shown in **FIG. 20****,** the third condition dramatically decreased the fraction of targeted reads usable (first row) and the fraction of raw reads on target and unambiguously mapped (second row); and dramatically increased the fraction of chimeric reads (third row) and the fraction of chimeric reads under the tissue (fourth row) as compared to the corresponding results of the untreated sample or samples treated under other conditions. The results indicate that the heating and cooling procedures of the hybridization buffer may have contributed to an increased chimera formation (e.g., a first probe from one probe pair and a second probe from a second probe pair hybridizing to adjacent sequences such that ligation occurs) under the tissue.

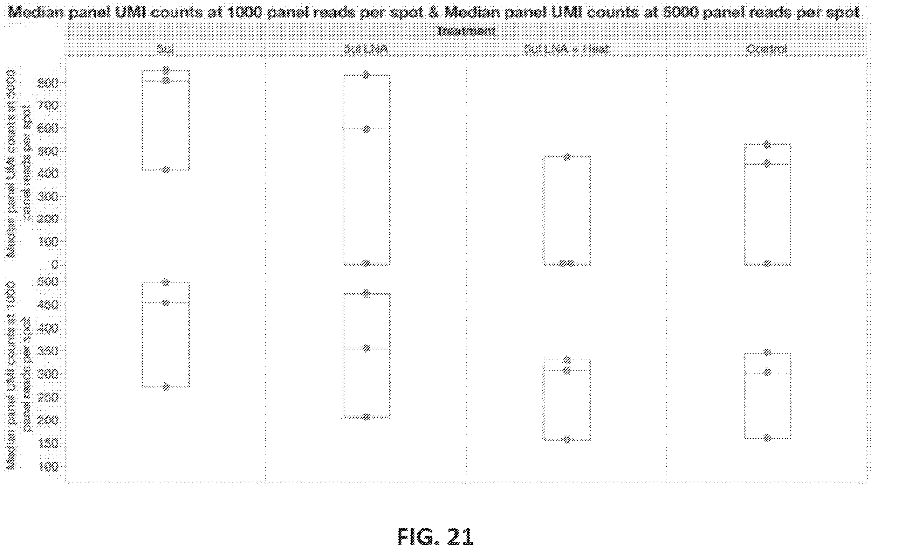

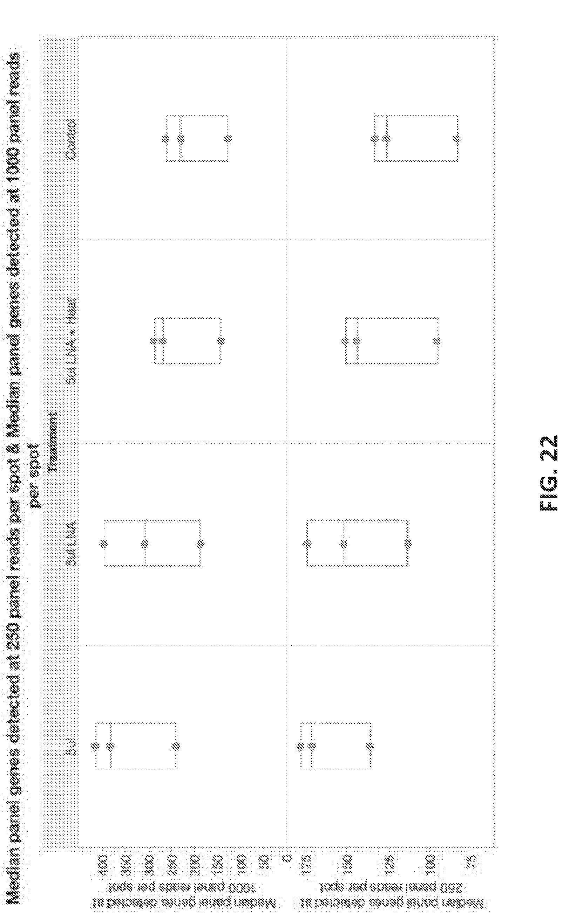

The sensitivity of the templated-ligation-based spatial gene expression results for *SERF1A* was compared between groups. As shown in **FIG. 21****,** median panel UMI counts at 1000 or 5000 panel reads per spot of samples treated by the first condition and the second condition significantly increased as compared to those of the untreated sample or the sample treated under the third condition. The results indicate that the antisense oligonucleotides, with or without LNA modifications, can increase the sensitivity of templated-ligation-based spatial gene expression analysis. The number of genes detected was also compared. As shown in **FIG. 22****,** median panel genes detected at 250 or 1000 panel reads per spot of samples treated by the first condition and the second condition significantly increased as compared to those of the untreated sample or the sample treated by the third condition. The results indicate that the antisense oligonucleotides, with or without LNA modifications, can increase the number of detected genes of templated-ligation-based spatial gene expression analysis. Further, the above results showed that the unmodified antisense oligonucleotide treatment (the first condition) showed the highest sensitivity and highest average number of detected genes.

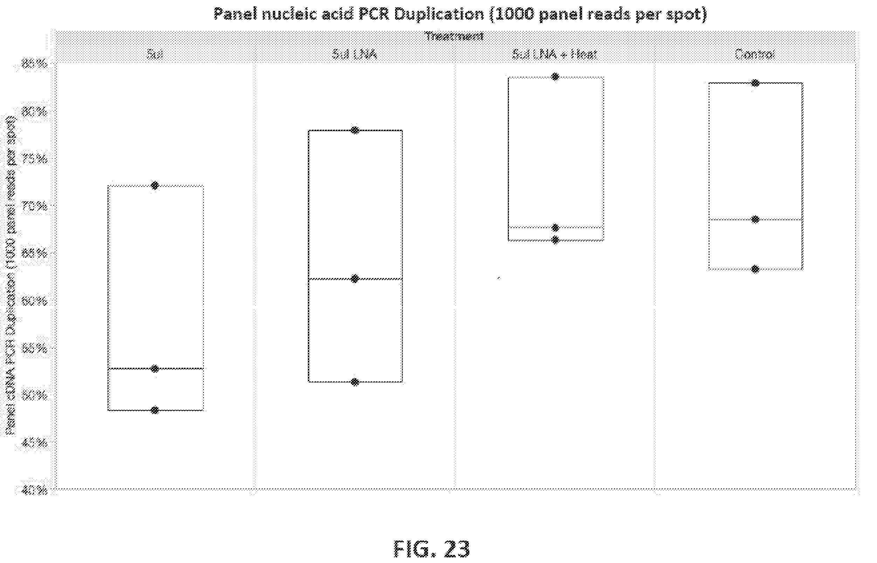

The sequence saturation of the templated-ligation-based spatial gene expression results for *SERF1A* was compared between groups. As shown in **FIG. 23****,** the percentage of panel nucleic acid PCR duplication (1000 panel reads per spot) of the sample treated under the first condition and the second condition was much lower as compared to that of the sample treated by the third condition or the untreated sample. The results indicate that addition of the antisense oligonucleotides, with or without LNA modifications, can decrease the degree of sequence saturation of templated-ligation-based spatial gene expression analysis, which allows for more gene transcripts to be detected. Further, it is noted that the unmodified antisense oligonucleotide treatment (the first condition) showed the lowest degree of sequence saturation.

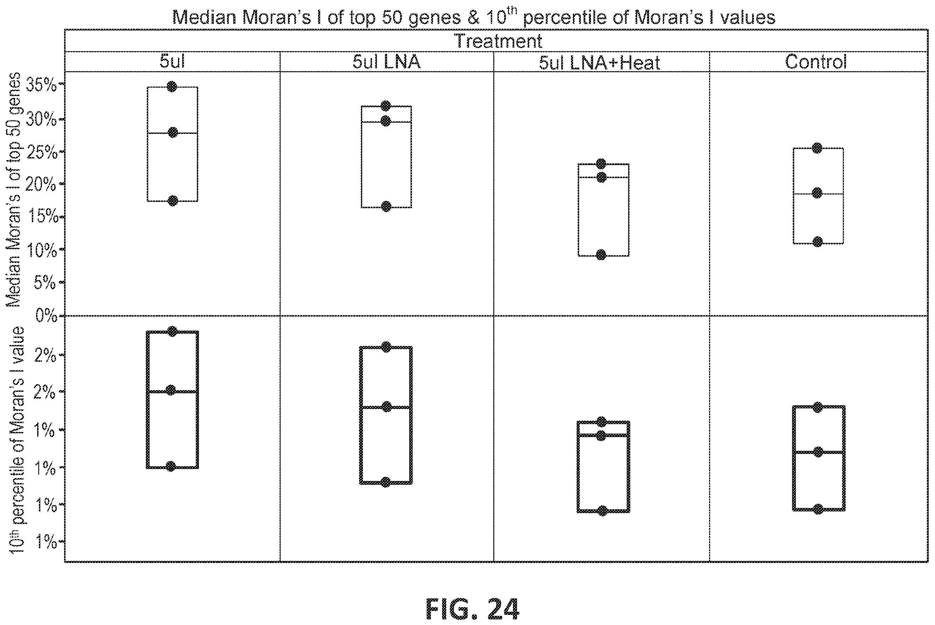

Finally, the spatiality (or diversity) of the templated-ligation-based spatial gene expression results for *SERF1A* were compared between groups. As shown in **FIG. 24****,** median Moran's I of top 50 genes and 10^{th} percentage of Moran's I values of samples of samples treated by the first condition and the second condition were significantly increased as compared to those of the untreated sample or the sample treated by the third condition. The results indicate that the antisense oligonucleotides, with or without LNA modifications, can increase the spatiality (or diversity) of templated-ligation-based spatial gene expression analysis, which allows for more gene transcripts to be detected.

Overall, spatial analysis data indicate that unmodified antisense oligonucleotides can be used to specifically silence templated ligation probe pairs targeting a particular gene transcript, as evidenced when practicing the method using *SERF1A* gene transcripts as a target analyte in a human brain tissue sample.

## Claims

1. A method for decreasing capture of one or more analytes in a tissue sample for spatial analysis comprising:
(a) providing an array comprising a plurality of capture probes, wherein a capture probe of the plurality of capture probes comprises: (i) a spatial barcode and (ii) a capture domain;
(b) contacting the tissue sample comprising a plurality of analytes, wherein the plurality of analytes comprises the one or more analytes for decreased capture and an analyte, with:
(i) a plurality of probe pairs, each probe pair comprising a first probe and a second probe, wherein the first probe and the second probe each comprises sequences that are substantially complementary to sequences of either the analyte or the one or more analytes for decreased capture, wherein the second probe comprises a capture probe capture domain which comprises a sequence that is complementary to all or a portion of the capture domain of the capture probe; and
(ii) a first blocker oligonucleotide, wherein the first blocker oligonucleotide is substantially complementary to the sequence of the first probe or the second probe in a probe pair targeting the one or more analytes for decreased capture;
(c) hybridizing:
(i) the plurality of probe pairs to the plurality of analytes, wherein the first probe and the second probe are hybridized to the analyte; and
(ii) the first blocker oligonucleotide to the sequence of the first probe or the second probe targeting the one or more analytes for decreased capture;
(d) ligating the hybridized first probe and the second probe, thereby generating a ligation product; and
(e) capturing the ligation product by the capture domain on the array,
wherein the capture of the ligation product of the one or more analytes for decreased capture is decreased compared to the capture of a ligation product where no blocker oligonucleotide is present.

2. A method for increasing nucleic acid diversity in spatial transcriptomic analysis of a tissue sample comprising:
(a) providing an array comprising a plurality of capture probes, wherein a capture probe of the plurality of capture probes comprises: (i) a spatial barcode and (ii) a capture domain;
(b) contacting the tissue sample comprising a plurality of nucleic acids, wherein the plurality of nucleic acids comprises one or more nucleic acids for decreased capture and a nucleic acid, with:
(i) a plurality of probe pairs, each probe pair comprising a first probe and a second probe, wherein the first probe and the second probe each comprises sequences that are substantially complementary to sequences of either the nucleic acid or the one or more nucleic acids for decreased capture, wherein the second probe comprises a capture probe capture domain which comprises a sequence that is complementary to all or a portion of the capture domain of the capture probe; and
(ii) a first blocker oligonucleotide, wherein the first blocker oligonucleotide is substantially complementary to the sequence of the first probe or the second probe in a probe pair targeting the one or more nucleic acids for decreased capture;
(c) hybridizing:
(i) the plurality of probe pairs to the plurality of nucleic acids, wherein the first probe and the second probe are hybridized to the nucleic acid; and
(ii) the first blocker oligonucleotide to the sequence of the first probe or the second probe targeting the one or more nucleic acids for decreased capture;
(d) ligating the hybridized first probe and the second probe, thereby generating a ligation product; and
(e) capturing the ligation product by the capture domain on the array,
wherein the nucleic acid diversity in spatial transcriptomics analysis is increased relative to the nucleic acid diversity in a spatial transcriptomics assay where no blocker oligonucleotide is present.

3. A method for increasing detection of one or more rare or low-expressing nucleic acids in a tissue sample for spatial analysis comprising:
(a) providing an array comprising a plurality of capture probes, wherein a capture probe of the plurality of capture probes comprises: (i) a spatial barcode and (ii) a capture domain;
(b) contacting the tissue sample comprising a plurality of nucleic acids, wherein the plurality of nucleic acids comprises one or more nucleic acids for decreased capture and a rare or low-expressing nucleic acid, with:
(i) a plurality of probe pairs, each probe pair comprising a first probe and a second probe, wherein the first probe and the second probe each comprises sequences that are substantially complementary to sequences of either the rare or low-expressing nucleic acid or the one or more nucleic acids for decreased capture, wherein the second probe comprises a capture probe capture domain which comprises a sequence that is complementary to all or a portion of the capture domain of the capture probe; and
(ii) a first blocker oligonucleotide, wherein the first blocker oligonucleotide is substantially complementary to the sequence of the first probe or the second probe in a probe pair targeting the one or more nucleic acids for decreased capture;
(c) hybridizing:
(i) the plurality of probe pairs to the plurality of nucleic acids, wherein the first probe and the second probe are hybridized to the rare or low-expressing nucleic acid; and
(ii) the first blocker oligonucleotide to the sequence of the first probe or the second probe targeting the one or more nucleic acids for decreased capture;
(d) ligating the hybridized first probe and the second probe, thereby generating a ligation product; and
(e) capturing the ligation product by the capture domain on the array,
wherein the detection of one or more rare or low-expressing nucleic acids is increased compared to the detection of one or more rare or low-expressing nucleic acids in a reference tissue sample where no blocker oligonucleotide is present.

4. The method of any one of the preceding claims, further comprising:
contacting the tissue sample with a second blocker oligonucleotide, wherein the second blocker oligonucleotide is substantially complementary to the sequence of the first probe or the second probe targeting the one or more analytes or nucleic acids for decreased capture; and
hybridizing the second blocker oligonucleotide to the sequence of the first probe or the second probe targeting the one or more analytes or nucleic acids for decreased capture, optionally wherein the first blocker oligonucleotide is substantially complementary to the sequence of the first probe targeting the one or more analytes or nucleic acids for decreased capture, and the second blocker oligonucleotide is substantially complementary to the sequence of the second probe targeting the one or more analytes or nucleic acids for decreased capture, optionally wherein the first blocker oligonucleotide and/or the second blocker oligonucleotide block the first probe and/or the second probe targeting the one or more analytes or nucleic acids for decreased capture from hybridizing to the one or more analytes or nucleic acids for decreased capture.

5. The method of any one of the preceding claims, wherein the one or more analytes or nucleic acids for decreased capture comprises a highly expressed gene transcript, optionally wherein the one or more analytes or nucleic acids for decreased capture comprises an RNA, optionally a messenger RNA (mRNA), ribosomal RNA (rRNA), mitochondrial RNA (mtRNA), and/or transfer RNA (tRNA), or fragment thereof; or a housekeeping analyte, optionally a glyceraldehyde-3-phosphate dehydrogenase (GAPDH), a TATA-binding protein (TBP), and/or a ribosomal protein (RP).

6. The method of any one of the preceding claims, wherein the first probe and the second probe targeting the one or more analytes or nucleic acids for decreased capture are not hybridized to the one or more analytes or nucleic acids for decreased capture, optionally wherein the first probe and the second probe pair targeting the one or more analytes or nucleic acids for decreased capture are not ligated together, optionally wherein the first probe and the second probe targeting the one or more analytes or nucleic acids for decreased capture are not captured by the capture domain on the array.

7. The method of any one of claims 4-6, wherein:
(i) the first blocker oligonucleotide and/or the second blocker oligonucleotide each does not comprise a phosphorylated nucleotide at its 5' end and/or does not comprise one or more ribonucleic acid bases at its 3' end;
(ii) the first blocker oligonucleotide and/or the second blocker oligonucleotide each does not comprise a sequence that is substantially complementary to the capture probe capture domain;
(iii) the first blocker oligonucleotide is substantially complementary to the sequence of the first probe targeting the one or more analytes or nucleic acids for decreased capture that is complementary to the one or more analytes or nucleic acids for decreased capture, and/or the second blocker oligonucleotide is substantially complementary to the sequence of the second probe targeting the one or more analytes or nucleic acids for decreased capture that is complementary to the one or more analytes or nucleic acids for decreased capture;
(iv) the first blocker oligonucleotide hybridizes to one or more portions of the sequence of the first probe targeting the one or more analytes or nucleic acids for decreased capture, and/or the second blocker oligonucleotide hybridizes to one or more portions of the sequence of the second probe targeting the one or more analytes or nucleic acids for decreased capture; and/or
(v) the first blocker oligonucleotide hybridizes to the sequence of the first probe targeting the one or more analytes or nucleic acids for decreased capture that is complementary to the one or more analytes or nucleic acids for decreased capture, and/or the second blocker oligonucleotide hybridizes to the sequence of the second probe targeting the one or more analytes or nucleic acids for decreased capture that is complementary to the one or more analytes or nucleic acids for decreased capture.

8. The method of any one of claims 4-7, wherein the first blocker oligonucleotide and/or the second blocker oligonucleotide comprise one or more modifications, optionally wherein the one or more modifications comprise:
(i) biotin, amino modifiers, azide, cholesteryl, alkynes, and/or thiol modifiers;
(ii) a fluorophore; and/or
(iii) a 3'-3'-inverted thymine.

9. The method of claim 8, wherein the first blocker oligonucleotide and/or the second blocker oligonucleotide comprise a non-natural nucleic acid, optionally wherein the non-natural nucleic acid is a locked nucleic acid (LNA).

10. The method of any one of the preceding claims, wherein the first probe and the second probe:
(i) are substantially complementary to adjacent sequences of the analyte, the nucleic acid, or the rare or low-expressing nucleic acid; or
(ii) hybridize to sequences that are not adjacent to each other on the analyte, the nucleic acid, or the rare or low-expressing nucleic acid, wherein the first probe is extended with a DNA polymerase, thereby filling in a gap between the first probe and the second probe and generating an extended first probe.

11. The method of any one of the preceding claims, wherein the first probe comprises at least two ribonucleic acid bases at the 3' end, and the second probe comprises a phosphorylated nucleotide at the 5' end.

12. The method of any one of the preceding claims, wherein generating the ligation product comprises ligating (i) the first probe to the second probe or (ii) the extended first probe to the second probe using enzymatic ligation or chemical ligation, wherein the enzymatic ligation utilizes a ligase, optionally wherein the ligase is a T4 RNA ligase (Rnl2), a PBCV-1 ligase, a Chlorella virus DNA ligase, a single stranded DNA ligase, or a T4 DNA ligase.

13. The method of any one of the preceding claims, wherein the second probe comprises a pre-adenylated phosphate group at its 5' end, and the first probe comprises at least two ribonucleic acid bases at the 3' end, optionally wherein the ligating the hybridized first probe and the second probe comprises ligating a 3' end of the first probe to the 5' end of the second probe using a ligase that does not require adenosine triphosphate for ligase activity, optionally wherein the ligase is a thermostable 5' AppDNA/RNA Ligase, a truncated T4 RNA Ligase 2, a truncated T4 RNA Ligase 2 K227Q, a truncated T4 RNA Ligase 2 KQ, a PBCV-1 DNA ligase, or a Chlorella Virus DNA Ligase.

14. The method of any one of claims 1-13, further comprising providing a capture probe capture domain blocking moiety that interacts with the capture probe capture domain, optionally further comprising releasing the capture probe capture domain blocking moiety from the capture probe capture domain prior to (e), optionally wherein the capture probe capture domain comprises a poly-adenylated (poly(A)) sequence or a complement thereof, optionally wherein the capture probe capture domain blocking moiety comprises a poly-uridine sequence, a poly-thymidine sequence, or both, optionally wherein releasing the poly-uridine sequence from the poly(A) sequence comprises denaturing the ligation product or contacting the ligation product with an endonuclease, exonuclease and/or ribonuclease.

15. The method of any one of claims 7-14, wherein the capture probe capture domain comprises a sequence that is complementary to all or a portion of the capture domain of the capture probe, optionally wherein the capture probe capture domain comprises a degenerate sequence.

16. The method of any one of the preceding claims, wherein the first probe and/or the second probe are DNA probes, optionally wherein the capture probe capture domain blocking moiety is a DNA probe.

17. The method of any one of the preceding claims, further comprising, prior to (e), releasing the ligation product from the analyte, the nucleic acid, or the rare or low-expressing nucleic acid, optionally wherein the releasing of (i) the ligation product from the analyte, the nucleic acid, or the rare or low-expressing nucleic acid, or (ii) the capture probe capture domain blocking moiety from the capture probe capture domain, comprises contacting the ligated probes with an endoribonuclease, optionally wherein the endoribonuclease is RNase H RNase A, RNase C, or RNase I, optionally wherein the RNase H is RNase H1, RNase H2, or RNase H1 and RNase H2),

18. The method of any one of the preceding claims, wherein the tissue sample is a formalin-fixed, paraffin-embedded (FFPE) tissue sample, a fresh or a frozen tissue sample, optionally wherein the FFPE tissue sample is decrosslinked, optionally wherein the tissue sample was previously stained, optionally using immunofluorescence, immunohistochemistry, or hematoxylin and eosin (H&E).

19. The method of any one of the preceding claims, wherein the method further comprises contacting the tissue sample with a permeabilization agent, wherein the permeabilization agent is an organic solvent, a detergent, or an enzyme, optionally wherein the permeabilization agent is an endopeptidase, a protease sodium dodecyl sulfate (SDS), polyethylene glycol tert-octylphenyl ether, polysorbate 80, polysorbate 20, N-lauroylsarcosine sodium salt solution, saponin, a nonionic detergent, or Tween-20^{™}, optionally wherein the endopeptidase is pepsin or proteinase K.

20. The method of any one of the preceding claims, further comprising: determining (i) all or part of the sequence of the ligation product bound to the capture domain, or a complement thereof, and (ii) the sequence of the spatial barcode, or a complement thereof, optionally wherein the determining comprises amplifying all or part of the ligation product specifically bound to the capture domain, thereby generating an amplifying product, optionally wherein the amplifying product comprises (i) all or part of the sequence of the ligation product specifically bound to the capture domain, or a complement thereof, and (ii) the sequence of the spatial barcode, or a complement thereof, optionally wherein the determining comprises sequencing (i) all or part of the sequence of the ligation product, or a complement thereof, and (ii) the sequence of the spatial barcode, or a complement thereof, optionally wherein the sequencing comprises *in situ* sequencing, Sanger sequencing methods, next-generation sequencing methods, or nanopore sequencing.

## Patentansprüche

1. Verfahren für ein Verringern des Erfassens von einem oder mehreren Analyten in einer Gewebeprobe für die räumliche Analyse, umfassend:
(a) Bereitstellen einer Anordnung umfassend eine Mehrzahl von Erfassungssonden, wobei eine Erfassungssonde der Mehrzahl von Erfassungssonden umfasst: (i) einen räumlichen Barcode und (ii) eine Erfassungsdomäne;
(b) Inkontaktbringen der Gewebeprobe, die eine Mehrzahl von Analyten umfasst, wobei die Mehrzahl von Analyten den einen oder die mehreren Analyten für eine verringerte Erfassung und einen Analyten umfasst, mit:
(i) einer Mehrzahl von Sondenpaaren, wobei jedes Sondenpaar eine erste Sonde und eine zweite Sonde umfasst, wobei die erste Sonde und die zweite Sonde jeweils Sequenzen umfassen, die im Wesentlichen komplementär zu Sequenzen entweder des Analyten oder des einen oder der mehreren Analyten für eine verringerte Erfassung sind, wobei die zweite Sonde eine Erfassungssonden-Erfassungsdomäne umfasst, die eine Sequenz umfasst, die komplementär zu der gesamten oder einem Abschnitt der Erfassungsdomäne der Erfassungssonde ist; und
(ii) einem ersten Blocker-Oligonukleotid, wobei das erste Blocker-Oligonukleotid im Wesentlichen komplementär zu der Sequenz der ersten Sonde oder der zweiten Sonde in einem Sondenpaar ist, das auf den einen oder die mehreren Analyten für eine verringerte Erfassung abzielt;
(c) Hybridisieren:
(i) der Mehrzahl der Sondenpaare an die Mehrzahl der Analyten, wobei die erste Sonde und die zweite Sonde an den Analyten hybridisiert sind; und
(ii) des ersten Blocker-Oligonukleotids an die Sequenz der ersten Sonde oder der zweiten Sonde, die auf den einen oder die mehreren Analyten für eine verringerte Erfassung abzielen;
(d) Ligieren der hybridisierten ersten Sonde und der zweiten Sonde, wodurch ein Ligationsprodukt erzeugt wird; und
(e) Erfassen des Ligationsprodukts durch die Erfassungsdomäne auf der Anordnung,
wobei das Erfassen des Ligationsprodukts des einen oder der mehreren Analyten für eine verringerte Erfassung im Vergleich zu einem Erfassen eines Ligationsprodukts, bei dem kein Blocker-Oligonukleotid vorhanden ist, verringert ist.

2. Verfahren für ein Erhöhen der Nukleinsäurediversität bei der räumlichen Transkriptom-Analyse einer Gewebeprobe, umfassend:
(a) Bereitstellen einer Anordnung umfassend eine Mehrzahl von Erfassungssonden, wobei eine Erfassungssonde der Mehrzahl von Erfassungssonden umfasst: (i) einen räumlichen Barcode und (ii) eine Erfassungsdomäne;
(b) Inkontaktbringen der Gewebeprobe, die eine Mehrzahl von Nukleinsäuren umfasst, wobei die Mehrzahl von Nukleinsäuren eine oder mehrere Nukleinsäuren für ein verringertes Erfassen und eine Nukleinsäure umfasst, mit:
(i) einer Mehrzahl von Sondenpaaren, wobei jedes Sondenpaar eine erste Sonde und eine zweite Sonde umfasst, wobei die erste Sonde und die zweite Sonde jeweils Sequenzen umfassen, die im Wesentlichen komplementär zu Sequenzen entweder der Nukleinsäure oder der einen oder den mehreren Nukleinsäuren für eine verringerte Erfassung sind, wobei die zweite Sonde eine Erfassungssonden-Erfassungsdomäne umfasst, die eine Sequenz umfasst, die komplementär zu der gesamten oder einem Abschnitt der Erfassungsdomäne der Erfassungssonde ist; und
(ii) einem ersten Blocker-Oligonukleotid, wobei das erste Blocker-Oligonukleotid im Wesentlichen komplementär zu der Sequenz der ersten Sonde oder der zweiten Sonde in einem Sondenpaar ist, das auf die eine oder die mehreren Nukleinsäuren für eine verringerte Erfassung abzielt;
(c) Hybridisieren:
(i) der Mehrzahl der Sondenpaare an die Mehrzahl der Nukleinsäuren, wobei die erste Sonde und die zweite Sonde an die Nukleinsäure hybridisiert sind; und
(ii) des ersten Blocker-Oligonukleotids an die Sequenz der ersten Sonde oder der zweiten Sonde, die auf die eine oder die mehreren Nukleinsäuren für eine verringerte Erfassung abzielen;
(d) Ligieren der hybridisierten ersten Sonde und der zweiten Sonde, wodurch ein Ligationsprodukt erzeugt wird; und
(e) Erfassen des Ligationsprodukts durch die Erfassungsdomäne auf der Anordnung,
wobei die Nukleinsäurediversität bei der räumlichen Transkriptom-Analyse im Vergleich zu der Nukleinsäurediversität in einem räumlichen Transkriptom-Assay, bei dem kein Blocker-Oligonukleotid vorhanden ist, erhöht ist.

3. Verfahren für ein Erhöhen eines Nachweises einer oder mehrerer selten oder niedrig exprimierender Nukleinsäuren in einer Gewebeprobe für eine räumliche Analyse, umfassend:
(a) Bereitstellen einer Anordnung umfassend eine Mehrzahl von Erfassungssonden, wobei eine Erfassungssonde der Mehrzahl von Erfassungssonden umfasst: (i) einen räumlichen Barcode und (ii) eine Erfassungsdomäne;
(b) Inkontaktbringen der Gewebeprobe, die eine Mehrzahl von Nukleinsäuren umfasst, wobei die Mehrzahl von Nukleinsäuren eine oder mehrere Nukleinsäuren für ein verringertes Erfassen und eine selten oder niedrig exprimierende Nukleinsäure umfasst, mit:
(i) einer Mehrzahl von Sondenpaaren, wobei jedes Sondenpaar eine erste Sonde und eine zweite Sonde umfasst, wobei die erste Sonde und die zweite Sonde jeweils Sequenzen umfassen, die im Wesentlichen komplementär zu Sequenzen entweder der selten oder niedrig exprimierenden Nukleinsäure oder der einen oder den mehreren Nukleinsäuren für eine verringerte Erfassung sind, wobei die zweite Sonde eine Erfassungssonden-Erfassungsdomäne umfasst, die eine Sequenz umfasst, die komplementär zu der gesamten oder einem Abschnitt der Erfassungsdomäne der Erfassungssonde ist; und
(ii) einem ersten Blocker-Oligonukleotid, wobei das erste Blocker-Oligonukleotid im Wesentlichen komplementär zu der Sequenz der ersten Sonde oder der zweiten Sonde in einem Sondenpaar ist, das auf die eine oder die mehreren Nukleinsäuren für eine verringerte Erfassung abzielt;
(c) Hybridisieren:
(i) der Mehrzahl der Sondenpaare an die Mehrzahl der Nukleinsäuren, wobei die erste Sonde und die zweite Sonde an die selten oder niedrig exprimierende Nukleinsäure hybridisiert sind; und
(ii) des ersten Blocker-Oligonukleotids an die Sequenz der ersten Sonde oder der zweiten Sonde, die auf die eine oder die mehreren Nukleinsäuren für eine verringerte Erfassung abzielen;
(d) Ligieren der hybridisierten ersten Sonde und der zweiten Sonde, wodurch ein Ligationsprodukt erzeugt wird; und
(e) Erfassen des Ligationsprodukts durch die Erfassungsdomäne auf der Anordnung,
wobei der Nachweis einer oder mehrerer selten oder niedrig exprimierender Nukleinsäuren im Vergleich zu dem Nachweis einer oder mehrerer selten oder niedrig exprimierender Nukleinsäuren in einer Referenzgewebeprobe, bei der kein Blocker-Oligonukleotid vorhanden ist, erhöht ist.

4. Verfahren nach einem der vorhergehenden Ansprüche, ferner umfassend:
Inkontaktbringen der Gewebeprobe mit einem zweiten Blocker-Oligonukleotid, wobei das zweite Blocker-Oligonukleotid im Wesentlichen komplementär zu der Sequenz der ersten Sonde oder der zweiten Sonde ist, die auf den/die eine(n) oder die mehreren Analyten oder Nukleinsäuren für eine verringerte Erfassung abzielt; und
Hybridisieren des zweiten Blocker-Oligonukleotids an die Sequenz der ersten Sonde oder der zweiten Sonde, die auf den/die eine(n) oder die mehreren Analyten oder Nukleinsäuren für eine verringerte Erfassung abzielt, optional, wobei das erste Blocker-Oligonukleotid im Wesentlichen komplementär zu der Sequenz der ersten Sonde ist, die auf den/die eine(n) oder die mehreren Analyten oder Nukleinsäuren für eine verringerte Erfassung abzielt, und das zweite Blocker-Oligonukleotid im Wesentlichen komplementär zu der Sequenz der zweiten Sonde ist, die auf den/die eine(n) oder die mehreren Analyten oder Nukleinsäuren für eine verringerte Erfassung abzielt, optional, wobei das erste Blocker-Oligonukleotid und/oder das zweite Blocker-Oligonukleotid die erste Sonde und/oder die zweite Sonde, die auf den/die eine(n) oder die mehreren Analyten oder Nukleinsäuren für eine verringerte Erfassung abzielt, daran hindert, an den/die eine(n) oder die mehreren Analyten oder Nukleinsäuren für eine verringerte Erfassung zu hybridisieren.

5. Verfahren nach einem der vorhergehenden Ansprüche, wobei der/die eine oder die mehreren Analyten oder Nukleinsäuren für eine verringerte Erfassung ein hochexprimiertes Gentranskript umfassen, optional wobei der/die eine oder die mehreren Analyten oder Nukleinsäuren für ein verringertes Erfassen eine RNA, optional eine Boten-RNA (mRNA), ribosomale RNA (rRNA), mitochondriale RNA (mtRNA) und/oder Transfer-RNA (tRNA), oder ein Fragment davon umfassen; oder einen Housekeeping-Analyten, optional eine Glyceraldehyd-3-phosphat-Dehydrogenase (GAPDH), ein TATAbindendes Protein (TBP) und/oder ein ribosomales Protein (RP) umfassen.

6. Verfahren nach einem der vorhergehenden Ansprüche, wobei die erste Sonde und die zweite Sonde, die auf den/die eine(n) oder die mehreren Analyten oder Nukleinsäuren für eine verringerte Erfassung abzielen, nicht an den/die eine(n) oder die mehreren Analyten oder Nukleinsäuren für eine verringerte Erfassung hybridisiert sind, optional, wobei die erste Sonde und das zweite Sondenpaar, die auf den/die eine(n)oder die mehreren Analyten oder Nukleinsäuren für eine verringerte Erfassung abzielen, nicht miteinander ligiert sind, optional, wobei die erste Sonde und die zweite Sonde, die auf den/die eine(n) oder die mehreren Analyten oder Nukleinsäuren für eine verringerte Erfassung abzielen, nicht von der Erfassungsdomäne auf der Anordnung erfasst werden.

7. Verfahren nach einem der Ansprüche 4-6, wobei:
(i) das erste Blocker-Oligonukleotid und/oder das zweite Blocker-Oligonukleotid jeweils kein phosphoryliertes Nukleotid an seinem 5'-Ende umfasst und/oder keine eine oder mehrere Ribonukleinsäurebasen an seinem 3'-Ende umfasst;
(ii) das erste Blocker-Oligonukleotid und/oder das zweite Blocker-Oligonukleotid jeweils keine Sequenz umfasst, die im Wesentlichen komplementär zu der Erfassungssonden-Erfassungsdomäne ist;
(iii) das erste Blocker-Oligonukleotid im Wesentlichen komplementär zu der Sequenz der ersten Sonde ist, die auf den/die eine(n) oder die mehreren Analyten oder Nukleinsäuren für eine verringerte Erfassung abzielt, die komplementär zu dem/der einen oder den mehreren Analyten oder Nukleinsäuren für eine verringerte Erfassung ist, und/oder das zweite Blocker-Oligonukleotid im Wesentlichen komplementär zu der Sequenz der zweiten Sonde ist, die auf den/die eine(n) oder die mehreren Analyten oder Nukleinsäuren für eine verringerte Erfassung abzielt, die komplementär zu dem/der einen oder mehreren Analyten oder Nukleinsäuren für eine verringerte Erfassung ist;
(iv) das erste Blocker-Oligonukleotid an einen oder mehrere Abschnitte der Sequenz der ersten Sonde hybridisiert, die auf den/die eine(n) oder die mehreren Analyten oder Nukleinsäuren für eine verringerte Erfassung abzielt, und/oder das zweite Blocker-Oligonukleotid an einen oder mehrere Abschnitte der Sequenz der zweiten Sonde hybridisiert, die auf den/die eine(n) oder die mehreren Analyten oder Nukleinsäuren für eine verringerte Erfassung abzielt; und/oder
(v) das erste Blocker-Oligonukleotid an die Sequenz der ersten Sonde hybridisiert, die auf den/die eine(n) oder die mehreren Analyten oder Nukleinsäuren für eine verringerte Erfassung abzielt, die komplementär zu dem/der einen oder den mehreren Analyten oder Nukleinsäuren für eine verringerte Erfassung ist, und/oder das zweite Blocker-Oligonukleotid an die Sequenz der zweiten Sonde hybridisiert, die auf den/die eine(n) oder die mehreren Analyten oder Nukleinsäuren für eine verringerte Erfassung abzielt, die komplementär zu dem/der einen oder mehreren Analyten oder Nukleinsäuren für eine verringerte Erfassung ist.

8. Verfahren nach einem der Ansprüche 4-7, wobei das erste Blocker-Oligonukleotid und/oder das zweite Blocker-Oligonukleotid eine oder mehrere Modifikationen umfassen, optional, wobei die eine oder mehreren Modifikationen umfassen:
(i) Biotin, Aminomodifikatoren, Azid, Cholesteryl, Alkine, und/oder Thiolmodifikatoren;
(ii) ein Fluorophor; und/oder
(iii) ein 3'-3'-invertiertes Thymin.

9. Verfahren nach Anspruch 8, wobei das erste Blocker-Oligonukleotid und/oder das zweite Blocker-Oligonukleotid eine nicht natürliche Nukleinsäure umfasst, optional, wobei die nicht natürliche Nukleinsäure eine verbrückte Nukleinsäure (LNA) ist.

10. Verfahren nach einem der vorhergehenden Ansprüche, wobei die erste Sonde und die zweite Sonde:
(i) im Wesentlichen komplementär zu benachbarten Sequenzen des Analyten, der Nukleinsäure oder der selten oder niedrig exprimierenden Nukleinsäure sind; oder
(ii) an Sequenzen hybridisieren, die auf dem Analyten, der Nukleinsäure oder der selten oder niedrig exprimierenden Nukleinsäure nicht benachbart zueinander sind, wobei die erste Sonde mit einer DNA-Polymerase verlängert wird, wodurch eine Lücke zwischen der ersten Sonde und der zweiten Sonde gefüllt wird und eine verlängerte erste Sonde erzeugt wird.

11. Verfahren nach einem der vorhergehenden Ansprüche, wobei die erste Sonde mindestens zwei Ribonukleinsäurebasen an dem 3'-Ende umfasst, und die zweite Sonde ein phosphoryliertes Nukleotid an dem 5'-Ende umfasst.

12. Verfahren nach einem der vorhergehenden Ansprüche, wobei ein Erzeugen des Ligationsprodukts das Ligieren umfasst (i) der ersten Sonde an die zweite Sonde oder (ii) der verlängerten ersten Sonde an die zweite Sonde unter Verwendung enzymatischer Ligation oder chemischer Ligation, wobei die enzymatische Ligation eine Ligase verwendet, optional wobei die Ligase eine T4-RNA-Ligase (Rnl2), eine PBCV-1-Ligase, eine Chlorella-Virus-DNA-Ligase, eine einzelsträngige DNA-Ligase oder eine T4-DNA-Ligase ist.

13. Verfahren nach einem der vorhergehenden Ansprüche, wobei die zweite Sonde eine voradenylierte Phosphatgruppe an ihrem 5'-Ende umfasst und die erste Sonde mindestens zwei Ribonukleinsäurebasen an dem 3'-Ende umfasst, optional, wobei das Ligieren der hybridisierten ersten Sonde und der zweiten Sonde ein Ligieren eines 3'-Endes der ersten Sonde an das 5'-Ende der zweiten Sonde unter Verwendung einer Ligase umfasst, die kein Adenosintriphosphat für die Ligaseaktivität benötigt, wobei die Ligase optional eine thermostabile 5'-AppDNA/RNA-Ligase, eine trunkierte T4-RNA-Ligase 2, eine trunkierte T4-RNA-Ligase 2 K227Q, eine trunkierte T4-RNA-Ligase 2 KQ, eine PBCV-1-DNA-Ligase oder eine Chlorella-Virus-DNA-Ligase ist.

14. Verfahren nach einem der Ansprüche 1-13, ferner umfassend Bereitstellen einer Erfassungssonden-Erfassungsdomänen-Blockiereinheit, die mit der Erfassungssonden-Erfassungsdomäne interagiert, optional ferner umfassend Freisetzen der Erfassungssonden-Erfassungsdomänen-Blockiereinheit von der Erfassungssonden-Erfassungsdomäne vor (e), wobei die Erfassungssonden-Erfassungsdomäne eine polyadenylierte (Poly(A))-Sequenz oder ein Komplement davon umfasst, optional, wobei die Erfassungssonden-Erfassungsdomänen-Blockiereinheit eine Polyuridin-Sequenz, eine Polythymidin-Sequenz oder beide umfasst, optional, wobei das Freisetzen der Polyuridin-Sequenz von der Poly(A)-Sequenz ein Denaturieren des Ligationsprodukts oder das Inkontaktbringen des Ligationsprodukts mit einer Endonuklease, Exonuklease und/oder Ribonuklease umfasst.

15. Verfahren nach einem der Ansprüche 7-14, wobei die Erfassungssonden-Erfassungsdomäne eine Sequenz umfasst, die komplementär zu der gesamten oder einem Abschnitt der Erfassungsdomäne der Erfassungssonde ist, optional, wobei die Erfassungssonden-Erfassungsdomäne eine degenerierte Sequenz umfasst.

16. Verfahren nach einem der vorhergehenden Ansprüche, wobei die erste Sonde und/oder die zweite Sonde DNA-Sonden sind, optional wobei die Erfassungssonden-Erfassungsdomänen-Blockiereinheit eine DNA-Sonde ist.

17. Verfahren nach einem der vorhergehenden Ansprüche, ferner umfassend vor (e) Freisetzen des Ligationsprodukts aus dem Analyten, der Nukleinsäure oder der selten oder niedrig exprimierenden Nukleinsäure, optional, wobei das Freisetzen von (i) dem Ligationsprodukt aus dem Analyten, der Nukleinsäure oder der selten oder niedrig exprimierenden Nukleinsäure, oder (ii) der Erfassungssonden-Erfassungsdomänen-Blockiereinheit aus der Erfassungssonden-Erfassungsdomäne das Inkontaktbringen der ligierten Sonden mit einer Endoribonuklease umfasst, wobei die Endoribonuklease RNase H, RNase A, RNase C oder RNase I ist, optional wobei die RNase H RNase H1, RNase H2 oder RNase H1 und RNase H2 ist.

18. Verfahren nach einem der vorhergehenden Ansprüche, wobei die Gewebeprobe eine formalinfixierte, paraffineingebettete (FFPE) Gewebeprobe, eine frische oder eine gefrorene Gewebeprobe ist, optional, wobei die FFPE-Gewebeprobe entnetzt ist, optional, wobei die Gewebeprobe zuvor gefärbt wurde, optional durch Verwendung von Immunfluoreszenz, Immunhistochemie oder Hämatoxylin und Eosin (H&E).

19. Verfahren nach einem der vorhergehenden Ansprüche, wobei das Verfahren ferner ein Inkontaktbringen der Gewebeprobe mit einem Permeabilisierungsmittel umfasst, wobei das Permeabilisierungsmittel ein organisches Lösungsmittel, ein Detergens oder ein Enzym ist, wobei das Permeabilisierungsmittel optional eine Endopeptidase, eine Protease, Natriumdodecylsulfat (SDS), Polyethylenglykol-tert-octylphenylether, Polysorbat 80, Polysorbat 20, N-Lauroylsarcosin-Natriumsalzlösung, Saponin, ein nichtionisches Detergens oder Tween-20^{™} ist, optional wobei die Endopeptidase Pepsin oder Proteinase K ist.

20. Verfahren nach einem der vorhergehenden Ansprüche, ferner umfassend: Bestimmen (i) der gesamten oder eines Teils der Sequenz des Ligationsprodukts, das an die Erfassungsdomäne gebunden ist, oder eines Komplements davon, und (ii) der Sequenz des räumlichen Barcodes oder eines Komplements davon, wobei das Bestimmen optional ein Amplifizieren des gesamten oder eines Teils des Ligationsprodukts, das spezifisch an die Erfassungsdomäne gebunden ist, umfasst, wodurch ein Amplifikationsprodukt erzeugt wird, wobei das Amplifikationsprodukt optional (i) die gesamte oder einen Teil der Sequenz des Ligationsprodukts, das spezifisch an die Erfassungsdomäne gebunden ist, umfasst, oder ein Komplement davon und (ii) die Sequenz des räumlichen Barcodes oder ein Komplement davon umfasst, wobei das Bestimmen optional die Sequenzierung (i) der gesamten oder eines Teils der Sequenz des Ligationsprodukts oder eines Komplements davon und (ii) der Sequenz des räumlichen Barcodes oder eines Komplements davon umfasst, wobei die Sequenzierung optional die In-situ-Sequenzierung, Sanger-Sequenzierungsverfahren, Sequenzierungsverfahren der nächsten Generation oder die Nanopore-Sequenzierung umfasst.

## Revendications

1. Procédé pour réduire la capture d'un ou plusieurs analytes dans un échantillon de tissu pour une analyse spatiale comprenant :
(a) la fourniture d'un réseau comprenant une pluralité de sondes de capture, dans lequel une sonde de capture de la pluralité de sondes de capture comprend : (i) un code à barres spatial et (ii) un domaine de capture ;
(b) la mise en contact de l'échantillon de tissu comprenant une pluralité d'analytes, dans lequel la pluralité d'analytes comprend les un ou plusieurs analytes pour une capture réduite et un analyte, avec :
(i) une pluralité de paires de sondes, chaque paire de sondes comprenant une première sonde et une deuxième sonde, dans lequel la première sonde et la deuxième sonde comprennent chacune des séquences qui sont sensiblement complémentaires de séquences soit de l'analyte soit des un ou plusieurs analytes pour une capture réduite, dans lequel la deuxième sonde comprend un domaine de capture de sonde de capture qui comprend une séquence qui est complémentaire de la totalité ou d'une portion du domaine de capture de la sonde de capture ; et
(ii) un premier oligonucléotide bloquant, dans lequel le premier oligonucléotide bloquant est sensiblement complémentaire de la séquence de la première sonde ou de la deuxième sonde dans une paire de sondes ciblant les un ou plusieurs analytes pour une capture réduite ;
(c) l'hybridation :
(i) de la pluralité de paires de sondes à la pluralité d'analytes, dans lequel la première sonde et la deuxième sonde sont hybridées à l'analyte ; et
(ii) du premier oligonucléotide bloquant à la séquence de la première sonde ou de la deuxième sonde ciblant les un ou plusieurs analytes pour une capture réduite ;
(d) la ligature de la première sonde hybridée et de la deuxième sonde, pour ainsi générer un produit de ligature ; et
(e) la capture du produit de ligature avec le domaine de capture sur le réseau,
dans lequel la capture du produit de ligature des un ou plusieurs analytes pour une capture réduite est réduite comparativement à la capture d'un produit de ligature où aucun oligonucléotide bloquant n'est présent.

2. Procédé pour augmenter la diversité d'acides nucléiques dans l'analyse transcriptomique spatiale d'un échantillon de tissu comprenant :
(a) la fourniture d'un réseau comprenant une pluralité de sondes de capture, dans lequel une sonde de capture de la pluralité de sondes de capture comprend : (i) un code à barres spatial et (ii) un domaine de capture ;
(b) la mise en contact de l'échantillon de tissu comprenant une pluralité d'acides nucléiques, dans lequel la pluralité d'acides nucléiques comprend un ou plusieurs acides nucléiques pour une capture réduite et un acide nucléique, avec :
(i) une pluralité de paires de sondes, chaque paire de sondes comprenant une première sonde et une deuxième sonde, dans lequel la première sonde et la deuxième sonde comprennent chacune des séquences qui sont sensiblement complémentaires de séquences soit de l'acide nucléique soit des un ou plusieurs acides nucléiques pour une capture réduite, dans lequel la deuxième sonde comprend un domaine de capture de sonde de capture qui comprend une séquence qui est complémentaire de la totalité ou d'une portion du domaine de capture de la sonde de capture ; et
(ii) un premier oligonucléotide bloquant, dans lequel le premier oligonucléotide bloquant est sensiblement complémentaire de la séquence de la première sonde ou de la deuxième sonde dans une paire de sondes ciblant les un ou plusieurs acides nucléiques pour une capture réduite ;
(c) l'hybridation :
(i) de la pluralité de paires de sondes à la pluralité d'acides nucléiques, dans lequel la première sonde et la deuxième sonde sont hybridées à l'acide nucléique ; et
(ii) du premier oligonucléotide bloquant à la séquence de la première sonde ou de la deuxième sonde ciblant les un ou plusieurs acides nucléiques pour une capture réduite ;
(d) la ligature de la première sonde hybridée et de la deuxième sonde, pour ainsi générer un produit de ligature ; et
(e) la capture du produit de ligature avec le domaine de capture sur le réseau,
dans lequel la diversité d'acides nucléiques dans l'analyse transcriptomique spatiale est augmentée par rapport à la diversité d'acides nucléiques dans un dosage de transcriptomique spatiale où aucun oligonucléotide bloquant n'est présent.

3. Procédé pour augmenter la détection d'un ou plusieurs acides nucléiques rares ou à faible expression dans un échantillon de tissu pour une analyse spatiale comprenant :
(a) la fourniture d'un réseau comprenant une pluralité de sondes de capture, dans lequel une sonde de capture de la pluralité de sondes de capture comprend : (i) un code à barres spatial et (ii) un domaine de capture ;
(b) la mise en contact de l'échantillon de tissu comprenant une pluralité d'acides nucléiques, dans lequel la pluralité d'acides nucléiques comprend un ou plusieurs acides nucléiques pour une capture réduite et un acide nucléique rare ou à faible expression, avec :
(i) une pluralité de paires de sondes, chaque paire de sondes comprenant une première sonde et une deuxième sonde, dans lequel la première sonde et la deuxième sonde comprennent chacune des séquences qui sont sensiblement complémentaires de séquences soit de l'acide nucléique rare ou à faible expression soit des un ou plusieurs acides nucléiques pour une capture réduite, dans lequel la deuxième sonde comprend un domaine de capture de sonde de capture qui comprend une séquence qui est complémentaire de la totalité ou d'une portion du domaine de capture de la sonde de capture ; et
(ii) un premier oligonucléotide bloquant, dans lequel le premier oligonucléotide bloquant est sensiblement complémentaire de la séquence de la première sonde ou de la deuxième sonde dans une paire de sondes ciblant les un ou plusieurs acides nucléiques pour une capture réduite ;
(c) l'hybridation :
(i) de la pluralité de paires de sondes à la pluralité d'acides nucléiques, dans lequel la première sonde et la deuxième sonde sont hybridées à l'acide nucléique rare ou à faible expression ; et
(ii) du premier oligonucléotide bloquant à la séquence de la première sonde ou de la deuxième sonde ciblant les un ou plusieurs acides nucléiques pour une capture réduite ;
(d) la ligature de la première sonde hybridée et de la deuxième sonde, générant ainsi un produit de ligature ; et
(e) la capture du produit de ligature avec le domaine de capture sur le réseau,
dans lequel la détection d'un ou de plusieurs acides nucléiques rares ou à faible expression est augmentée comparativement à la détection d'un ou de plusieurs acides nucléiques rares ou à faible expression dans un échantillon de tissu de référence où aucun oligonucléotide bloquant n'est présent.

4. Procédé selon l'une quelconque des revendications précédentes, comprenant en outre :
la mise en contact de l'échantillon de tissu avec un deuxième oligonucléotide bloquant, dans lequel le deuxième oligonucléotide bloquant est sensiblement complémentaire de la séquence de la première sonde ou de la deuxième sonde ciblant les un ou plusieurs analytes ou acides nucléiques pour une capture réduite ; et
l'hybridation du deuxième oligonucléotide bloquant à la séquence de la première sonde ou de la deuxième sonde ciblant les un ou plusieurs analytes ou acides nucléiques pour une capture réduite, facultativement dans lequel le premier oligonucléotide bloquant est sensiblement complémentaire de la séquence de la première sonde ciblant les un ou plusieurs analytes ou acides nucléiques pour une capture réduite, et le deuxième oligonucléotide bloquant est sensiblement complémentaire de la séquence de la deuxième sonde ciblant les un ou plusieurs analytes ou acides nucléiques pour une capture réduite, facultativement dans lequel le premier oligonucléotide bloquant et/ou le deuxième oligonucléotide bloquant bloquent l'hybridation de la première sonde et/ou de la deuxième sonde ciblant les un ou plusieurs analytes ou acides nucléiques pour une capture réduite aux un ou plusieurs analytes ou acides nucléiques pour une capture réduite.

5. Procédé selon l'une quelconque des revendications précédentes, dans lequel les un ou plusieurs analytes ou acides nucléiques pour une capture réduite comprennent un transcrit de gène fortement exprimé, facultativement dans lequel les un ou plusieurs analytes ou acides nucléiques pour une capture réduite comprennent un ARN, facultativement un ARN messager (ARNm), un ARN ribosomique (ARNr), un ARN mitochondrial (ARNmt) et/ou un ARN de transfert (ARNt) ou un fragment de ceux-ci ; ou un analyte dit de ménage (« housekeeping »), facultativement une glycéraldéhyde-3-phosphate déshydrogénase (GAPDH), une protéine de liaison TATA (TBP) et/ou une protéine ribosomique (RP).

6. Procédé selon l'une quelconque des revendications précédentes, dans lequel la première sonde et la deuxième sonde ciblant les un ou plusieurs analytes ou acides nucléiques pour une capture réduite ne sont pas hybridées aux un ou plusieurs analytes ou acides nucléiques pour une capture réduite, facultativement dans lequel la première sonde et la deuxième paire de sondes ciblant les un ou plusieurs analytes ou acides nucléiques pour une capture réduite ne sont pas ligaturées ensemble, facultativement dans lequel la première sonde et la deuxième sonde ciblant les un ou plusieurs analytes ou acides nucléiques pour une capture réduite ne sont pas capturées par le domaine de capture sur le réseau.

7. Procédé selon l'une quelconque des revendications 4 à 6, dans lequel :
(i) le premier oligonucléotide bloquant et/ou le deuxième oligonucléotide bloquant ne comprennent chacun pas de nucléotide phosphorylé à leur extrémité 5' et/ou ne comprennent pas une ou plusieurs bases d'acide ribonucléique à leur extrémité 3' ;
(ii) le premier oligonucléotide bloquant et/ou le deuxième oligonucléotide bloquant ne comprennent chacun pas une séquence qui est sensiblement complémentaire du domaine de capture de sonde de capture ;
(iii) le premier oligonucléotide bloquant est sensiblement complémentaire de la séquence de la première sonde ciblant les un ou plusieurs analytes ou acides nucléiques pour une capture réduite qui est complémentaire des un ou plusieurs analytes ou acides nucléiques pour une capture réduite, et/ou le deuxième oligonucléotide bloquant est sensiblement complémentaire de la séquence de la deuxième sonde ciblant les un ou plusieurs analytes ou acides nucléiques pour une capture réduite qui est complémentaire des un ou plusieurs analytes ou acides nucléiques pour une capture réduite ;
(iv) le premier oligonucléotide bloquant s'hybride à une ou plusieurs portions de la séquence de la première sonde ciblant les un ou plusieurs analytes ou acides nucléiques pour une capture réduite, et/ou le deuxième oligonucléotide bloquant s'hybride à une ou plusieurs portions de la séquence de la deuxième sonde ciblant les un ou plusieurs analytes ou acides nucléiques pour une capture réduite ; et/ou
(v) le premier oligonucléotide bloquant s'hybride à la séquence de la première sonde ciblant les un ou plusieurs analytes ou acides nucléiques pour une capture réduite qui est complémentaire des un ou plusieurs analytes ou acides nucléiques pour une capture réduite, et/ou le deuxième oligonucléotide bloquant s'hybride à la séquence de la deuxième sonde ciblant les un ou plusieurs analytes ou acides nucléiques pour une capture réduite qui est complémentaire des un ou plusieurs analytes ou acides nucléiques pour une capture réduite.

8. Procédé selon l'une quelconque des revendications 4 à 7, dans lequel le premier oligonucléotide bloquant et/ou le deuxième oligonucléotide bloquant comprennent une ou plusieurs modifications, facultativement dans lequel les une ou plusieurs modifications comprennent :
(i) biotine, modificateurs d'amino, azide, cholestéryle, alcynes et/ou modificateurs de thiol ;
(ii) un fluorophore ; et/ou
(iii) une thymine 3'-3' inversée.

9. Procédé selon la revendication 8, dans lequel le premier oligonucléotide bloquant et/ou le deuxième oligonucléotide bloquant comprennent un acide nucléique non naturel, facultativement dans lequel l'acide nucléique non naturel est un acide nucléique verrouillé (locked nucleic acid, LNA).

10. Procédé selon l'une quelconque des revendications précédentes, dans lequel la première sonde et la deuxième sonde :
(i) sont sensiblement complémentaires de séquences adjacentes de l'analyte, de l'acide nucléique, ou de l'acide nucléique rare ou à faible expression ; ou
(ii) s'hybrident à des séquences qui ne sont pas adjacentes les unes des autres sur l'analyte, l'acide nucléique, ou l'acide nucléique rare ou à faible expression, dans lequel la première sonde est étendue avec une ADN polymérase, remplissant ainsi un espace entre la première sonde et la deuxième sonde et générant une première sonde étendue.

11. Procédé selon l'une quelconque des revendications précédentes, dans lequel la première sonde comprend au moins deux bases d'acide ribonucléique à l'extrémité 3', et la deuxième sonde comprend un nucléotide phosphorylé à l'extrémité 5'.

12. Procédé selon l'une quelconque des revendications précédentes, dans lequel la génération du produit de ligature comprend la ligature (i) de la première sonde à la deuxième sonde ou (ii) de la première sonde étendue à la deuxième sonde en utilisant une ligature enzymatique ou une ligature chimique, dans lequel la ligature enzymatique utilise une ligase, facultativement dans lequel la ligase est une T4 ARN ligase (Rnl2), une PBCV-1 ligase, une Chlorella virus ADN ligase, une ADN ligase simple brin, ou une T4 ADN ligase.

13. Procédé selon l'une quelconque des revendications précédentes, dans lequel la deuxième sonde comprend un groupe phosphate pré-adénylé à son extrémité 5', et la première sonde comprend au moins deux bases d'acide ribonucléique à l'extrémité 3', facultativement dans lequel la ligature de la première sonde hybridée et de la deuxième sonde comprend la ligature d'une extrémité 3' de la première sonde à l'extrémité 5' de la deuxième sonde en utilisant une ligase qui ne nécessite pas d'adénosine triphosphate pour l'activité de ligase, facultativement dans lequel la ligase est une 5' AppADN/ARN Ligase thermostable, une T4 ARN Ligase 2 tronquée, une T4 ARN Ligase 2 K227Q tronquée, une T4 ARN Ligase 2 KQ tronquée, une PBCV-1 ADN ligase, ou une Chlorella Virus ADN Ligase.

14. Procédé selon l'une quelconque des revendications 1 à 13, comprenant en outre la fourniture d'une fraction de blocage de domaine de capture de sonde de capture qui interagit avec le domaine de capture de sonde de capture, facultativement comprenant en outre la libération de la fraction de blocage de domaine de capture de sonde de capture du domaine de capture de sonde de capture avant (e), dans lequel le domaine de capture de sonde de capture comprend une séquence poly-adénylée (poly(A)) ou un complément de celle-ci, facultativement dans lequel la fraction de blocage de domaine de capture de sonde de capture comprend une séquence poly-uridine, une séquence poly-thymidine, ou les deux, facultativement dans lequel la libération de la séquence poly-uridine de la séquence poly(A) comprend la dénaturation du produit de ligature ou la mise en contact du produit de ligature avec une endonucléase, exonucléase et/ou ribonucléase.

15. Procédé selon l'une quelconque des revendications 7 à 14, dans lequel le domaine de capture de la sonde de capture comprend une séquence qui est complémentaire de la totalité ou d'une portion du domaine de capture de sonde de capture, facultativement dans lequel le domaine de capture de sonde de capture comprend une séquence dégénérée.

16. Procédé selon l'une quelconque des revendications précédentes, dans lequel la première sonde et/ou la deuxième sonde sont des sondes d'ADN, facultativement dans lequel la fraction de blocage de domaine de capture de sonde de capture est une sonde d'ADN.

17. Procédé selon l'une quelconque des revendications précédentes, comprenant en outre, avant (e), la libération du produit de ligature de l'analyte, de l'acide nucléique, ou de l'acide nucléique rare ou à faible expression, facultativement dans lequel la libération (i) du produit de ligature de l'analyte, de l'acide nucléique, ou de l'acide nucléique rare ou à faible expression, ou (ii) de la fraction de blocage de domaine de capture de sonde de capture du domaine de capture de sonde de capture, comprend la mise en contact des sondes ligaturées avec une endoribonucléase, dans lequel l'endoribonucléase est la RNase H, RNase A, RNase C, ou RNase I, facultativement dans lequel la RNase H est RNase H1, RNase H2, ou RNase H1 et RNase H2,

18. Procédé selon l'une quelconque des revendications précédentes, dans lequel l'échantillon de tissu est un échantillon de tissu fixé au formol et inclus en paraffine (FFPE), un échantillon de tissu frais ou congelé, facultativement dans lequel l'échantillon de tissu FFPE est déréticulé, facultativement dans lequel l'échantillon de tissu a été préalablement coloré, facultativement en utilisant l'immunofluorescence, l'immunohistochimie, ou l'hématoxyline et l'éosine (H&E).

19. Procédé selon l'une quelconque des revendications précédentes, dans lequel le procédé comprend la mise en contact de l'échantillon de tissu avec un agent de perméabilisation, dans lequel l'agent de perméabilisation est un solvant organique, un détergent ou une enzyme, facultativement dans lequel l'agent de perméabilisation est une endopeptidase, une protéase, le dodécylsulfate de sodium (SDS), le polyéthylène glycol, l'éther tert-octylphénylique, le polysorbate 80, le polysorbate 20, la solution de sel de sodium de N-lauroylsarcosine, la saponine, un tensioactif non ionique ou Tween-20^{™}, facultativement dans lequel l'endopeptidase est la pepsine ou la protéinase K.

20. Procédé selon l'une quelconque des revendications précédentes, comprenant en outre : la détermination (i) de la totalité ou d'une partie de la séquence du produit de ligature lié au domaine de capture, ou d'un complément de celle-ci, et (ii) de la séquence du code à barres spatial, ou d'un complément de celle-ci, facultativement dans lequel la détermination comprend l'amplification de la totalité ou d'une partie du produit de ligature spécifiquement lié au domaine de capture, générant ainsi un produit d'amplification, facultativement dans lequel le produit d'amplification comprend (i) la totalité ou une partie de la séquence du produit de ligature spécifiquement lié au domaine de capture, ou un complément de celle-ci, et (ii) la séquence du code à barres spatial, ou un complément de celle-ci, facultativement dans lequel la détermination comprend le séquençage (i) de la totalité ou d'une partie de la séquence du produit de ligature, ou d'un complément de celle-ci, et (ii) de la séquence du code à barres spatial, ou d'un complément de celle-ci, facultativement dans lequel le séquençage comprend un séquençage *in situ,* des procédés de séquençage Sanger, des procédés de séquençage de nouvelle génération, ou un séquençage par nanopores.
